(19) Europäisches Patentamt
European Patent Office
Office européen des brevets

(11) EP 1 900 772 B1

(12) **EUROPÄISCHE PATENTSCHRIFT**

(45) Veröffentlichungstag und Bekanntmachung des Hinweises auf die Patenterteilung:
**31.03.2010 Patentblatt 2010/13**

(51) Int Cl.:
***C08L 33/14*** *(2006.01)* ***C08F 20/34*** *(2006.01)*
***A61Q 5/00*** *(2006.01)* ***A61K 8/18*** *(2006.01)*

(21) Anmeldenummer: **07016150.0**

(22) Anmeldetag: **17.08.2007**

(54) **Formfixierung faserhaltiger Materialien durch Polymere**

Shape retention of fibrous materials using polymers

Fixation de forme de matériaux contenant des fibres à l'aide de polymères

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **22.08.2006 DE 102006039283**

(43) Veröffentlichungstag der Anmeldung:
**19.03.2008 Patentblatt 2008/12**

(73) Patentinhaber: **Henkel AG & Co. KGaA**
**40589 Düsseldorf (DE)**

(72) Erfinder:
- **Kuhnert, Oliver**
  **40597 Düsseldorf (DE)**
- **Hätzelt, Andre**
  **40591 Düsseldorf (DE)**
- **Ferencz, Andreas**
  **40223 Düsseldorf (DE)**
- **Krügermann, Ina**
  **40764 Langenfeld (DE)**
- **Knappe, Thorsten**
  **22869 Schenefeld (DE)**

(56) Entgegenhaltungen:
**GB-A- 1 306 544 GB-A- 2 018 447**
**US-A- 3 445 438**

EP 1 900 772 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft ausgewählte Polymere, eine Zubereitung zur Verformung faserhaltiger Materialien, insbesondere menschlicher Haare, welche diese Polymere enthält, ein Umformungsverfahren sowie die Verwendung dieser Polymere bzw. der Zubereitung zur Behandlung und zur dauerhaften Verformung faserhaltiger Materialien, insbesondere menschlicher Haare.

Faserhaltige Materialien, das heißt die Faser selbst bzw. Faserbündel sowie aus den Fasern gewonnen Erzeugnisse (wie beispielsweise Stoffe bzw. Textilien) sollen Ihre Form über einen langen Zeitraum behalten oder müssen des Öfteren in eine neue äußere Form gebracht werden. Jede Formgebung bzw. Formfixierung soll für das Material schonend ablaufen, möglichst dauerhaft aber gegebenenfalls dennoch reversibel sein. Für die Umformung und Formfixierung kennt der Fachmann diverse Möglichkeiten.

Das faserhaltige Material kann permanent umgeformt werden. Dies gelingt beispielsweise durch Brechung von intramolekularen Bindungen der Fasern, anschließender Verformung des Materials und folgender Fixierung der neuen Form per Knüpfung neuer intramolekularer Bindungen. Diese Prozedur wendet man gleichfalls bei der Umformung keratinhaltiger Fasern, insbesondere menschlicher Haare, im Rahmen einer Dauerwelle an. Das sogenannte Dauerwell- oder Kaltwellverfahren birgt jedoch den Nachteil, dass die Faserstruktur des Materials sowohl durch den reduktiven Schritt der Bindungsbrechung als auch dem oxidativen Schritt der Formfixierung beeinträchtigt wird. Darüber hinaus eignet sich das Kaltwellverfahren nur für Fasern, in denen Disulfidbrücken bzw. Sulfhydrylgruppen vorhanden sind.

Der Anwender wünscht sich eine zur permanenten Formfixierung bzw. Umformung faserschonende Alternative mit permanenter Wirkung.

Bei der Verformung keratinhaltiger Fasern spielt neben der permanenten Formgebung auch die faserschonende temporäre Formgebung eine wichtige Rolle. Temporäre Formgebungen, die Halt ergeben sollen, ohne das gesunde Aussehen beispielsweise der Haare, i.e. deren Glanz, zu beeinträchtigen, können beispielsweise durch Haarsprays, Haarwachse, Fönwellen etc. erzielt werden. Eine ansprechend aussehende Frisur ist als modischer Akzent selbstredend an Trends gebunden. Viele extrem modische Stylings lassen sich in reversibler Form bei den meisten Haartypen nur unter Verwendung von formgebenden und formfixierenden, polymeren Wirkstoffe aufbauen.

Haarsprays enthalten folglich als formgebenden und formfixierenden Wirkstoff üblicherweise synthetische Polymere, die die Fasern festigen. Zubereitungen, die ein gelöstes oder dispergiertes Polymer enthalten, können mittels Treibgasen oder durch einen Pumpmechanismus auf die Fasern aufgebracht werden. Die für die temporäre Umformung üblicherweise eingesetzten kosmetischen Polymere zeigen in wäßrigen, wäßrig-alkoholischen oder alkoholischen Lösungen gute Festigungseigenschaften, die nach der Anwendung mehr oder weniger gut die Fasern verformen und festigen und die den Fasern zusätzlich auch mehr Volumen geben können.

Die formgebenden und formfixierenden polymeren Wirkstoffe können auch in übliche Reinigungs- oder Konditioniermittel eingearbeitet werden. In vielen Fällen ist es aber vorteilhaft, sie in Form spezieller Mittel wie Haarfestiger, Gele, Wachse oder Sprays anzuwenden.

Häufig hält der Effekt von polymerbasierten temporären Verformungsmitteln nicht lange an. Bereits beim Durchkämmen der Haare oder bei einer Wäsche der Fasern geht der erwünschte Volumeneffekt bzw. die Formfixierung nahezu verloren. Der Anwender wünscht sich zwar, dass die temporäre Umformung reversibel vorzunehmen ist, dennoch empfindet der Anwender den erzielten Effekt wegen des schnellen Wirkungsverlusts als zu kurzlebig. Daher besteht die Aufgabe, die Festigung, Formfixierung und Volumengebung faserhaltiger Materialien, insbesondere keratinhaltiger Fasern, wie beispielsweise menschlichem Haar, durch geeignete Polymere beständiger zu gestalten.

Viele der festigenden oder Volumen gebenden Polymere haben häufig unerwünschte Nebeneffekte, die sich dadurch bemerkbar machen, daß das behandelte Haar einen zu rauhen Griff, eine zu hohe Belastung oder eine ungenügende Elastizität aufweist. Außerdem bilden sich oft sichtbare Polymerrückstände auf dem Haar, die den ästhetischen Eindruck des Haars herabsetzen.

Es hat viele Bemühungen gegeben, die permanenten und die temporären, haarfestigenden Mittel weiterzuentwickeln und zu optimieren. Insbesondere fehlte es in der Vergangenheit nicht an Versuchen, die Dauerhaftigkeit einer durch Polymere aufgeprägten Formgebung keratinhaltiger Fasern zu verbessern. Dabei soll die Festigung, die Volumengebung oder die Sprungkraft der Fasern optimal sein sowie die Faserstruktur geschont werden.

Aus der Auslegeschrift DE 1 224 320 kennt der Fachmann $\alpha,\beta$-ungesättigte 2-Acyloxyalkylamino-4,6-dichlor-s-triazine, die als Herbizide oder als Stabilisatoren für Polyvinylchlorid und chloriertem Kautschuk fungieren.

Aus der Druckschrift US-A-4 168 976 sind Polymere mit halogenierten Triazinen oder Pyrimidinen bekannt, die in der Fotografie als Trägermaterial zur Abscheidung von Farbstoffen auf einem Substrat Einsatz finden.

Die oben genannte Aufgabe wird überraschenderweise durch ein Mittel gelöst, welches in einem komsetischen Träger mindestens ein Polymer mit mindestens einer Struktureinheit der Formel (IV) enthält,

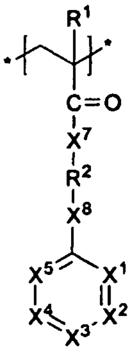

(IV)

worin

- die Reste $X^1$ bis $X^5$ unabhängig voneinander für eine Methanylylidengruppe, ein Stickstoffatom oder eine Halogenmethanylylidengruppe stehen, mit der Maßgabe, dass mindestens zwei der Reste $X^1$ bis $X^5$ ein Stickstoffatom und mindestens zwei der Reste $X^1$ bis $X^5$ eine Halogenmethanylylidengruppe und höchstens ein Rest aus $X^1$ bis $X^5$ eine Methanylylidengruppe bedeutet,
- $X^7$ für ein Sauerstoffatom oder eine Gruppe NH steht,
- $X^8$ für eine direkte Bindung, ein Sauerstoffatom oder eine Gruppe NH steht,
- $R^1$ ein Wasserstoffatom oder eine ($C_1$- bis $C_4$)-Alkylgruppe bedeutet,
- $R^2$ für eine direkte Bindung, ($C_2$- bis $C_6$-)Alkylengruppe, eine Oligoalkylenoxidgruppe mit 1 bis 20 Einheiten ($C_2$- bis $C_4$)-Alkylenoxid oder eine Phenylengruppe steht,

Die mit diesem Mittel erhaltene Formgebung ist insbesondere stabilisiert gegenüber Waschen und mechanischer Einwirkung, wie beispielsweise kämmen oder reiben.

In der Formel (IV) und allen folgenden Formeln steht eine chemische Bindung, die mit dem Symbol * gekennzeichnet ist, für eine freie Valenz des entsprechenden Strukturfragments.

Ein Polymer im Sinne der Erfindung ist ein Makromolekül, welches mindestens eine Art an wiederkehrenden Struktureinheiten enthält und eine Molmasse von größer 1000 g/mol aufweist.

Bevorzugte Halogenmethanylylidengruppen sind die Chlormethanylylidengruppe, die Brommethanylylidengruppe und die Iodmethanylylidengruppe. Die Chlormethanylylidengruppe wird erfindungsgemäß bevorzugt verwendet.

Bevorzugt geeignete Mittel enthalten mindestens ein Polymer mit mindestens einer Struktureinheit der Formel (IV), in der $X^1$, $X^3$, und $X^5$ für ein Stickstoffatom, $X^6$ für eine Methanylylidengruppe und $X^2$ und $X^4$ für eine Halogenmethanylylidengruppe (insbesondere eine Chlormethanylylidengruppe) stehen.

Weiterhin sind Mittel bevorzugt, die mindestens ein Polymer mit mindestens einer Struktureinheit der Formel (IV) enthalten, in der $X^4$ und $X^5$ mit dem Restmolekül einen ankondensierten aromatischen Sechsring bilden an den der Rest L bindet, $X^3$ und $X^6$ ein Stickstoffatom bedeuten und $X^1$ und $X^2$ für eine Halogenmethanylylidengruppe (insbesondere eine Chlormethanylylidengruppe) stehen.

Bevorzugte Mittel enthalten ebenso mindestens ein Polymer mit mindestens einer Struktureinheit der Formel (IV), in der $X^1$, $X^2$ und $X^4$ für eine Halogenmethanylylidengruppe (insbesondere eine Chlormethanylylidengruppe), $X^6$ für eine Methanylylidengruppe und $X^3$ und $X^5$ für ein Stickstoffatom stehen.

Desweiteren können in einer Synthesevariante zunächst Monomere dargestellt werden, welche einen oligohalogenierten Stickstoffheterozyklus enthalten, wie beispielsweise die in den Druckschriften DE-OS-1 224 320 und US-A-4 168 976 beschrieben Monomere. Die erhaltenen Monomere werden dann unter Bildung des erfindungsgemäßen (Co)Polymers umgesetzt.

Als besonders bevorzugte Struktureinheit der Formel (IV) ist mindestens eine Struktureinheit der Formeln (I-1) bis (I-26) in den Polymeren des erfindungsgemäßen Mittels enthalten:

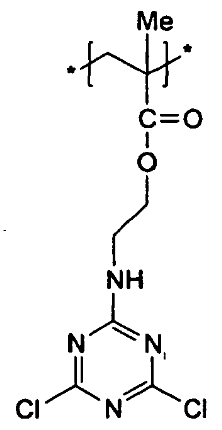 (I-1)

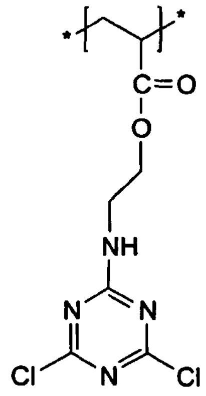 (I-2)

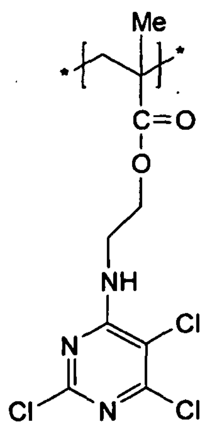 (I-3)

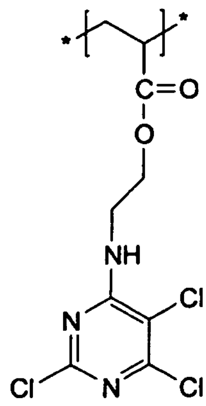 (I-4)

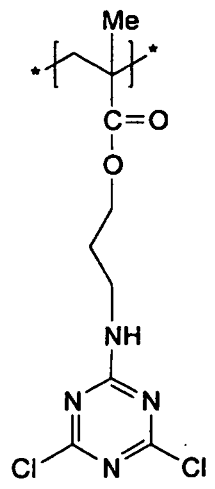 (I-5)

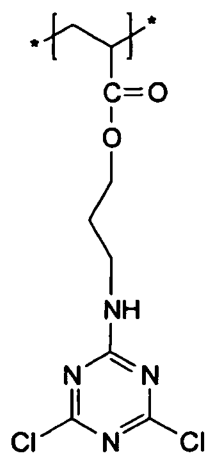 (I-6)

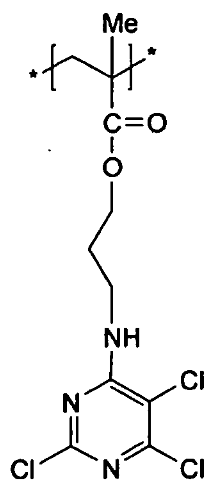 (I-7)

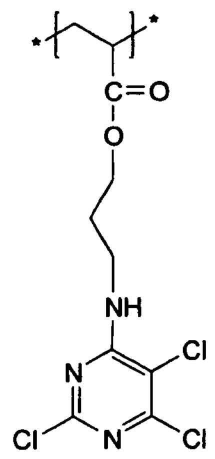 (I-8)

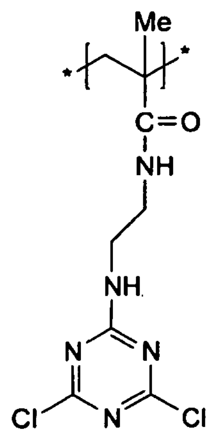 (I-9)

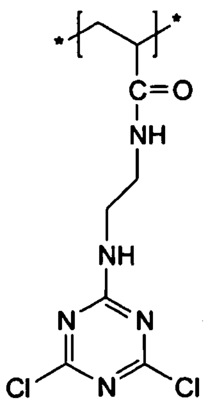 (I-10)

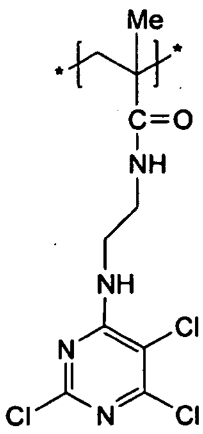 (I-11)

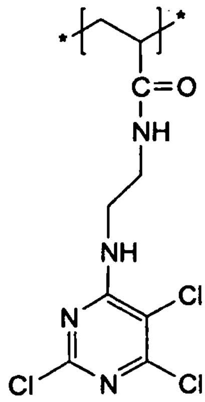 (I-12)

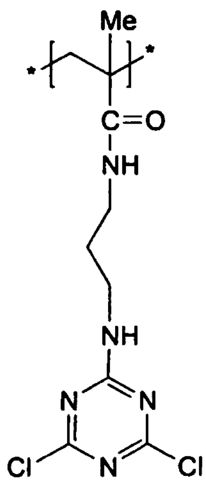 (I-13)

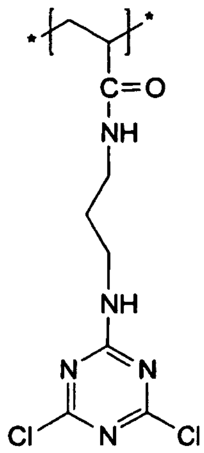 (I-14)

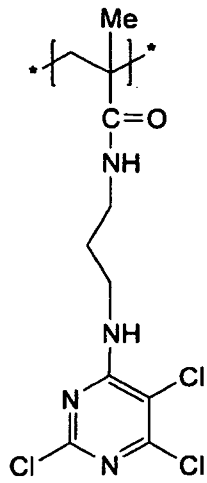 (I-15)

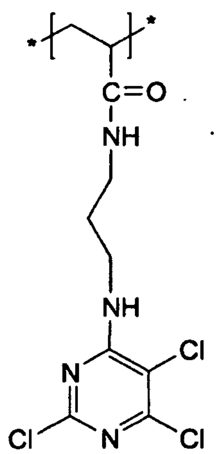 (I-16)

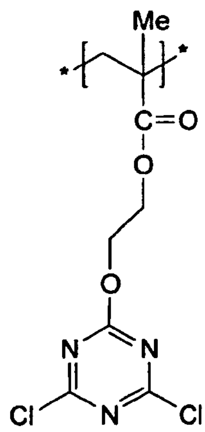 (I-19)

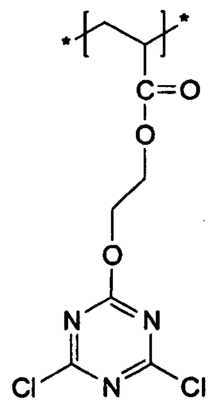 (I-20)

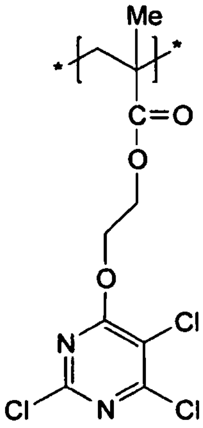 (I-21) 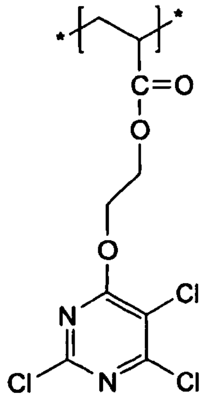 (I-22) 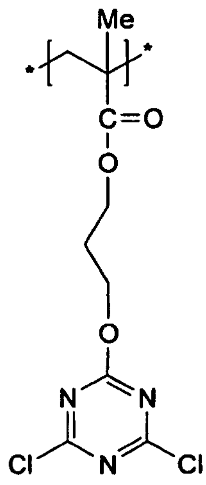 (I-23)

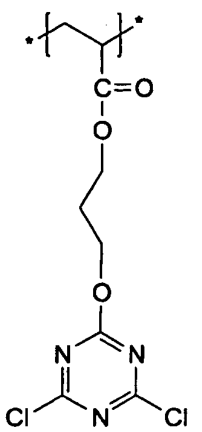 (I-24) 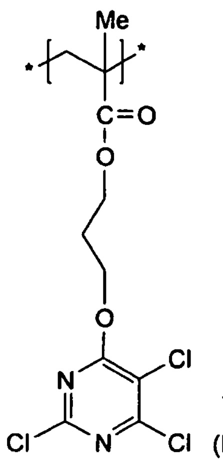 (I-25) 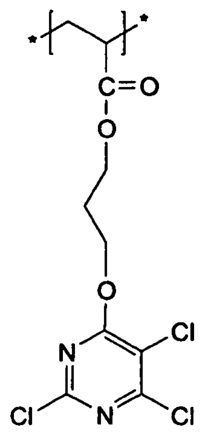 (I-26)

Die Polymere des erfindungsgemäß Mittels, welche mindestens eine Struktureinheit der Formel (IV) enthalten, besitzen bevorzugt eine mittlere Molmasse (in g/mol) von 1500 bis 1250000, insbesondere von 10000 bis 500000, besonders bevorzugt von 20000 bis 275000, ganz besonders bevorzugt von 30000 bis 120000.

Darüber hinaus ist es besonders bevorzugt, wenn es sich bei den Polymeren des erfindungsgemäßen Mittels, welche mindestens eine Struktureinheit der Formel (IV) enthalten, um Copolymere handelt. Diese Copolymere enthalten entweder mehrere unterschiedliche Struktureinheiten gemäß Formel (IV) oder bevorzugt mindestens eine, von der Struktureinheit gemäß Formel (IV) verschiedene Struktureinheit. Alle Struktureinheiten des Copolymers können in dem Polymer als Block, als Pfropf oder dendritisch angeordnet sein, oder in dem Copolymer zufällig statistisch verteilt vorliegen. Es handelt sich bei den besagten Copolymeren bevorzugt um Polymere mit 2, 3, 4, 5 oder 6 verschiedenen, wiederkehrenden Struktureinheiten, besonders bevorzugt um Polymere mit 2, 3 oder 4 verschiedenen, wiederkehrenden Struktureinheiten.

Es ist bevorzugt, wenn die Struktureinheit der Formel (IV) zu mindestens 5 Mol%, bevorzugt zu mindestens 20 Mol% in dem Copolymer enthalten ist.

Zur Lösung der Aufgabe bevorzugt eignen sich Mittel, die mindestens ein Polymer enthalten, das neben besagter Struktureinheit der Formel (IV) zusätzlich mindestens eine der nachfolgenden Struktureinheiten enthält

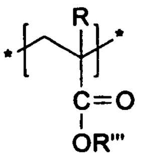 (M1) 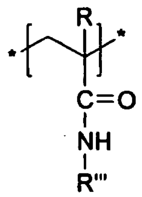 (M2) 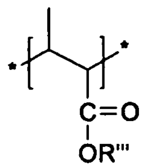 (M3)

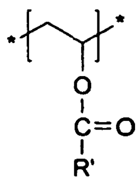 (M4) 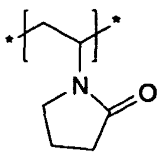 (M5) 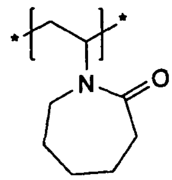 (M6)

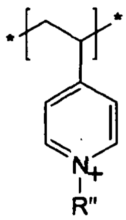 (M7) 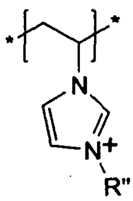 (M8) 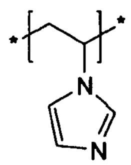 (M9)

worin

R steht für ein Wasserstoffatom oder eine ($C_1$- bis $C_4$)-Alkylgruppe,

R' steht für eine ($C_1$- bis $C_{30}$)-Alkylgruppe oder eine Arylgruppe,

R" steht für eine ($C_1$- bis $C_4$)-Alkylgruppe, eine ($C_1$- bis $C_4$)-Aralkylgruppe oder eine ($C_2$- bis $C_4$)-Alkenylgruppe,

R'" steht für ein Wasserstoffatom, eine lineare oder verzweigte ($C_1$- bis $C_{30}$)-Alkylgruppe, eine ($C_2$- bis $C_4$)-Alkenylgruppe, eine ω-Amino($C_2$-$C_6$)alkylgruppe, eine ω-Di($C_1$- bis $C_4$)alkylamino($C_2$- bis $C_6$)alkylgruppe, ω-Tri($C_1$- bis $C_4$)alkylammonio ($C_2$- bis $C_6$)alkylgruppe, eine Gruppe - $(CH_2CH_2O)_n$-$R^{IV}$ mit n gleich einer ganzen Zahl von 1 bis 60, insbesondere von 10 bis 40, weiterhin von 1 bis 30, und $R^{IV}$ steht für ein Wasserstoffatom oder eine ($C_1$- bis $C_{30}$)-Alkylgruppe.

Zur Kompensation von Ladungen im Polymer sind zusätzlich Kationen (insbesondere wenn R"' in (M1) bzw. (M3) für ein Wasserstoffatom steht und das erfindungsgemäße Mittel einen basischen pH-Wert besitzt) bzw. entsprechende Anionen (insbesondere für Formeln (M7) und (M8) oder wenn R"' in den Formeln (M1), (M2) bzw. (M3) eine kationische Gruppe bedeutet) zum Ladungsausgleich notwendig.

Als Kationen kommen prinzipiell alle Kationen in Frage, die keine Reaktion mit dem übrigen Polymerbestandteilen eingehen. Insbesondere sind dies Metallkationen der physiologisch verträglichen Metalle aus den Gruppen Ia, Ib, IIa. IIb, IIIb, VIa oder VIII des Periodensystems der Elemente, Ammoniumionen, sowie organische Verbindungen mit quaterniertem Stickstoffatom.

Letztere werden bevorzugt durch permanente Quaternisierung organischer Amine gebildet. Ein prominentes Beispiel für diese kationischen organischen Ammoniumverbindungen sind Trimethylammonioalkanole wie 2-Trimethylammonioethanol. Bevorzugte Metallkationen sind Alkalimetallionen, insbesondere Natriumion bzw. Kaliumion.

Als Anionen kommen prinzipiell alle Anionen in Frage, die keine Reaktion mit dem übrigen Polymerbestandteilen eingehen. Die besagten Anionen werden bevorzugt ausgewählt aus Halogenid, Sulfat, Methylsulfat, Triflat, Hydrogensulfat, Carbonat, Hydrogencarbonat, Tetrafluoroborat, Phosphat, Hydrogenphosphat, Dihydrogenphosphat oder p-Toluolsulfonat. Besonders bevorzugt steht das Anion für Chlorid, Bromid, p-Toluolsulfonat, Methylsulfat, Triflat oder Hydrogensulfat.

Im Folgenden sollen Beispiele für die als Substituenten im Rahmen dieser Anmeldung genannten Gruppen bzw. Reste erwähnt werden:

| | |
|---|---|
| ($C_1$ bis $C_{30}$)-Alkylgruppe: | Methyl, Ethyl, Propyl, Isopropyl, Butyl, sec-Butyl, Isobutyl, t-Butyl, Pentyl, 2,4,4-Trimethylpent-2-yl, Hexyl, 2-Ethylhex-1-yl, Octyl, Heptyl, 2-Methyloct-2-yl, Undecyl, Decyl, Dodecyl, Pentadecyl, Tetradecyl, Pentadecyl, Hexadecyl, Heptadecyl, Octadecyl |
| ($C_1$ bis $C_4$)-Alkylgruppe: | Methyl, Ethyl, n-Propyl, Isopropyl, n-Butyl, sec-Butyl, Isobutyl, t-Butyl |
| ($C_2$ bis $C_4$)-Alkenylgruppe | Vinyl, Allyl, 3-Butenyl |
| ($C_1$ bis $C_4$)-Aralkylgruppe | Benzyl, 2-Phenylethyl |
| ($C_2$ bis $C_4$)-Alkylengruppe | Ethan-1,2-diyl, Propan-1,3-diyl, Propan-1,2-diyl, Butan-1,4-diyl |
| Arylgruppe | Phenyl, Naphthyl (bevorzugt Phenyl) |
| ω-Amino($C_2$-$C_6$)alkylgruppe | 2-Aminoethyl, 3-Aminopropyl, 4-Aminobutyl, 5-Aminopentyl, 6-Aminohexyl |
| ω-Di($C_1$- bis $C_4$)alkylamino($C_2$- bis $C_6$)alkylgruppe: | 2-Dimethylaminoethyl, 3-Dimethylaminopropyl, 4-Dimethylaminobutyl, 5-Dimethylaminopentyl, 6-Dimethylaminohexyl |
| ω-Tri($C_1$- bis $C_4$)alkylammonio($C_2$- bis $C_6$)alkylgruppe: | 2-Trimethylammonioethyl, 3-Trimethylammoniopropyl, 2-Dimethylethylammonioethyl, 3-Dimethylethylammoniopropyl, 4-Trimethylammoniobutyl, 5-Trimethylammoniopentyl, 6-Trimethylammoniohexyl |
| ($C_2$ bis $C_4$)-Alkylenoxid | Ethylenoxid, Propylenoxid, Butylenoxid |
| Arylengruppe | Phenylen, Naphthylen |
| Poly($C_2$ bis $C_4$)alkylenoxidgruppe | Poly(ethylenoxid), Poly(propylenoxid), Poly(butylenoxid) |
| ($C_2$ bis $C_6$)-Alkylen-α,ω-dioxy | 1,2-Dioxyethylen, 1,3-Dioxypropylen, 1,4-Dioxybutylen, 1,5-Dioxypentylen, 1,6-Dioxyhexylen |

Die Reste $-(CH_2CH_2O)-Me$, $-(CH_2CH_2O)_2-Me$, $-(CH_2CH_2O)_3-Me$, $-(CH_2CH_2O)_4-Me$, $-(CH_2CH_2O)_5-Me$, $-(CH_2CH_2O)_6-Me$, $-(CH_2CH_2O)_7$-Me, $-(CH_2CH_2O)_7-Me$, $-(CH_2CH_2O)_9-Me$, $-(CH_2CH_2O)_{10}-Me$, $-(CH_2CH_2O)_{11}-Me$, $-(CH_2CH_2O)_{12}-Me$, $-(CH_2CH_2O)_{13}-Me$, $-(CH_2CH_2O)_{14}-Me$, $-(CH_2CH_2O)_{15}-Me$, $-(CH_2CH_2O)_{16}-Me$, $-(CH_2CH_2O)_{17}-Me$, $-(CH_2CH_2O)_{18}-Me$, $-(CH_2CH_2O)_{19}-Me$, $-(CH_2CH_2O)_{20}-Me$, $-(CH_2CH_2O)_{21}-Me$, $-(CH_2CH_2O)_{22}-Me$, $-(CH_2CH_2O)_{23}-Me$, $-(CH_2CH_2O)_{24}-Me$, $-(CH_2CH_2O)_{25}-Me$, $-(CH_2CH_2O)_{26}-Me$, $-(CH_2CH_2O)_{27}-Me$, $-(CH_2CH_2O)_{28}-Me$, $-(CH_2CH_2O)_{29}-Me$, $-(CH_2CH_2O)_{30}-Me$, $-(CH_2CH_2O)_{31}-Me$, $-(CH_2CH_2O)_{32}-Me$, $-(CH_2CH_2O)_{33}-Me$, $-(CH_2CH_2O)_{34}-Me$, $-(CH_2CH_2O)_{35}-Me$, $-(CH_2CH_2O)_{36}-Me$, $-(CH_2CH_2O)_{37}-Me$, $-(CH_2CH_2O)_{38}-Me$, $-(CH_2CH_2O)_{39}-Me$, $-(CH_2CH_2O)_{40}-Me$, $-(CH_2CH_2O)_{50}-Me$, $-(CH_2CH_2O)_{60}-Me$, $-(CH_2CH_2O)-H$, $-(CH_2CH_2O)_2-H$,- $(CH_2CH_2O)_3-H$, $-(CH_2CH_2O)_4-H$, $-(CH_2CH_2O)_5-H$, $-(CH_2CH_2O)_6-H$, $-(CH_2CH_2O)_7-H$, $-(CH_2CH_2O)_7-H$, $-(CH_2CH_2O)_9-H$, $-(CH_2CH_2O)_{10}H$, $-(CH_2CH_2O)_{11}-H$, $-(CH_2CH_2O)_{12}-H$, $-(CH_2CH_2O)_{13}-H$, $-(CH_2CH_2O)_{14}-H$, $-(CH_2CH_2O)_{15}-H$, $-(CH_2CH_2O)_{16}-H$, $-(CH_2CH_2O)_{17}-H$, $-(CH_2CH_2O)_{18}-H$, $-(CH_2CH_2O)_{19}-H$, $-(CH_2CH_2O)_{20}-H$, $-(CH_2CH_2O)_{21}-H$, $-(CH_2CH_2O)_{22}-H$, $-(CH_2CH_2O)_{23}-H$, $-(CH_2CH_2O)_{24}-H$, $-(CH_2CH_2O)_{25}-H$, $-(CH_2CH_2O)_{26}-H$, $-(CH_2CH_2O)_{27}-H$, $-(CH_2CH_2O)_{28}-H$, $-(CH_2CH_2O)_{29}-H$, $-(CH_2CH_2O)_{30}-H$, $-(CH_2CH_2O)_{31}-H$, $-(CH_2CH_2O)_{32}-H$, $-(CH_2CH_2O)_{33}-H$, $-(CH_2CH_2O)_{34}-H$, $-(CH_2CH_2O)_{35}-H$, $-(CH_2CH_2O)_{36}-H$, $-(CH_2CH_2O)_{37}-H$, $-(CH_2CH_2O)_{38}-H$, $-(CH_2CH_2O)_{39}-H$, $-(CH_2CH_2O)_{40}-H$, $-(CH_2CH_2O)_{50}-H$, $-(CH_2CH_2O)_{60}-H$ sind bevorzugte Reste $-(CH_2CH_2O)_n-R^{IV}$. Reste mit Methylgruppe als $R^{IV}$ sind dabei wiederum bevorzugt:

Erfindungsgemäß werden bevorzugt folgende Struktureinheiten (M1-1) bis (M1-30) aus den Verbindungen der Formel (M1) ausgewählt und mindestens eine dieser Struktureinheiten als Struktureinheit der Formel (M1) in dem erfindungsgemäß bevorzugten Copolymer verwendet:

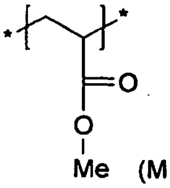
(M1-1)

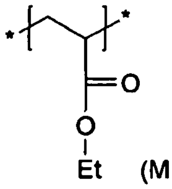
(M1-2)

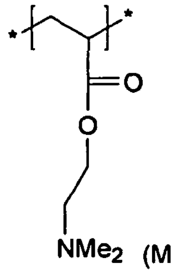
(M1-3)

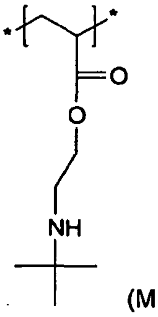
(M1-4)

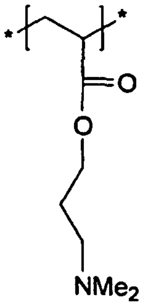
(M1-5)

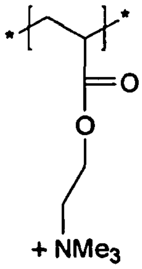
(M1-6)

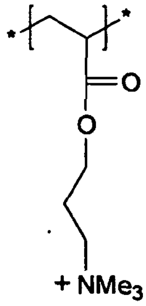
(M1-7)

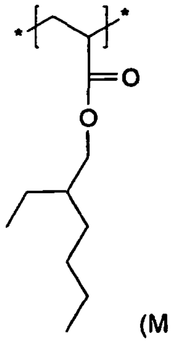
(M1-8)

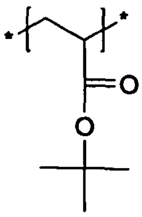
(M1-9)

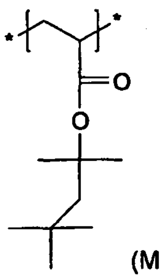
(M1-10)

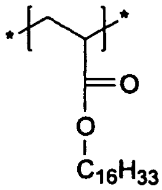
(M1-11)

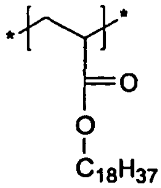
(M1-12)

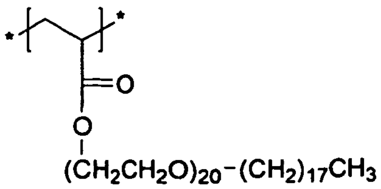
(M1-13)

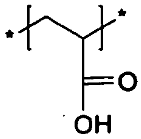
(M1-14)

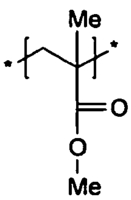
(M1-15)

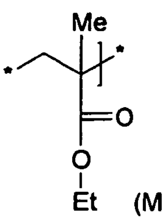
(M1-16)

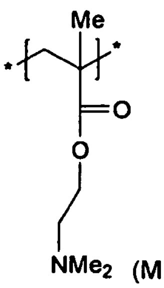
(M1-17)

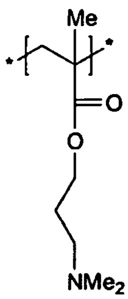
(M1-18)

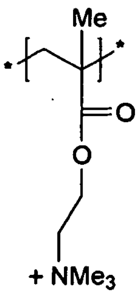
(M1-19)

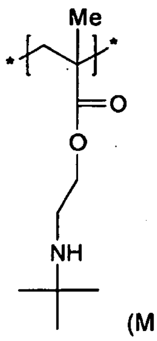 (M1-20) 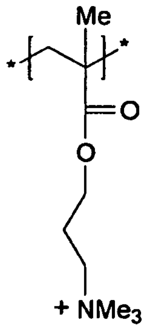 (M1-21) 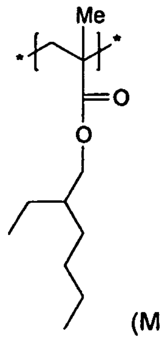 (M1-22) 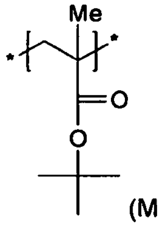 (M1-23)

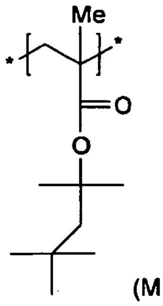 (M1-24) 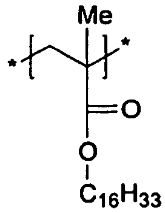 (M1-25) 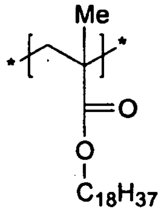 (M1-26)

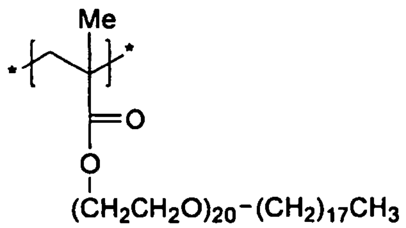 (M1-27) 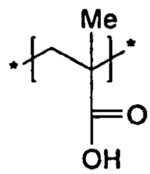 (M1-28)

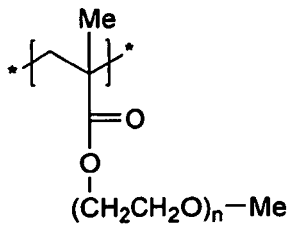 (M1-29)

mit n gleich einer ganzen Zahl von 1 bis 60, insbesondere von 10 bis 40

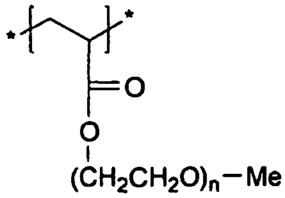 (M1-30)

mit n gleich einer ganzen Zahl von 1 bis 60, insbesondere von 10 bis 40.

Erfindungsgemäß werden bevorzugt folgende Struktureinheiten (M2-1) bis (M2-30) aus den Verbindungen der Formel (M2) ausgewählt und mindestens eine dieser Struktureinheiten als Struktureinheit der Formel (M2) in dem erfindungsgemäß bevorzugten Copolymer verwendet:

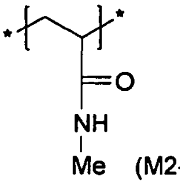 (M2-1)

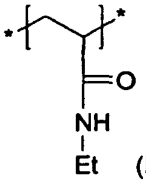 (M2-2)

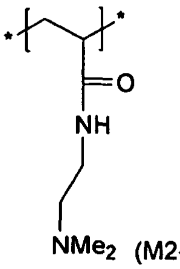 (M2-3)

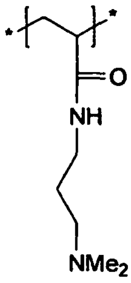 (M2-4)

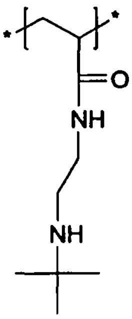 (M2-5)

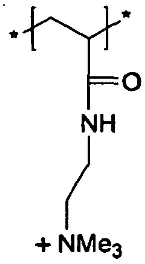 (M2-6)

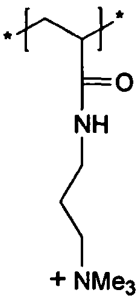 (M2-7)

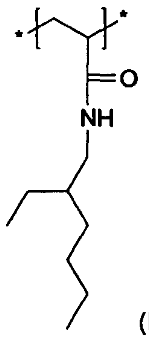 (M2-8)

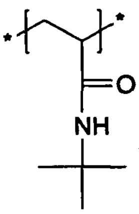 (M2-9)

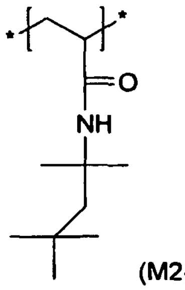 (M2-10)

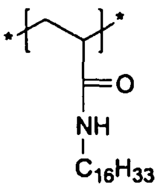 (M2-11)

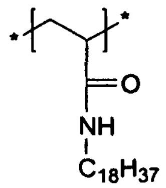 (M2-12)

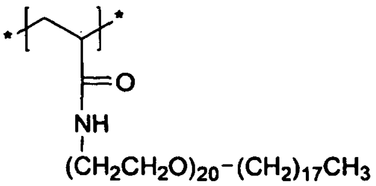 (M2-13)

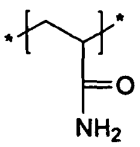 (M2-14)

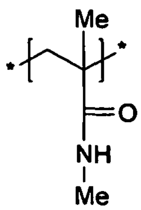 (M2-15)

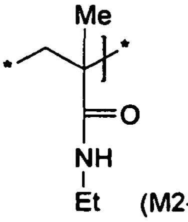 (M2-16)

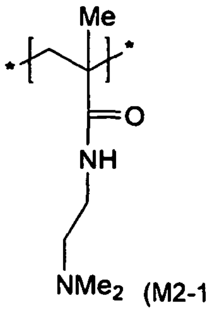 (M2-17)

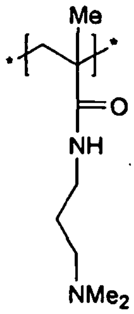 (M2-18)

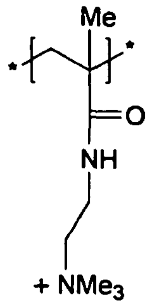 (M2-19)

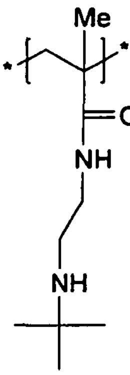 (M2-20)

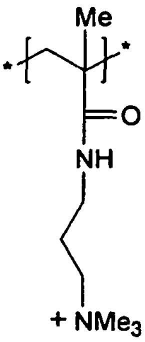 (M2-21)

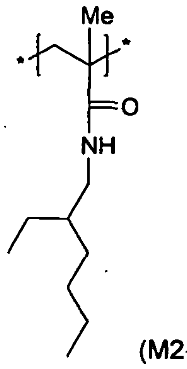 (M2-22)

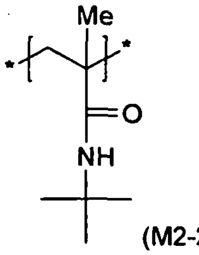 (M2-23)

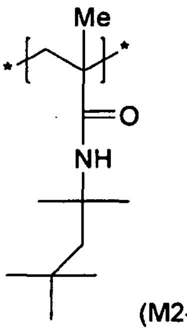 (M2-24)

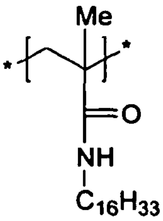 (M2-25)

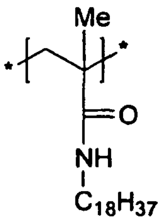 (M2-26)

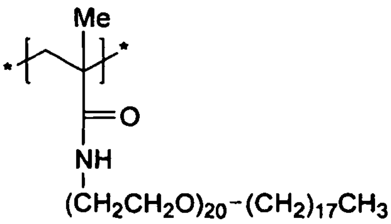 (M2-27)

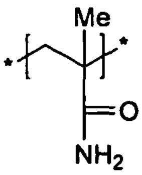 (M2-28)

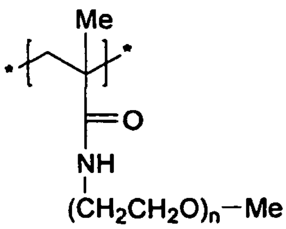 (M2-29)

mit n gleich einer ganzen Zahl von 1 bis 60, insbesondere von 10 bis 40

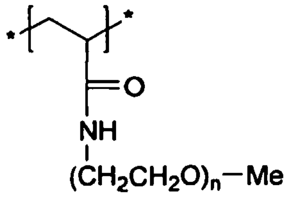 (M2-30)

mit n gleich einer ganzen Zahl von 1 bis 60, insbesondere von 10 bis 40.

Erfindungsgemäß werden bevorzugt folgende Struktureinheiten (M3-1) bis (M3-14) aus den Verbindungen der Formel (M3) ausgewählt und mindestens eine dieser Struktureinheiten als Struktureinheit der Formel (M3) in dem erfindungsgemäß bevorzugten Copolymer verwendet:

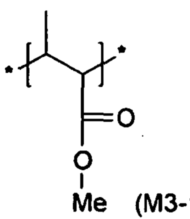 (M3-1)

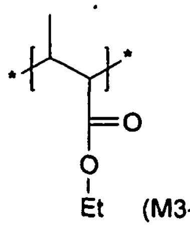 (M3-2)

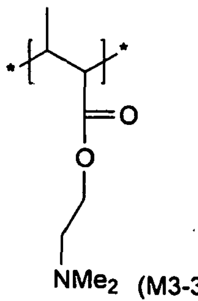 (M3-3)

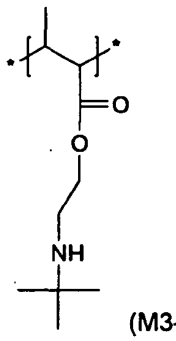 (M3-4)

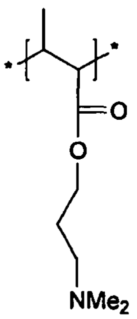 (M3-5)

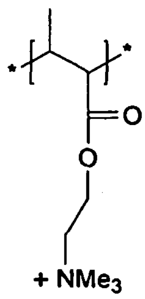 (M3-6)

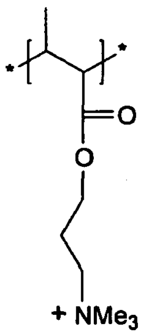 (M3-7)

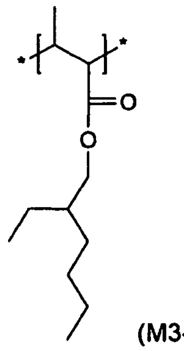 (M3-8)

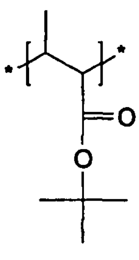 (M3-9)

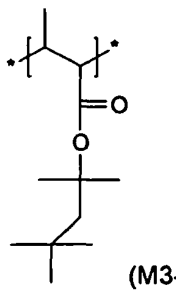 (M3-10)

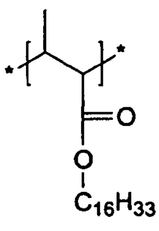 (M3-11)

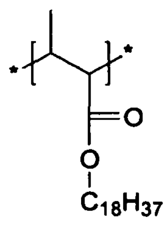 (M3-12)

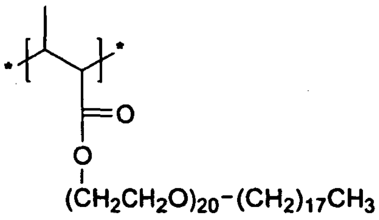 (M3-13) 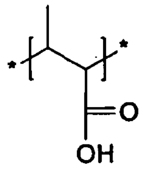 (M3-14)

Erfindungsgemäß werden bevorzugt folgende Struktureinheiten (M4-1) bis (M4-3) aus den Verbindungen der Formel (M4) ausgewählt und mindestens eine dieser Struktureinheiten als Struktureinheit der Formel (M4) in dem erfindungsgemäß bevorzugten Copolymer verwendet:

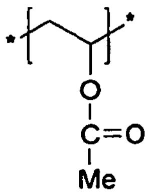 (M4-1) 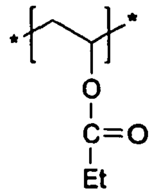 (M4-2) 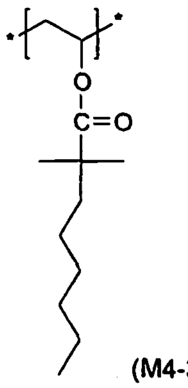 (M4-3)

Mindestens eines der folgenden Copolymere (P1) bis (P107), enthaltend die nachstehenden Kombinationen aus jeweils mindestens einer der pro Copolymer angeführten Struktureinheiten, ist bevorzugt in dem erfindungsgemäßen Mittel enthalten:

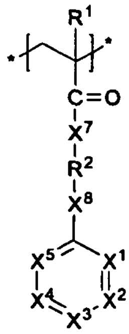 (IV) und 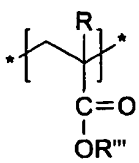 (M1) (P3)

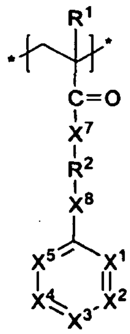 (IV) und 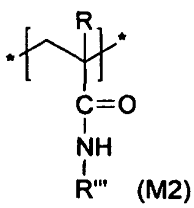 (M2) (P6)

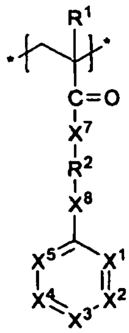 (IV) und 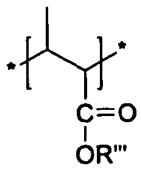 (M3) (P9)

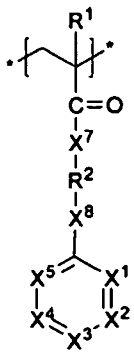 (IV) und 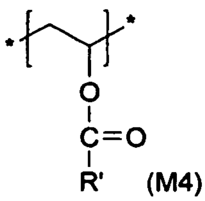 (M4) (P12)

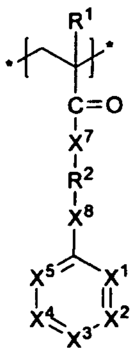 (IV) und 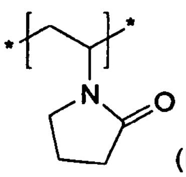 (M5) (P15)

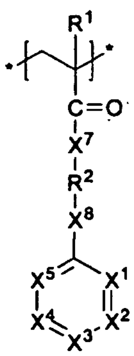 (IV) und 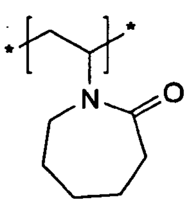 (M6) (P18)

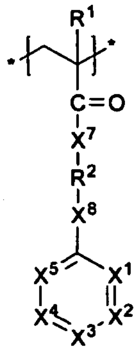 (IV) und 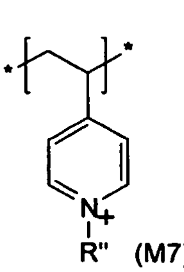 (M7) (P21)

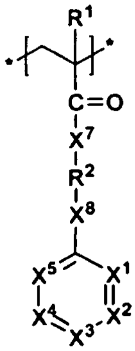 (IV) und 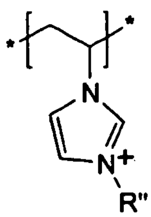 (M8) (P24)

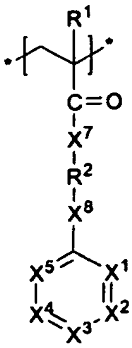 (IV) und 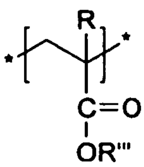 (M1) und 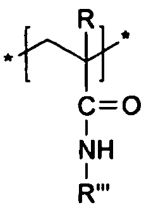 (M2) (P27)

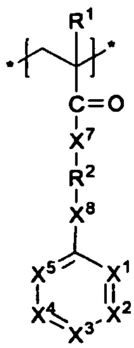 (IV) und 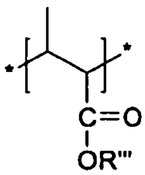 (M3) und 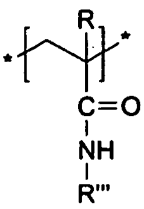 (M2) (P30)

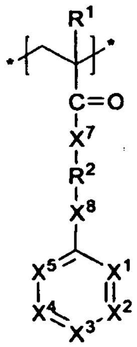 (IV) und 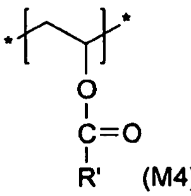 (M4) und 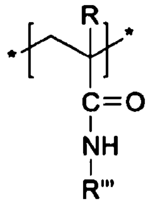 (M2) (P33)

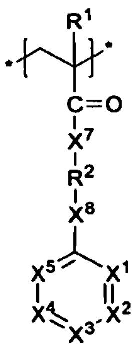 (IV) und 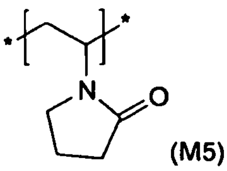 (M5) und 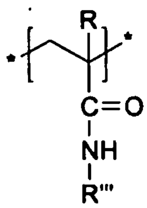 (M2) (P36)

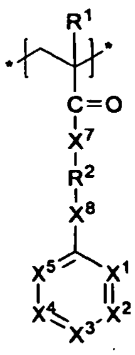 (IV) und 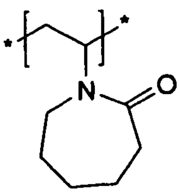 (M6) und 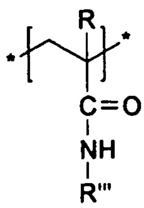 (M2) (P39)

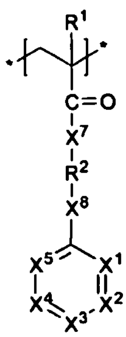 (IV) und 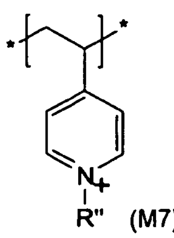 (M7) und 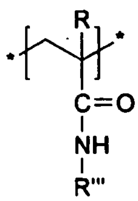 (M2) (P42)

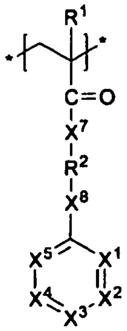 (IV) und 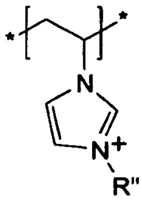 (M8) und 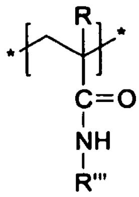 (M2) (P45)

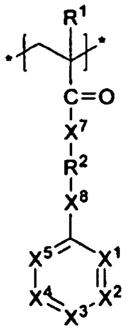 (IV) und 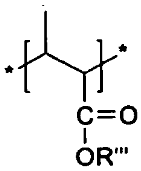 (M3) und 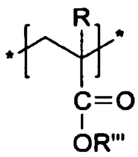 (M1) (P48)

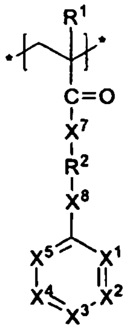 (IV) und 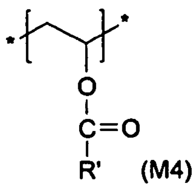 (M4) und 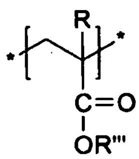 (M1) (P51)

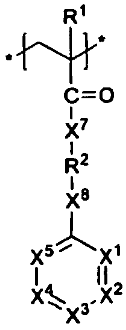 (IV) und 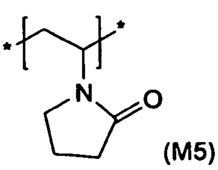 (M5) und 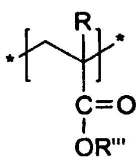 (M1) (P54)

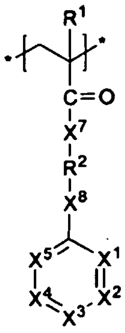 (IV) und 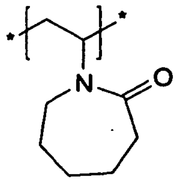 (M6) und 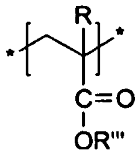 (M1) (P57)

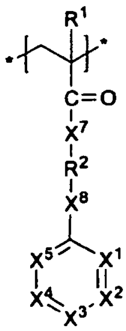 (IV) und 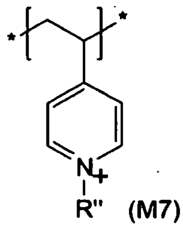 (M7) und 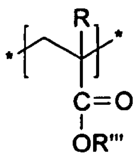 (M1) (P60)

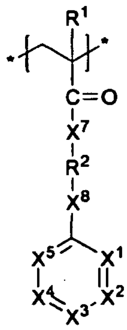 (IV) und 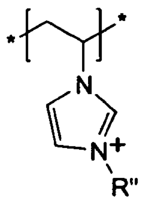 (M8) und 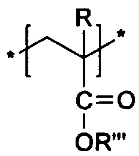 (M1) (P63)

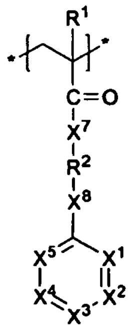 (IV) und 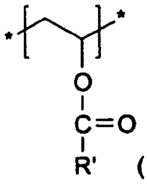 (M4) und 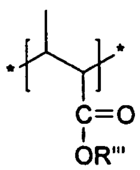 (M3) (P66)

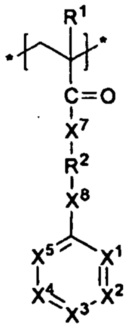 (IV) und 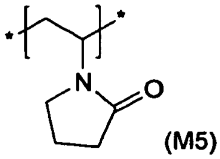 (M5) und 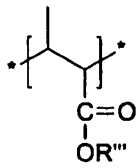 (M3) (P69)

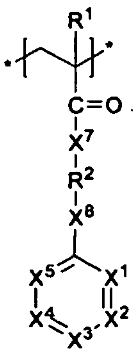 (IV) und 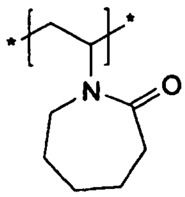 (M6) und 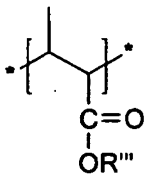 (M3) (P72)

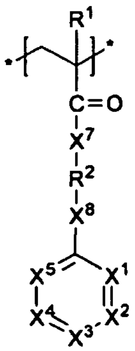 (IV) und 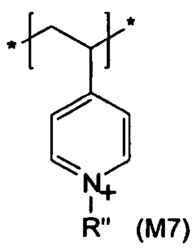 (M7) und 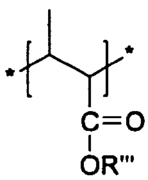 (M3) (P74)

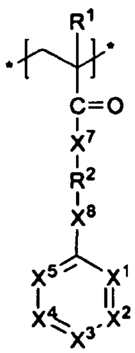 (IV) und 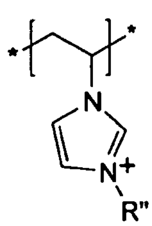 (M8) und 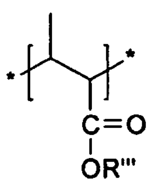 (M3) (P77)

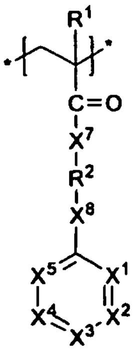 (IV) und 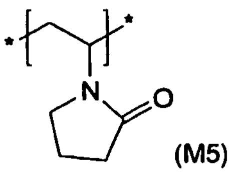 (M5) und 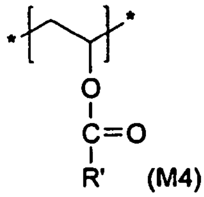 (M4) (P80)

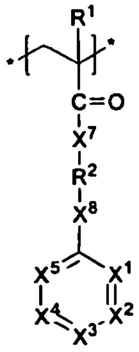 (IV) und 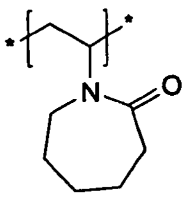 (M6) und 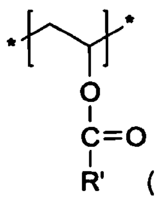 (M4) (P83)

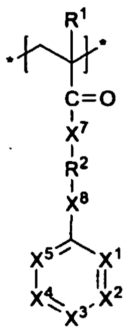 (IV) und 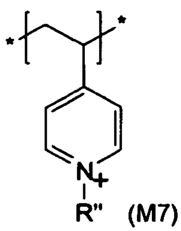 (M7) und 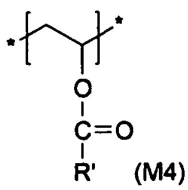 (M4) (P86)

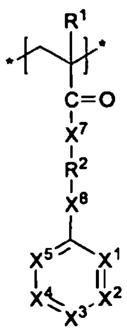 (IV) und 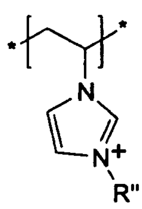 (M8) und 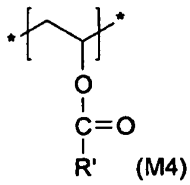 (M4) (P89)

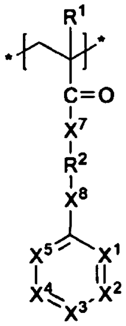 (IV) und 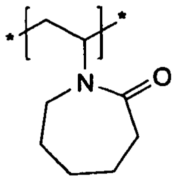 (M6) und 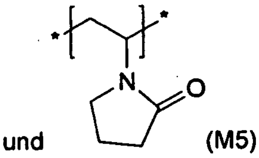 (M5) (P92)

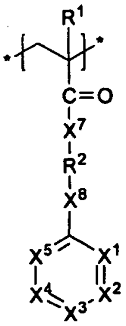 (IV) und 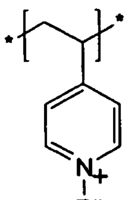 (M7) und 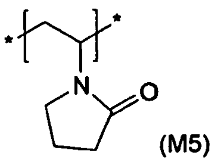 (M5) (P95)

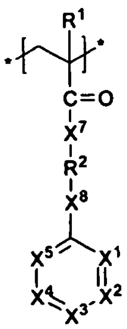 (IV) und 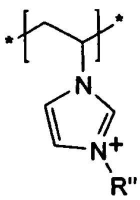 (M8) und 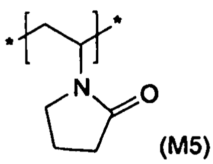 (M5) (P98)

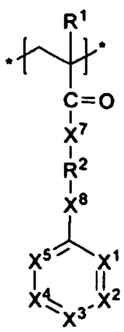 (IV) und 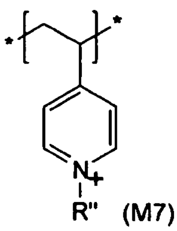 (M7) und 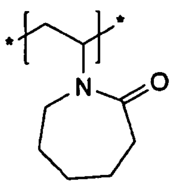 (M6) (P101)

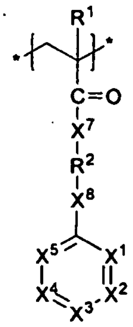 (IV) und 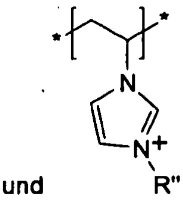 (M8) und 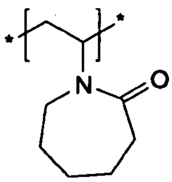 (M6) (P104)

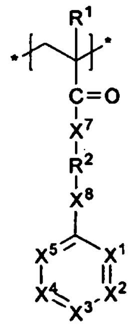 (IV) und 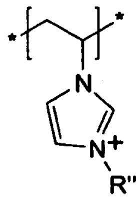 (M8) und 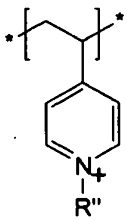 (M7) (P107)

wobei die variablen Reste die zuvor im Rahmen der Offenbarung der jeweiligen Struktureinheiten der Formeln (I), (II), (IV) und (M1) bis (M8) definierten Bedeutungen oder die Bedeutungen der bevorzugten Ausführungsformen dieser Struktureinheiten einnehmen (*vide supra*).

In den Polymeren gemäß Formeln (P1) bis P(107) steht die Formel (IV) bevorzugt für mindestens eine der Struktureinheiten der Formeln (I-1) bis (I-16), (I-19) bis (I-26), insbesondere für mindestens eine der Struktureinheiten der Formeln (I-1), (I-2), (I-5), (I-6), (I-19), (I-20), (I-20), (I-23) und (I-24).

Ganz besonders bevorzugt sind die Copolymere, enthaltend die nachstehenden Kombinationen aus jeweils mindestens einer der pro Copolymer angeführten Struktureinheiten:

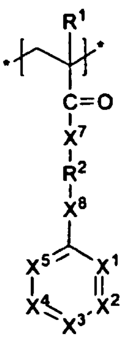 und 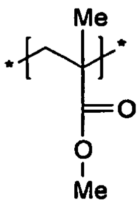 (P121)

(IV) (M1-15)

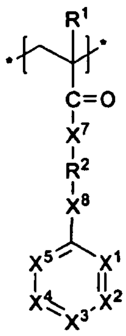 und 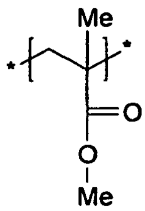 und 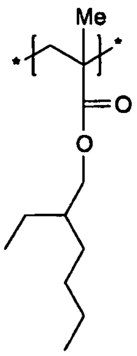 (P122)

(IV) (M1-15) (M1-22)

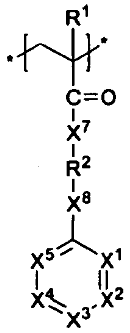 und 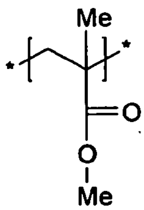 und 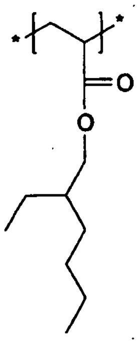 (P122a)

(IV) (M1-15) (M1-8)

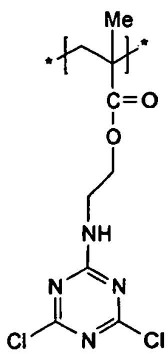 und 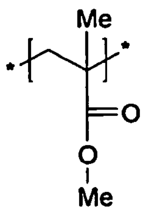 (P134)

(I-1) (M1-15)

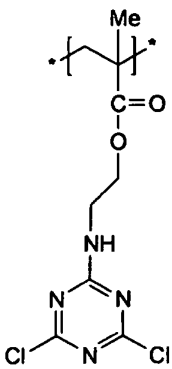 und 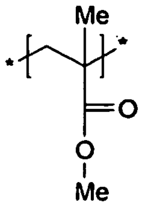 und 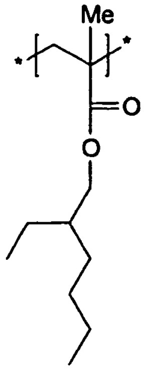 (P135)

(I-1) (M1-15) (M1-22)

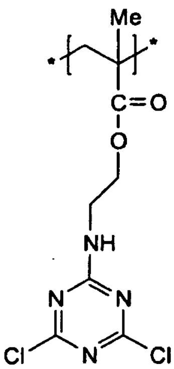 und 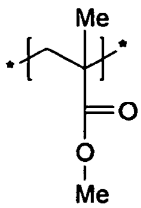 und 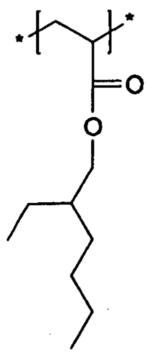 (P135a)

(I-1) (M1-15) (M1-8)

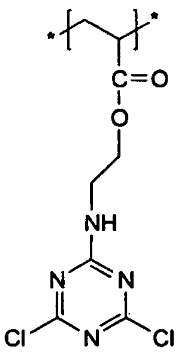 und 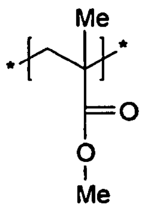 (P147)

(I-2) (M1-15)

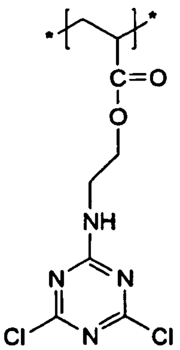 und 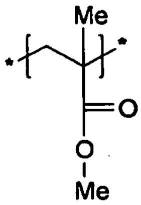 und 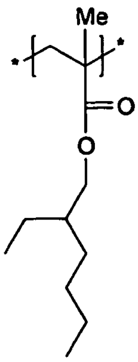 (P148)

(I-2) (M1-15) (M1-22)

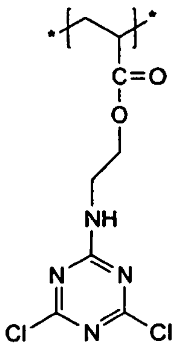 und 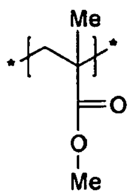 und 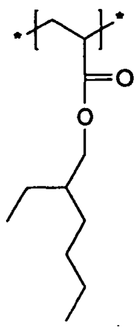 (P148a)

(I-2) (M1-15) (M1-8)

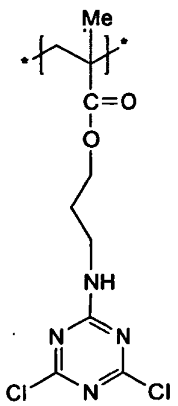 und 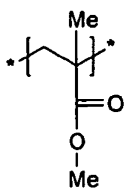 (P160)

(I-5) (M1-15)

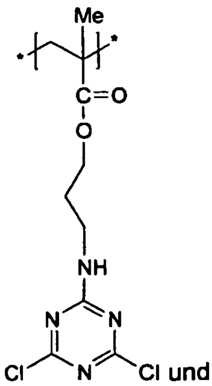 und 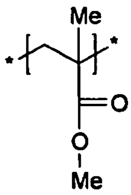 und 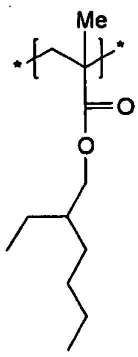 (P161)

(I-5) (M1-15) (M1-22)

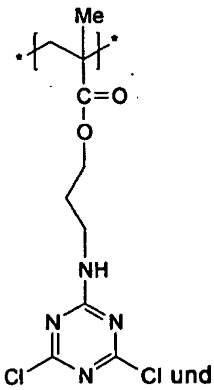 und 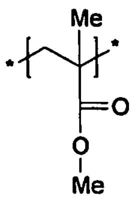 und 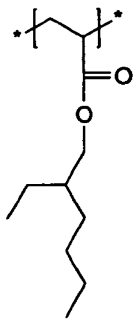 (P161a)

(I-5) (M1-15) (M1-8).

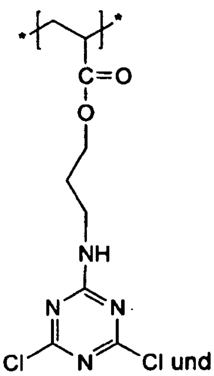 und 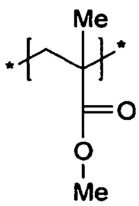 (P173)

(I-6) (M1-15)

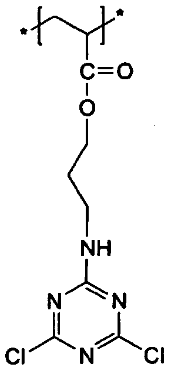 und 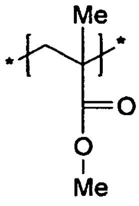 und 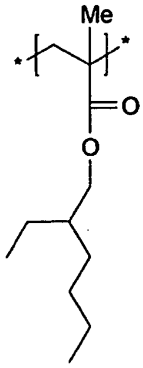 (P174)

(I-6) (M1-15) (M1-22)

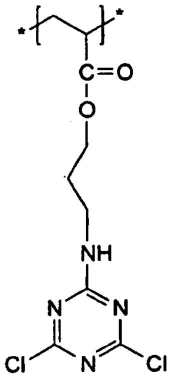 und 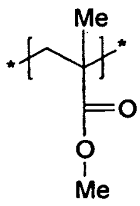 und 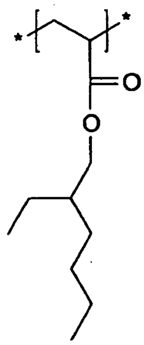 (P174a)

(I-6) (M1-15) (M1-8)

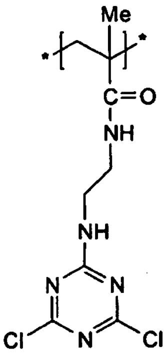 und 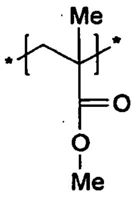 (P186)

(I-9) (M1-15)

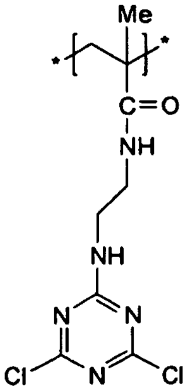 und 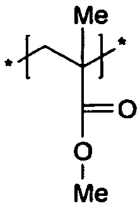 und 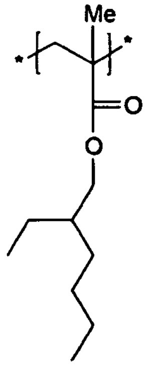

(I-9) (M1-15) (M1-22)

(P187)

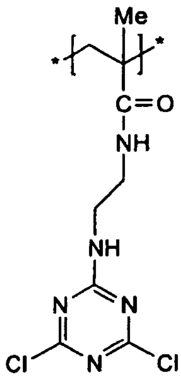 und 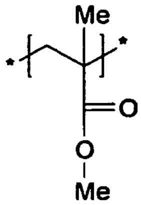 und 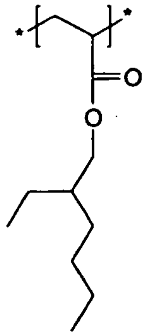

(I-9) (M1-15) (M1-8)

(P187a)

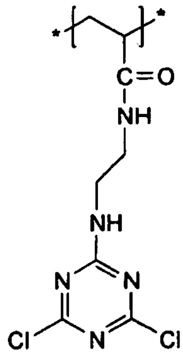 und 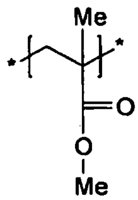

(I-10) (M1-15)

(P199)

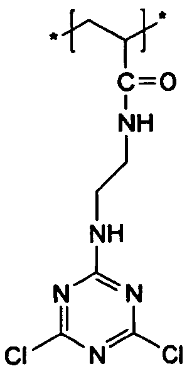 und 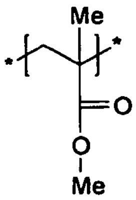 und 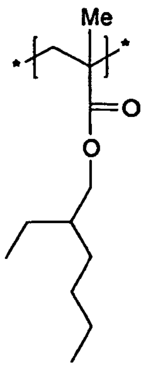 (P200)

(I-10) (M1-15) (M1-22)

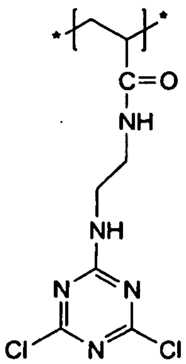 und 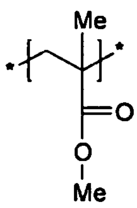 und 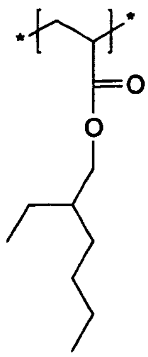 (P200a)

(I-10) (M1-15) (M1-8)

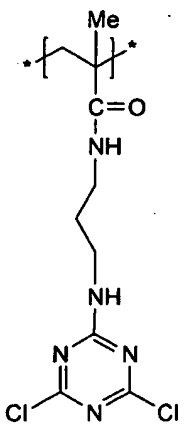 und 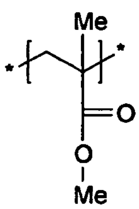 (P212)

(I-13) (M1-15)

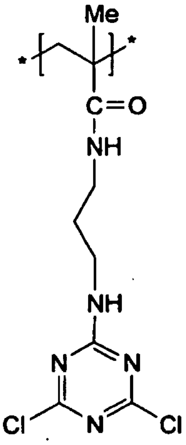 und 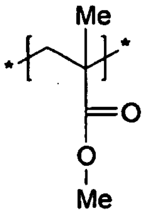 und 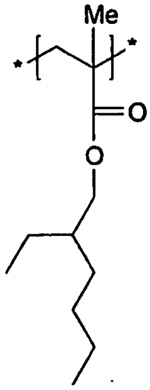 (P213)

(I-13) (M1-15) (M1-22)

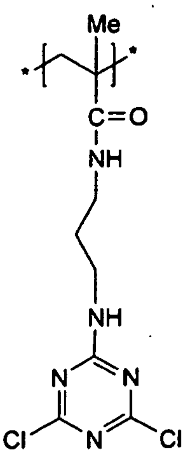 und 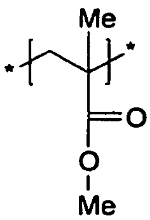 und 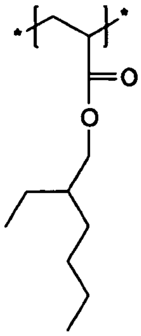 (P213a)

(I-13) (M1-15) (M1-8)

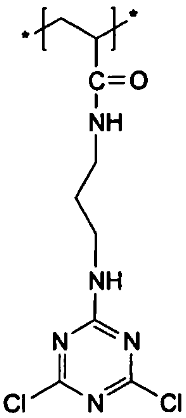 und 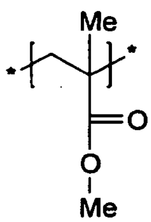 (P225)

(I-14) (M1-15)

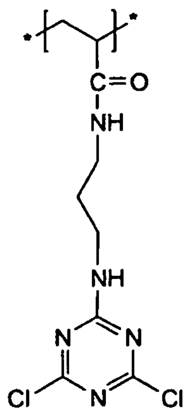 und 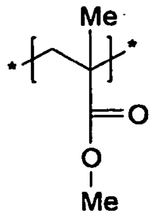 und 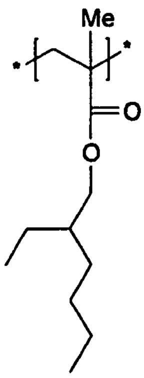 (P226)

(I-14) (M1-15) (M1-22)

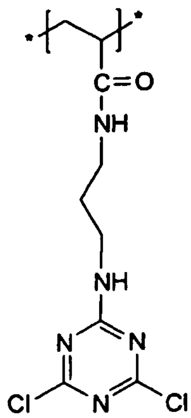 und 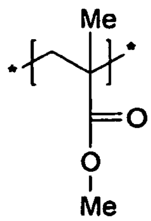 und 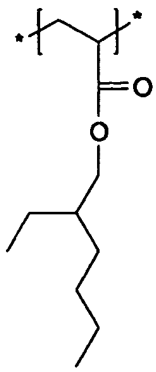 (P226a)

(I-14) (M1-15) (M1-8)

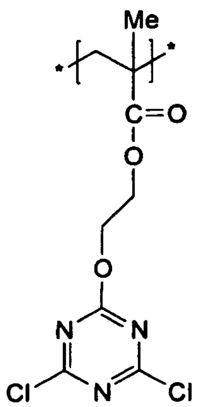 und 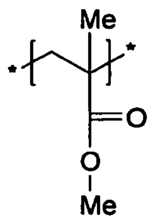 (P238)

(I-19) (M1-15)

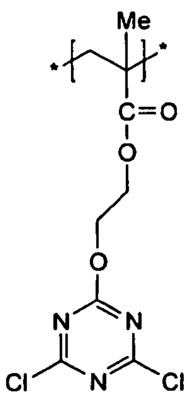 und 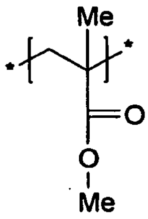 und 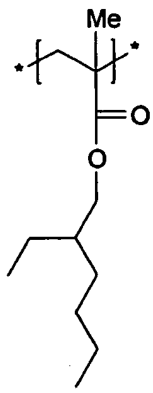

(I-19) (M1-15) (M1-22) (P239)

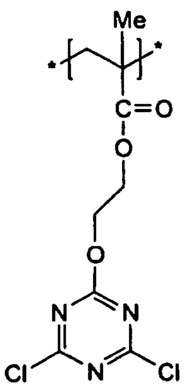 und 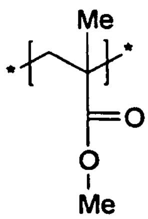 und 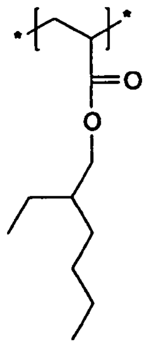

(I-19) (M1-15) (M1-8) (P239a)

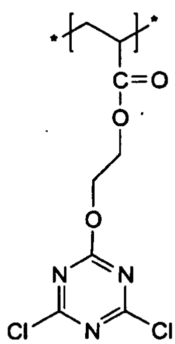 und 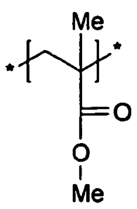

(I-20) (M1-15) (P251)

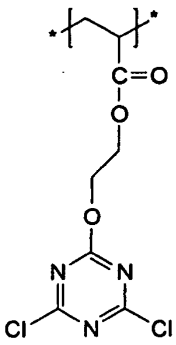 und 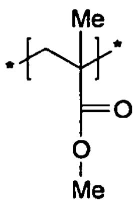 und 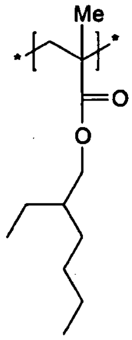 (P252)

(I-20) (M1-15) (M1-22)

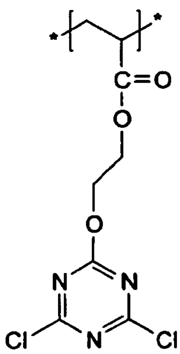 und 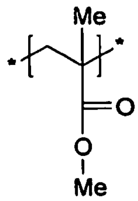 und 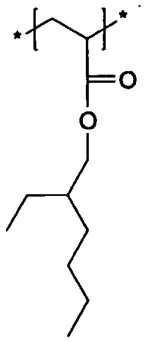 (P252a)

(I-20) (M1-15) (M1-8)

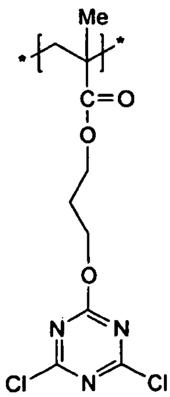 und 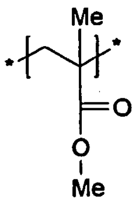 (P265)

(I-23) (M1-15)

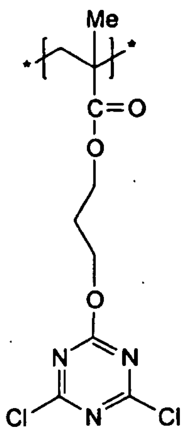 und 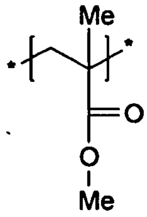 und 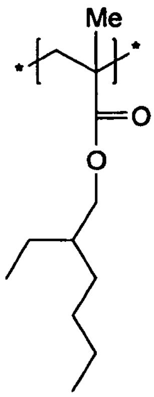 (P266)

(I-23) (M1-15) (M1-22)

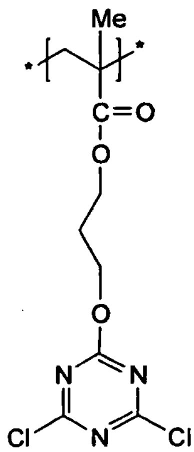 und 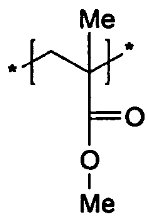 und 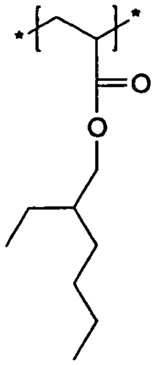 (P266a)

(I-23) (M1-15) (M1-8)

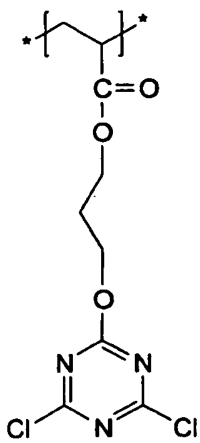 und 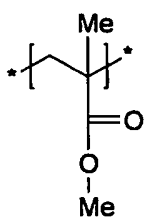 (P278)

(I-24) (M1-15)

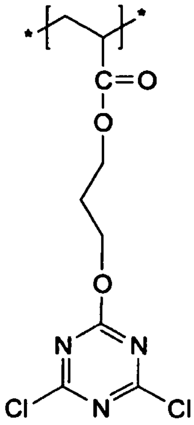 und 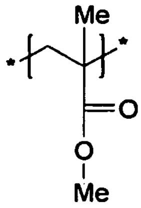 und 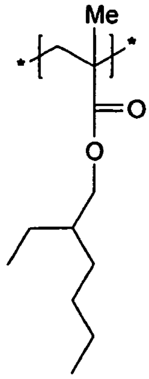

(I-24) (M1-15) (M1-22) (P279)

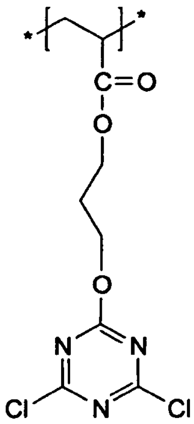 und 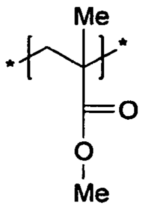 und 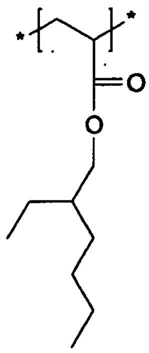

(I-24) (M1-15) (M1-8) (P279a)

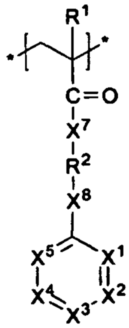 und 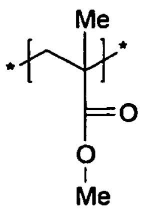 und 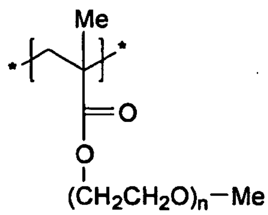

(IV) (M1-15) (P295)

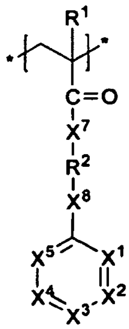 und 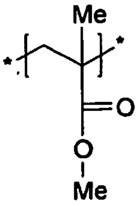 und 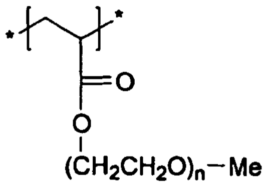

(IV) (M1-15) (P296)

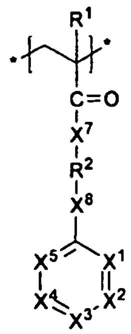 und 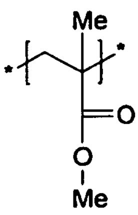 und 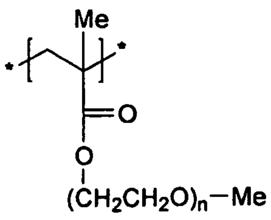 und 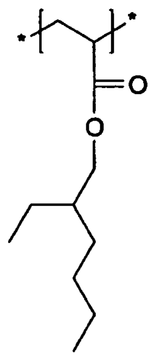

(IV) (M1-15) (M1-8) (P297)

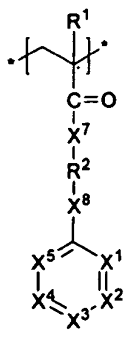 und 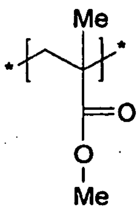 und 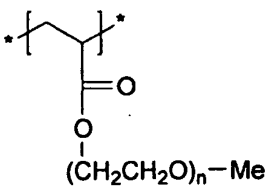 und 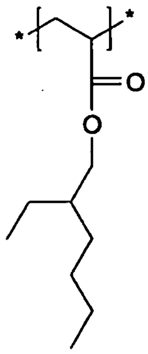

(IV) (M1-15) (M1-8) (P298)

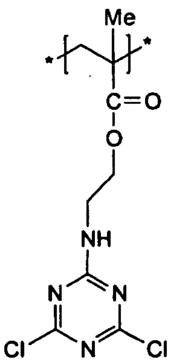 und 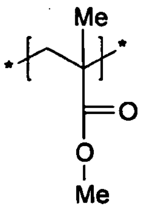 und 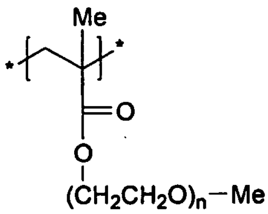

(I-1) (M1-15) (P299)

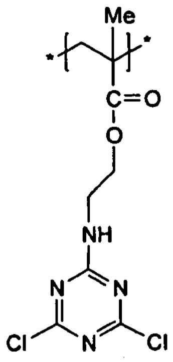 und 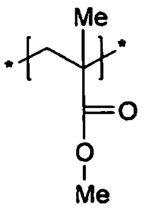 und 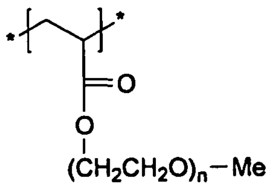

(I-1) (M1-15) (P300)

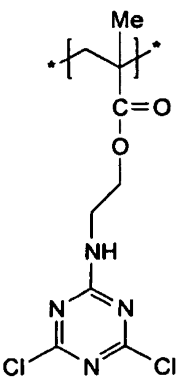 und 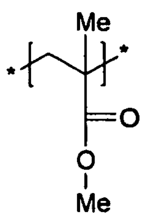 und 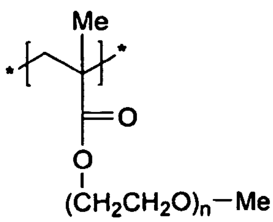 und 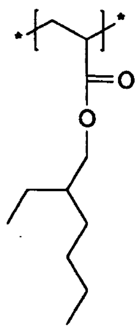

(I-1) (M1-15) (M1-8) (P301)

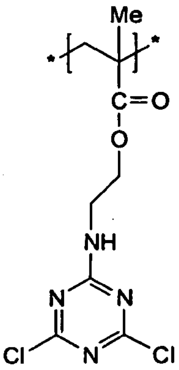 und 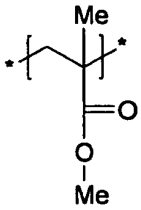 und 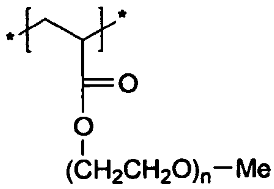 und 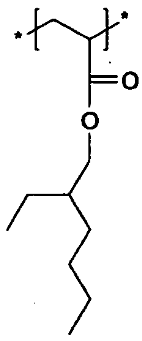

(I-1) (M1-15) (M1-8) (P302)

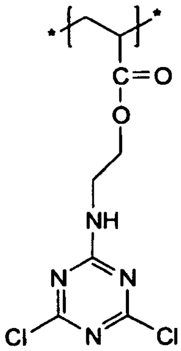 und 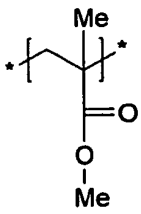 und 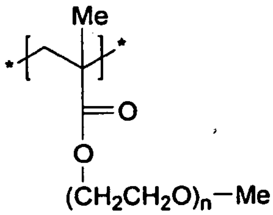

(I-2) (M1-15) (P303)

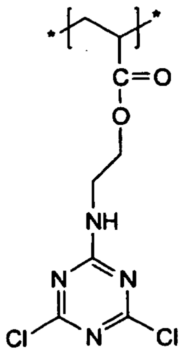 und 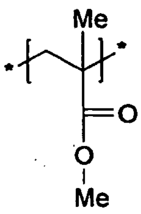 und 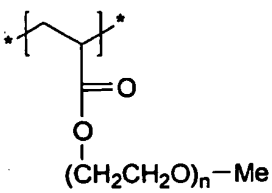

(I-2) (M1-15) (P304)

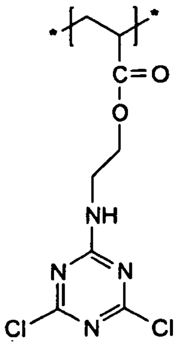 und 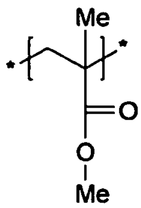 und 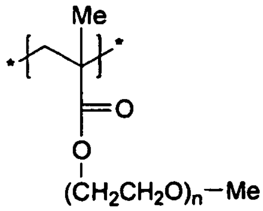 und 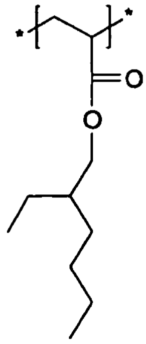

(I-2) (M1-15) (M1-8) (P305)

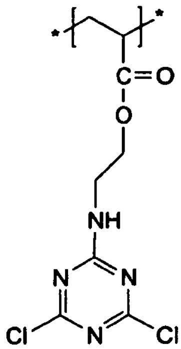 und 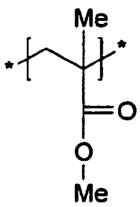 und 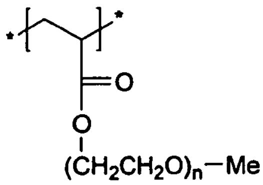 und 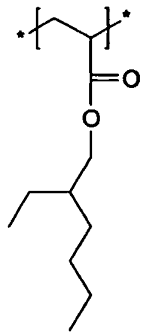

(I-2) (M1-15) (M1-8) (P306)

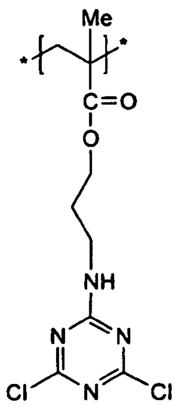 und 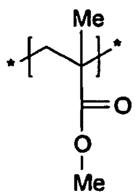 und 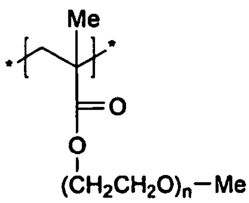

(I-5) (M1-15) (P307)

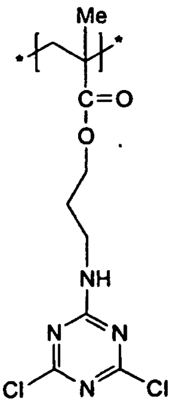 und 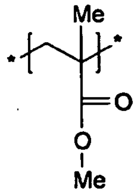 und 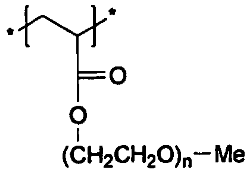

(I-5) (M1-15) (P308)

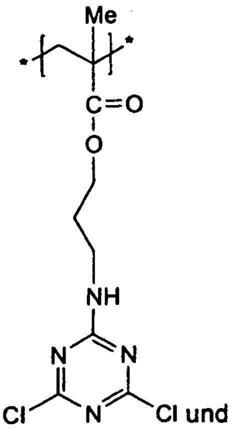 und 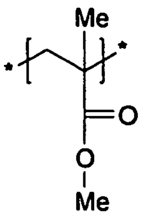 und 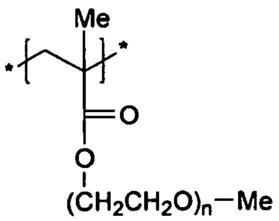 und 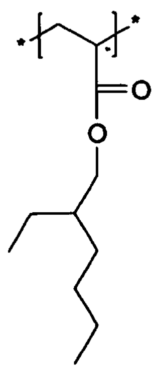

(I-5) (M1-15) (M1-8) (P309)

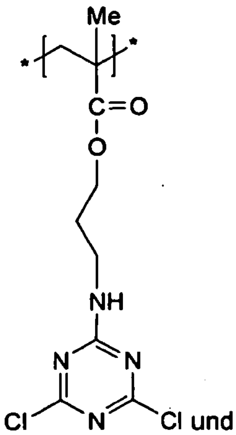 und 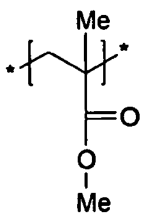 und 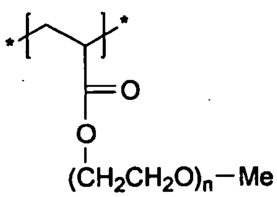 und 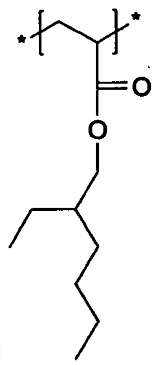

(I-5) (M1-15) (M1-8) (P310)

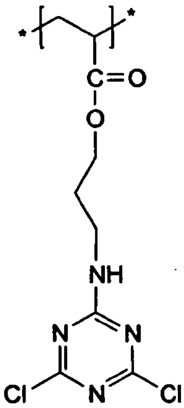 und 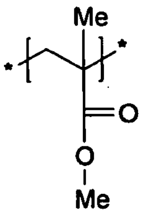 und 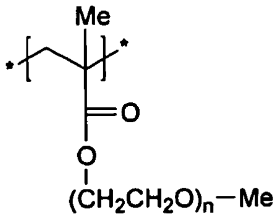

(I-6) (M1-15) (P311)

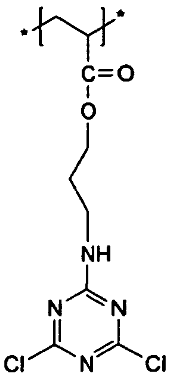 und 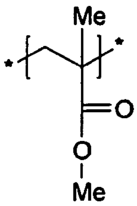 und 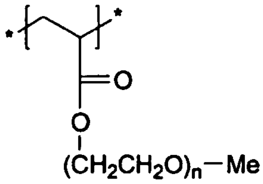

(I-6) (M1-15) (P312)

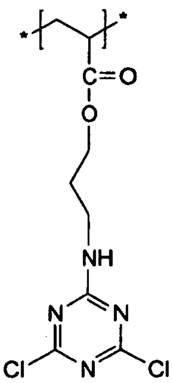 und 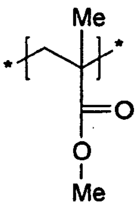 und 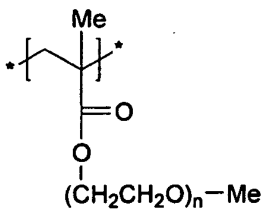 und 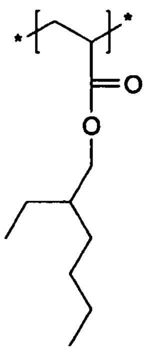

(I-6) (M1-15) (M1-8) (P313)

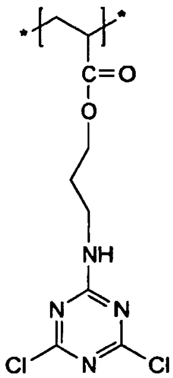 und 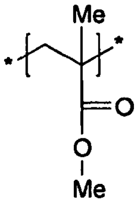 und 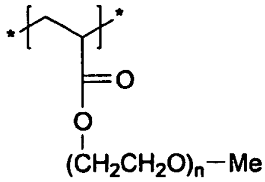 und 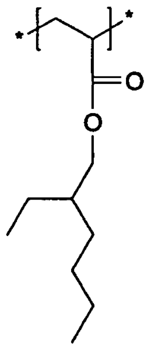

(I-6) (M1-15) (M1-8) (P314)

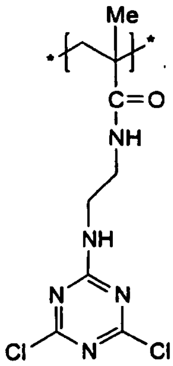 und 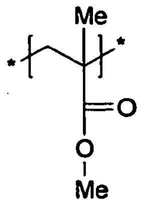 und 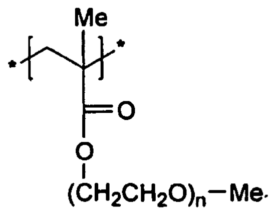

(I-9) (M1-15) (P315)

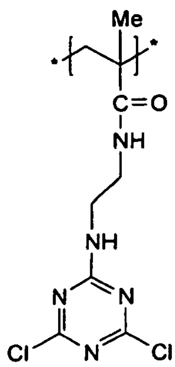 und 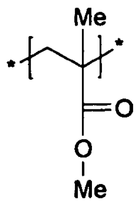 und 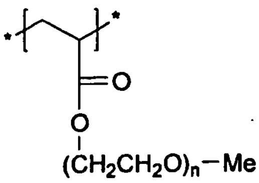

(I-9) (M1-15) (P316)

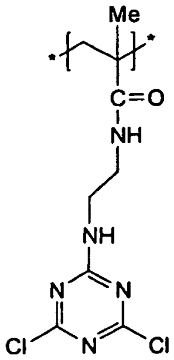 und 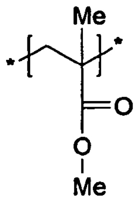 und 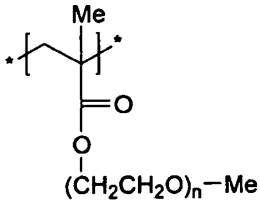 und 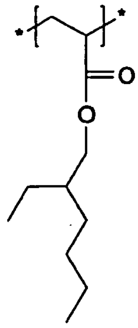

(I-9) (M1-15) (M1-8) (P317)

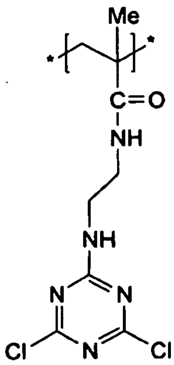 und 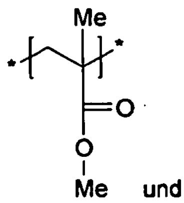 und 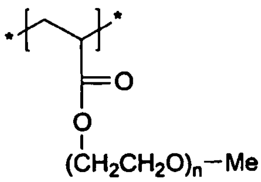 und 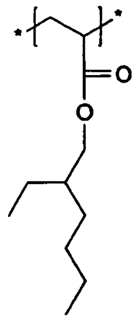

(I-9) (M1-15) (M1-8) (P318)

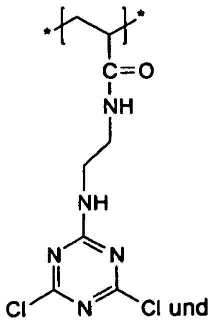 und 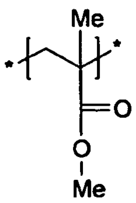 und 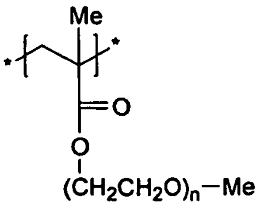

(I-10) (M1-15) (P319)

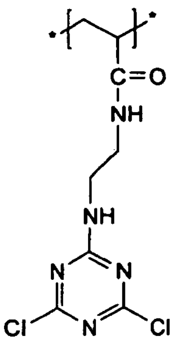 und 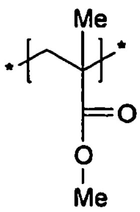 und 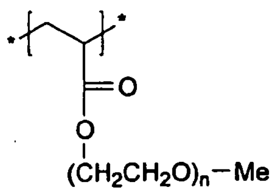

(I-10) (M1-15) (P320)

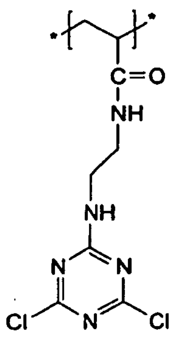 und 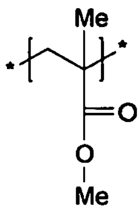 und 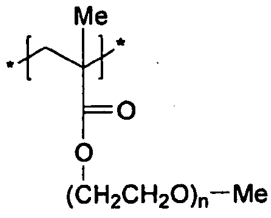 und 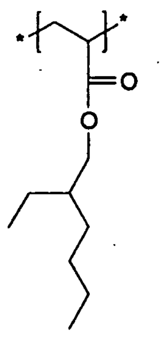

(I-10) (M1-15) (M1-8) (P321)

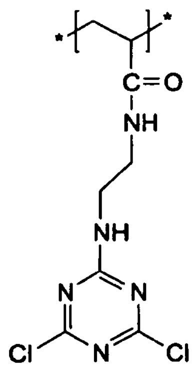 und 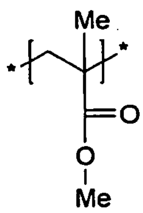 und 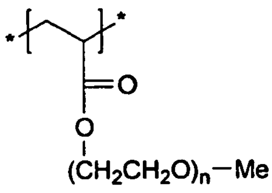 und 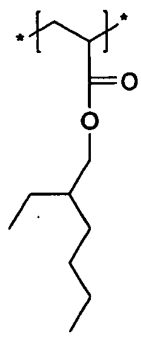

(I-10) (M1-15) (M1-8) (P322)

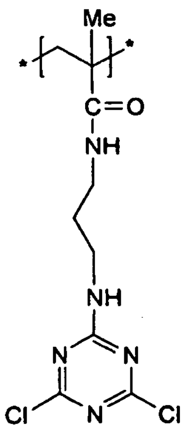 und 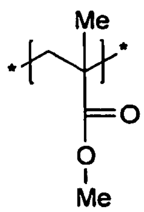 und 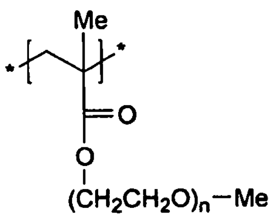

(I-13) (M1-15) (P323)

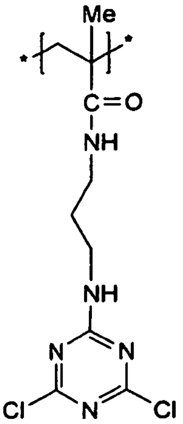 und 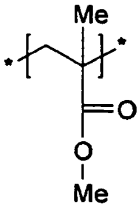 und 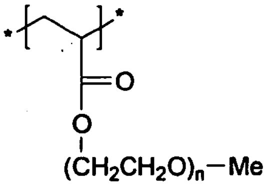

(I-13) (M1-15) (P324)

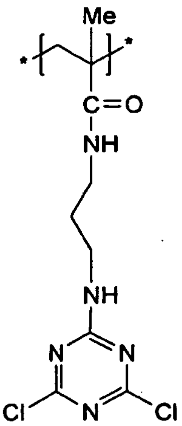 und 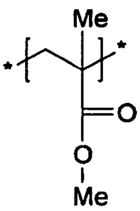 und 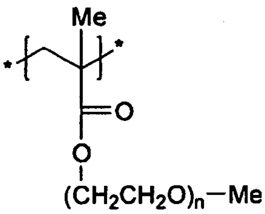 und 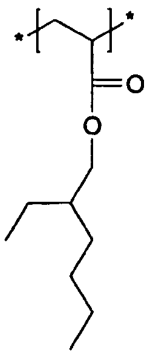

(I-13) (M1-15) (M1-8) (P325)

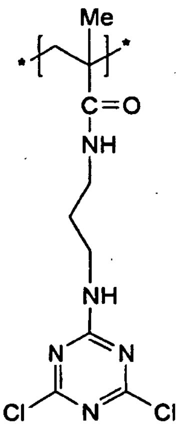 und 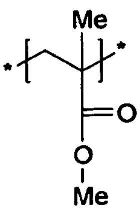 und 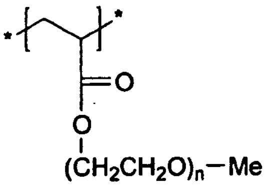 und 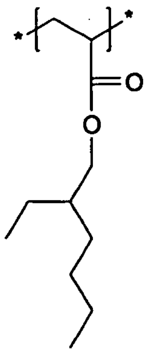

(I-13) (M1-15) (M1-8) (P326)

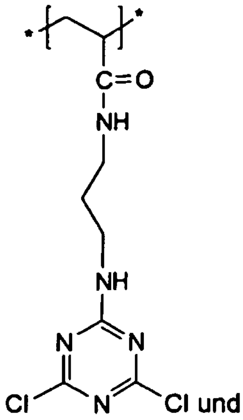 und 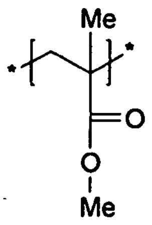 und 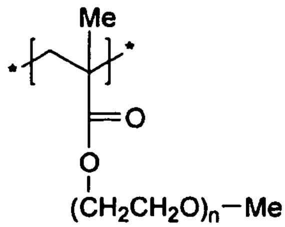

(I-14) (M1-15) (P327)

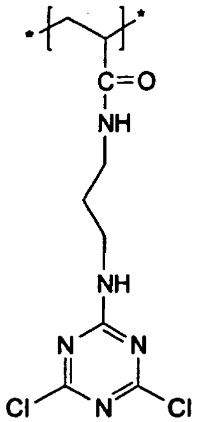 und 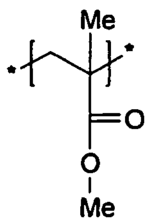 und 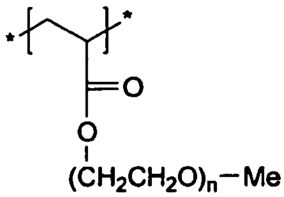

(I-14) (M1-15) (P328)

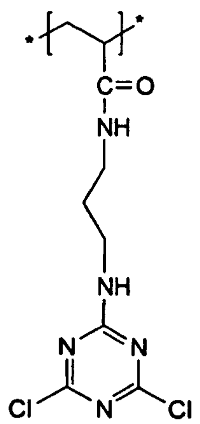 und 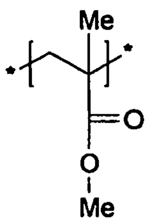 und 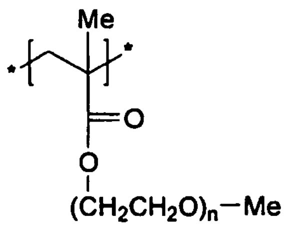 und 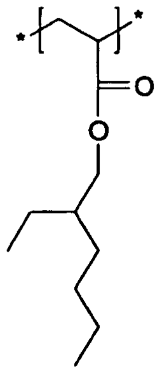

(I-14) (M1-15) (M1-8) (P329)

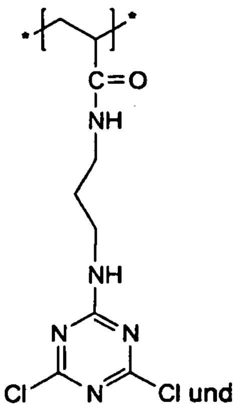 und 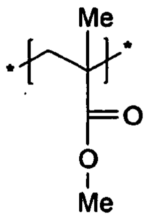 und 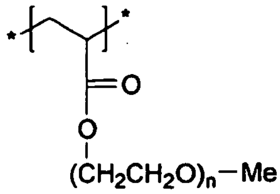 und 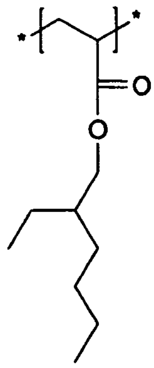

(I-14) (M1-15) (M1-8) (P330)

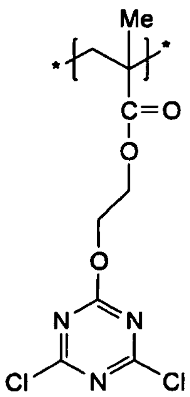 und 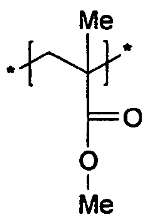 und 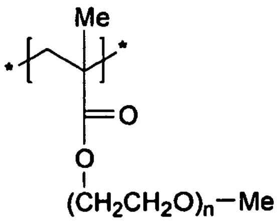

(I-19) (M1-15) (P331)

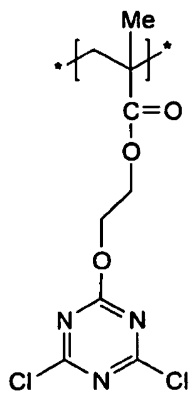 und 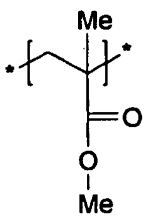 und 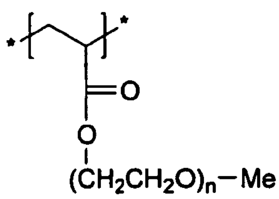

(I-19) (M1-15) (P332)

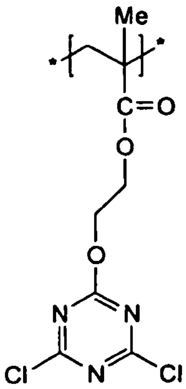 und 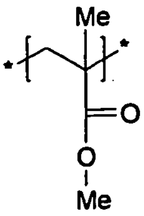 und 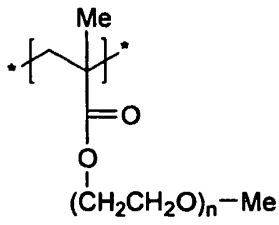 und 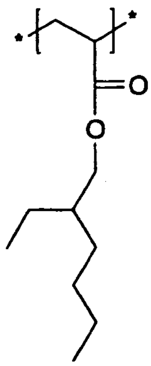

(I-19) (M1-15) (M1-8) (P333)

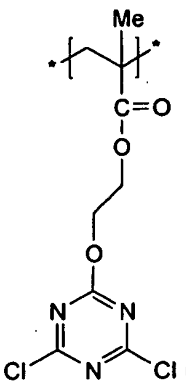 und 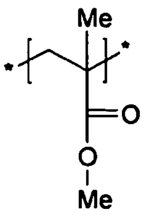 und 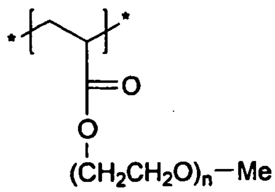 und 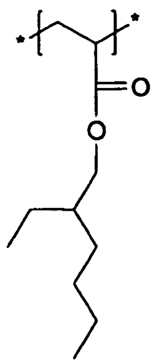

(I-19) (M1-15) (M1-8) (P334)

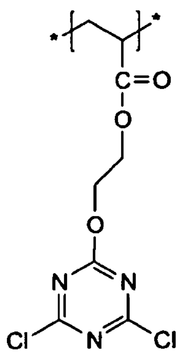 und 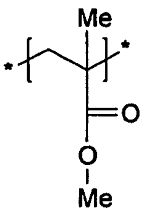 und 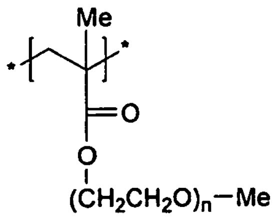

(I-20) (M1-15) (P335)

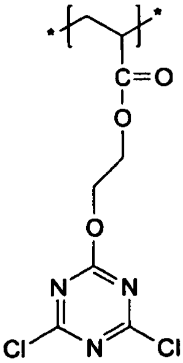 und 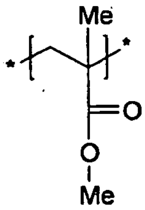 und 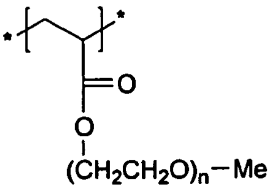

(I-20) (M1-15) (P336)

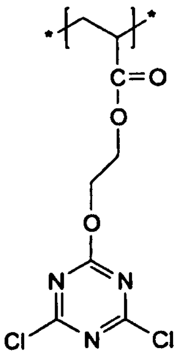 und 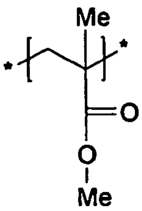 und 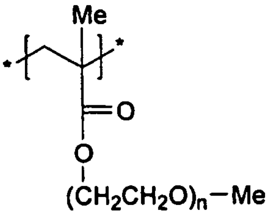 und 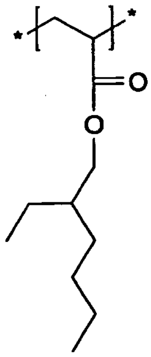

(I-20) (M1-15) (M1-8) (P337)

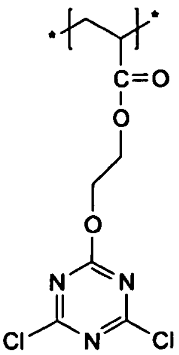 und 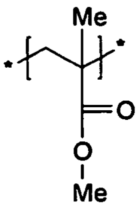 und 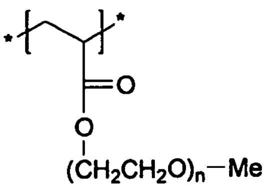 und 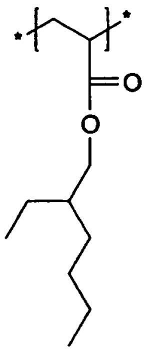

(I-20) (M1-15) (M1-8) (P338)

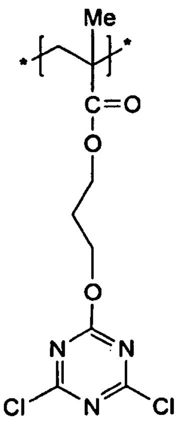 und 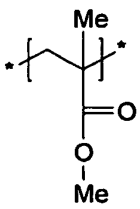 und 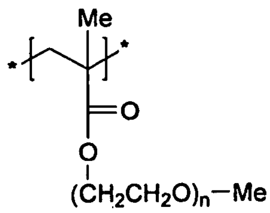

(I-23) (M1-15) (P339)

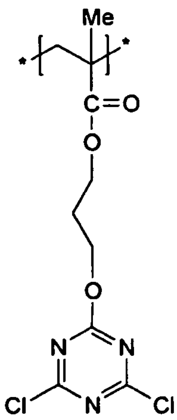 und 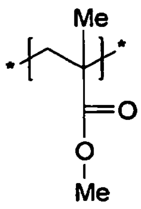 und 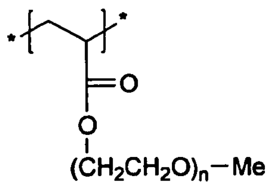

(I-23) (M1-15) (P340)

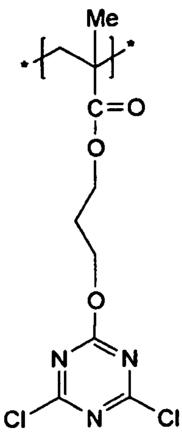 und 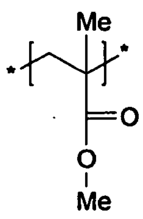 und 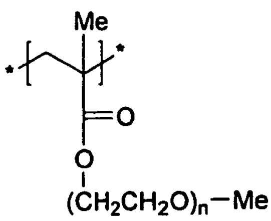 und 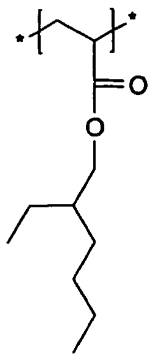

(I-23) (M1-15) (M1-8) (P341)

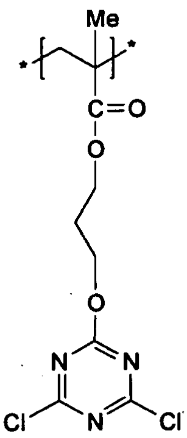 und 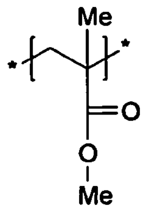 und 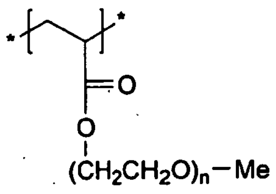 und 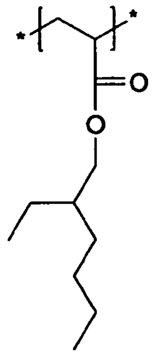

(I-23) (M1-15) (M1-8) (P342)

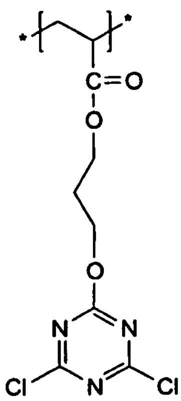 und 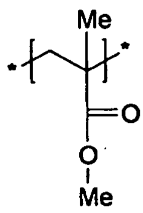 und 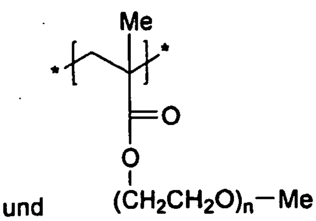

(I-24) (M1-15) (P343)

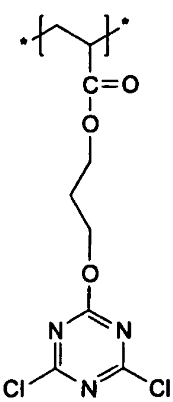 und 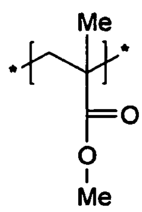 und 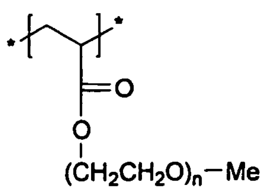

(I-24) (M1-15) (P344)

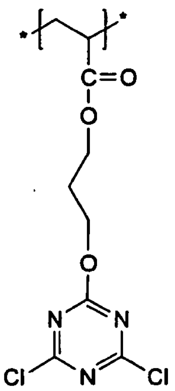 und 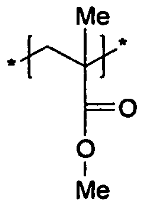 und 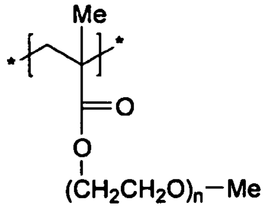 und 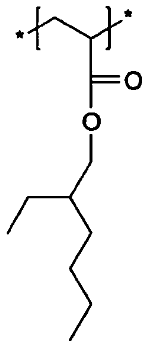

(I-24) (M1-15) (M1-8) (P345)

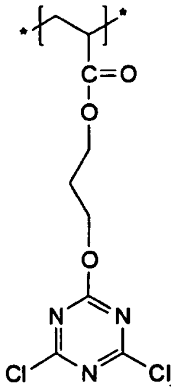 und 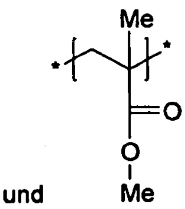 und 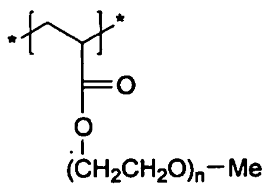 und 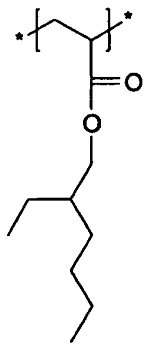

(I-24) (M1-15) (M1-8) (P346)

wobei die variablen Reste die zuvor in der jeweiligen Struktureinheit der Formel (IV) definierten Bedeutungen, bzw. die Bedeutungen aller bevorzugten Ausführungsformen dieser Struktureinheiten einnehmen und n für eine ganze Zahl von 1 bis 60, insbesondere von 10 bis 40, steht

Die Polymere mit der Struktureinheit der Formel (IV) können gemeinsam mit mindestens einem der Katalysatoren, ausgewählt aus der Gruppe, die gebildet wird aus tertiären Aminen, Alkalicarbonat (insbesondere Natriumcarbonat), $Zn^{2+}$ , $Al^{3+}$, $Ag^{+}$, $Cu^{2+}$. $Cu^{+}$, $Mn^{2+}$, $Fe^{3+}$ und $Fe^{2+}$ in dem erfindungsgemäßen Mittel enthalten sein. Der Einsatz dieser Katalysatoren beschleunigt die Fixierungsreaktion bzw. setzt deren Aktivierungsenergie herab.

Der Träger des erfindungsgemäßen Mittels ist ein kosmetischer Träger. Das Polymer mit der/den Struktureinheit (en) der Formel (IV) wird in diesem Träger gelöst oder darin dispergiert In der dispergierten Ausführungsform, liegt das Polymer mit der/den Struktureinheit(en) der Formel (IV) in einem Lösemittel oder einer wasserhaltigen Phase gelöst oder als Feststoff dispergiert vor, oder es wird in einer Ölphase gelöst oder als Feststoff dispergiert, wobei diese Ölphase wiederum in einer wasserhaltigen Phase dispergiert bzw. emulgiert vorliegt.

Solche Träger sind beispielsweise Cremes, Emulsionen, Gele oder auch tensidhaltige Lösungen, wie beispielsweise Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in dem Träger gelöst wird.

Die erfindungsgemäßen Mittel können zusätzlich weitere Inhaltsstoffe enthalten. Dabei ist es bevorzugt, wenn die erfindungsgemäßen Mittel, enthaltend Polymere mit der Struktureinheit der Formel (IV) nur solche zusätzlichen Inhaltsstoffe enthalten, die nicht mit den Halogenmethanylylidengruppen der Formel (IV) reagieren. Dies gilt insbesondere für Polymere, die zusätzlich in den erfindungsgemäßen Mitteln enthalten sein können. Die unreaktiven, bevorzugten Inhaltsstoffe sollten insbesondere keine Hydroxy- oder primären oder sekundären Aminogruppen tragen. Sollten dennoch zusätzliche Inhaltsstoffe enthalten sein, die mit den Halogenmethanylylidengruppen der Formel (IV) reagieren, so ist es wiederum bevorzugt, die erfindungsgemäßen Polymere derart in das Mittel einzuarbeiten, dass eine Reaktion mit den Halogenmethanylgruppen vermieden wird. Dies gelingt beispielsweise durch Einarbeitung in inerte Emulsionströpfchen oder durch Verkapselung mit inertem Material als Schutzhülle.

Es ist bevorzugt, wenn die erfindungsgemäßen Mittel zusätzlich mindestens ein Silikon enthalten. Diese Silikone sind, wenn sie in den erfindungsgemäßen Mitteln zugegen sind, vorzugsweise in Mengen von 0,05 bis 5 Gew.%, vorzugsweise von 0,2 bis 5 Gew.%, jeweils bezogen auf das anwendungsbereite Mittel, enthalten. Wenn flüchtige Silikone, d.h. Silikone mit einem Siedepunkt von kleiner 100°C unter Standardbedingungen, als Lösemittel eingesetzt werden, so sind diese flüchtigen Silikone bevorzugt in Mengen von bis zu 80 Gew.-% bezogen auf das anwendungsbereite Mittel, enthalten

Weiterhin ist es erfindungsgemäß bevorzugt, wenn die erfindungsgemäßen Mittel zusätzlich mindestens einen Fettstoff enthalten.

Unter Fettstoffen sind Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen.

Die Einsatzmenge beträgt dabei 0,1 - 15 Gew.%, bezogen auf das gesamte Mittel. Bevorzugt beträgt die Menge 0,5-10 Gew.%, wobei ganz besonders vorteilhaft Mengen von 1 -.5 Gew.% sein können.

Als Fettalkohole können eingesetzt werden gesättigte, ein- oder mehrfach ungesättigte, verzweigte oder unverzweigte Fettalkohole mit $C_6$ - $C_{30}$-, bevorzugt $C_{10}$ - $C_{22}$- und ganz besonders bevorzugt $C_{12}$ - $C_{22}$- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole werden in Mengen von 0,1-30 Gew.%, bezogen auf die gesamte Zubereitung, bevorzugt in Mengen von 0,1 -20 Gew.-% eingesetzt.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau. Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Zu den natürlichen und synthetischen kosmetischen Ölkörpern, welche die Wirkung des erfindungsgemäßen Zusammensetzung steigern können, sind beispielsweise zu zählen:

- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether.

Die als Handelsprodukte erhältlichen Verbindungen 1,3-Di-(2-ethyl-hexyl)-cyclohexan (Cetiol® S) und Di-n-octylether (Cetiol® OE) können bevorzugt sein.

- Esteröle. Unter Esterölen sind zu verstehen die Ester von C6 - C30 - Fettsäuren mit C2 - C30 - Fettalkoholen. Bevorzugt sind die Monoester der Fettsäuren mit Alkoholen mit 2 bis 24 C-Atomen.
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin, Fettsäurepartialglyceride, das sind Monoglyceride, Diglyceride und deren technische Gemische.

Die Einsatzmenge der natürlichen und synthetischen kosmetischen Ölkörper in den erfindungsgemäß verwendeten Mitteln beträgt üblicherweise 0,1 bis 30 Gew.%, bezogen auf das gesamte Mittel, bevorzugt 0,1 bis 20 Gew.-%, und insbesondere 0,1 bis 15 Gew.-%.

Die Gesamtmenge an 01- und Fettkomponenten in den erfindungsgemäßen Mitteln beträgt üblicherweise 0,1 bis 50 Gew.-%, bezogen auf das gesamte Mittel. Mengen von 0,1 bis 30 Gew.-% sind erfindungsgemäß bevorzugt.

Nicht nur wenn in einer Ausführungsform der Erfindung die Zusammensetzung in Form einer Emulsion vorliegt, können die Emulsion stabilisierende Polymere vorteilhafter Weise verwendet werden. Die Stabilisierung einer Emulsion kann auf verschiedenen Wegen erreicht werden. Beispielsweise können Polymere verwendet werden, welche die Viskosität der Emulsion beeinflussen. Polymere können die Viskosität von wäßrigen und nicht-wäßrigen Phasen in kosmetischen Zubereitungen beeinflussen. In wäßrigen Phasen beruht ihre die Viskosität beeinflussende Funktion auf ihrer Löslichkeit in Wasser oder ihrer hydrophilen Natur. Sie werden sowohl in tensidischen als auch in emulsionsförmigen Systemen angewendet.

Wenn die erfindungsgemäße Zusammensetzung in Form einer Emulsion formuliert werden soll, können emulsionsstabilisierende Polymere vorteilhaft als die Viskosität beeinflussende Polymere mitverwendet werden. Hierunter sind Polymere zu verstehen, welche den Aufbau und die Stabilisierung von Emulsionen (O/W und W/O sowie multiple Emulsionen) wesentlich unterstützen. Tenside und Emulgatoren sind selbstverständlich die wesentlichen Bestandteile, jedoch tragen die stabilisierenden Polymere durch eine positive Beeinflussung der kontinuierlichen oder der dispersen Phase zu einer Verringerung der Koaleszenz der emulgierten Tröpfchen bei. Diese positive Beeinflussung kann auf einer elektrischen Abstoßung und einer Erhöhung der Viskosität oder einer Filmbildung auf der Tröpfchenoberfläche beruhen.

Eine weitere Möglichkeit zur Beeinflussung der Viskosität der erfindungsgemäßen Zusammensetzung ist die Verdickung der nicht-wäßrigen Phase, der Lipidphase. Hierzu werden Polymere eingesetzt, welche nicht wasserlöslich aber kompatibel mit Lipiden sind. Sie werden auch zur Gelbildung von kosmetischen Mitteln mit hohen Lipidanteilen verwendet.

In einer weiteren bevorzugten Ausführungsform enthalten die erfindungsgemäßen Mittel zusätzlich Emulgatoren. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Weiterführende Definitionen und Eigenschaften von Emulgatoren finden sich in "H.-D. Dörfler, Grenzflächen- und Kolloidchemie, VCH Verlagsgesellschaft mbH. Weinheim, 1994". Erfindungsgemäß verwendbare Emulgatoren sind beispielsweise

- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsaureester, Fettsäurealkanolamide und Fettsäureglucamide,
- $C_8$-$C_{22}$-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine, Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.%, insbesondere 0,5 - 15 Gew.%, bezogen auf das gesamte Mittel.

Bevorzugt können die erfindungsgemäßen Zusammensetzungen mindestens einen nichtionogenen Emulgator mit einem HLB-Wert von 3 bis 20, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 5

- 18 können erfindungsgemäß besonders bevorzugt sein. Ganz besonders bevorzugt können Emulgatoren mit einem HLB - Wert von 10 bis 15 sein.

Ebenfalls als vorteilhaft hat sich die Kombination aus Polymere(n) mit der/den Struktureinheit(en) der Formel (IV) und Tensiden erwiesen. In einer weiteren bevorzugten Ausführungsform enthalten die erfindungsgemäß verwendeten Mittel Tenside. Unter dem Begriff Tenside werden grenzflächenaktive Substanzen, die an Ober- und Grenzflächen Adsorptionsschichten bilden oder in Volumenphasen zu Mizellkolloiden oder lyotropen Mesophasen aggregieren können, verstanden. Man unterscheidet Aniontenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem , hydrophoben Rest eine positiv geladene hydrophile Gruppe aufweisen, und nichtionische Tenside, welche keine Ladungen sondern starke Dipolmomente aufweisen und in wäßriger Lösung stark hydratisiert sind. Weitergehende Definitionen und Eigenschaften von Tensiden finden sich in "H.-D.Dörfler, Grenzflächen- und Kolloidchemie, VCH Verlagsgesellschaft mbH. Weinheim, 1994". Die zuvor wiedergegebene Begriffsbestimmung findet sich ab S. 190 in dieser Druckschrift.

Als anionische Tenside (E1) eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,

- lineare und verzweigte Fettsäuren mit 8 bis 30 C-Atomen (Seifen),
- Ethercarbonsäuren der Formel $R-O-(CH_2-CH_2O)CH_2-COOH$, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel $R-O(CH_2-CH_2O)_xOSO_3H$, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I).

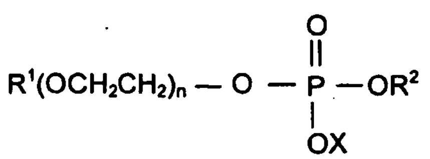 (E1-I)

in der $R^1$ bevorzugt für einen aliphatischen Kohlenwasserstoffrest mit 8 bis 30 Kohlenstoffatomen, $R^2$ für Wasserstoff, einen Rest $(CH_2CH_2O)_nR^1$ oder X, n für Zahlen von 1 bis 10 und X für Wasserstoff, ein Alkali- oder Erdalkalimetall oder $NR^3R^4R^5R^6$, mit $R^3$ bis $R^6$ unabhängig voneinander stehend für Wasserstoff oder einen $C_1$ bis $C_4$ - Kohlenwasserstoffrest, steht,

- sulfatierte Fettsäurealkylenglykolester der Formel (E1-II)

$$R_7CO(AlkO)_nSO_3M \qquad (E1\text{-}II)$$

in der $R^7CO$- steht für einen linearen oder verzweigten, aliphatischen, gesättigten und/oder ungesättigten Acylrest

mit 6 bis 22 C-Atomen, Alk für $CH_2CH_2$, $CHCH_3CH_2$ und/oder $CH_2CHCH_3$, n für Zahlen von 0,5 bis 5 und M für ein Kation steht, wie sie in der DE-OS 197 36 906.5 beschrieben sind,

- Monoglyceridsulfate und Monoglyceridethersulfate der Formel (E1-III)

$$CH_2O(CH_2CH_2O)_x - COR^8$$
$$|$$
$$CHO(CH_2CH_2O)_yH$$
$$|$$
$$CH_2O(CH_2CH_2O)_z - SO_3X$$

**(E1-III)**

in der $R^8CO$ für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Paimitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze.

- Amidethercarbonsäuren wie sie in der EP 0 690 044 beschrieben sind,
- Kondensationsprodukte aus $C_8$ bis $C_{30}$ - Fettalkoholen mit Proteinhydrolysaten und/oder Aminosäuren und deren Derivaten, welche dem Fachmann als Eiweissfettsäurekondensate bekannt sind, wie beispielsweise die Lamepon® - Typen, Gluadin® - Typen, Hostapon® KCG oder die Amisoft® - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside (E2) werden solche oberflächenaktiven Verbindungen bezeichnet, die im Molekül mindestens eine quartäre Ammoniumgruppe und mindestens eine $-COO^{(-)}$- oder - $SO3^{(-)}$ -Gruppe tragen. Besonders geeignete zwitterionische Tenside sind die sogenannten Betaine wie die N-Alkyl-N,N-dimethylammonium-glycinate, beispielsweise das Kokosalkyldimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Beispiele für Betaine umfassen C8- bis C18-Alkylbetaine wie Cocodimethylcarboxymethylbetain, Lauryldimethylcarboxymethylbetain, Lauryldimethylalphacarboxyethylbetain, Cetyldimethylcarboxymethylbetain, Oleyldimethylgammacarboxypropylbetain und Lauryl-bis(2-hydroxypropyl)alphacarboxyethylbetain; $C_8$- bis $C_{18}$-Sulfobetaine wie Cocodimethylsulfopropylbetain, Stearyldimethylsulfopropylbetain, Lauryldimethylsulfoethylbetain, Laurylbis-(2-hydroxyethyl)sulfopropylbetain; die Carboxylderivate des Imidazols, die $C_8$- bis $C_{18}$-Alkyldimethylammoniumacetate, die $C_8$- bis $C_{18}$-Alkyldimethylcarbonylmethylammoniumsalze sowie die $C_8$- bis $C_{18}$- Fettsäurealkylamidobetaine wie beispielsweise das Kokosfettsäureamidopropylbetain, welches beispielsweise in Form einer 30%igen wäßrigen Lösung unter der Handelsbezeichnung Tego® Betain L7 von der Firma Goldschmidt AG vertrieben wird und das N-Kokosfettsäureamidoethyl-N- [2-(carboxymethoxy)ethyl]- glycerin (CTFA-Name: Cocoamphocarboxyglycinate), welches zum Beispiel in Form einer 50%igen wäßrigen Lösung unter der Handelsbezeichnung Miranol® C2M von der Firma Miranol Chemical Co. Inc. vertrieben wird. Ein bevorzugtes zwitterionisches Tensid ist das unter der INCI-Bezeichnung Cocamidopropyl Betaine bekannte Fettsäureamid-Derivat.

Unter ampholytischen Tensiden (E3) werden solche oberflächenaktiven Verbindungen verstanden, die außer einer $C_8$ - $C_{24}$ - Alkyl- oder -Acylgruppe im Molekül mindestens eine freie Aminogruppe und mindestens eine -COOH- oder -S03H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C12 - C18 - Acylsarcosin.

Nichtionische Tenside (E4) enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, mit einem Methyl- oder $C_2$ - $C_6$ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,

- C12-C30-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (E4-I)

$$R^1CO\text{-}(OCH_2CHR^2)_wOR^3 \qquad \text{(E4-1)}$$

in der $R^1CO$ für einen linearen oder verzweigten, gesättigten und/oder ungesättigten Acylrest mit 6 bis 22 Kohlenstoffatomen, R2 für Wasserstoff oder Methyl, $R^3$ für lineare oder verzweigte Alkylreste mit 1 bis 4 Kohlenstoffatomen und w für Zahlen von 1 bis 20 steht,

- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsaureester und Anlagerungsprodukte von Ethylenoxid an Zuckerfettsaureester,
- Anlagerungsprodukte von Ethylenoxid an Fettsäurealkanolamide und Fettamine,
- Pyrrolidonderivate der Formel (E4-II),

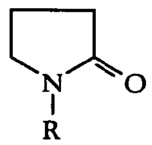

**(E4-II)**

wobei R = Alkyl, Alkenyl, Hydroxyalkyl, Hydroxyalkenyl, Aminoalkyl, Aminoalkenyl, vorzugsweise mit 8 bis 30 C-Atomen, bevorzugt 8 bis 22 C-Atome, ist.

Das Pyrrolidonderivat gemäß der allgemeinen Formel (E4-II) kann auch in Form seines Salzes, vorzugsweise quartemisiert mit Dimethylsulfat (DMS) oder als Alkylhalogenid, verwendet werden.

Das Pyrrolidonderivat (E4-II) wird in einer Menge von 0,01 bis 30 Gew.% bezogen auf das gesamte Mittel, bevorzugt in Mengen von 0,05 bis 20 Gew.% und ganz besonders bevorzugt in Mengen von 0,05 bis 10 Gew.% verwendet.

- Zuckertenside vom Typ der Alkyl- und Alkenyloligoglykoside gemäß Formel (E4-III),

$$R^4O\text{-}[G]_p \qquad \text{(E4-III),}$$

in der R4 für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht.

- Zuckertenside vom Typ der Fettsäure-N-alkylpolyhydroxyalkylamide, ein nichtionisches Tensid der Formel (E4-IV),

$$R^5CO\text{-}NR^6\text{-}[Z] \qquad \text{(E4-IV)}$$

in der $R^5CO$ für einen aliphatischen Acylrest mit 6 bis 22 Kohlenstoffatomen, R6 für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsaure erwiesen. Zubereitungen mit hervorragenden Eigenschaften werden ebenfalls erhalten, wenn sie als nichtionische Tenside Fettsäureester von ethoxyliertem Glycerin enthalten. Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre

lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin sind ganz besonders bevorzugte nichtionische Tenside die Zuckertenside. Diese können in den erfindungsgemäß verwendeten Mitteln bevorzugt in Mengen von 0,1 - 20 Gew.-%, bezogen auf das gesamte Mittel, enthalten sein. Mengen von 0,5 - 15 Gew.-% sind bevorzugt, und ganz besonders bevorzugt sind Mengen von 0,5 - 7,5 Gew.%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlärigen erhält.

Die zuvor genannten Tenside werden bevorzugt in Mengen von 0,1 bis 45 Gew.%, besonders bevorzugt 0,1 bis 30 Gew.% und ganz besonders bevorzugt von 0,1 bis 20 Gew.%, bezogen auf das gesamte erfindungsgemäß verwendete Mittel, eingesetzt.

Ganz besonders bevorzugt sind erfindungsgemäß einsetzbar kationische Tenside (E5). Typische Beispiele für kationische Tenside sind insbesondere Tetraalkylammoniumverbindungen Amidoamine oder aber Esterquats. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyttrimethylammoniumchlorid, Distearyldimethylammoniumchlorid, Lauryldimethylammoniumchlorid, Lauryldimethylbenzylammoniumchlorid, Tricetylmethylammoniumchlorid, Hydroxyethyl Hydroxycetyl Dimmonium Chloride sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten.

Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Die kationischen Tenside (E5) sind in den erfindungsgemäß verwendeten Mitteln bevorzugt in Mengen von 0,05 bis 10 Gew.%, bezogen auf das gesamte Mittel, enthalten. Mengen von 0,1 bis 5 Gew.-% sind besonders bevorzugt.

Kationische, nichtionische, zwitterionische und/oder amphotere Tenside sowie deren Mischungen können erfindungsgemäß bevorzugt sein.

Neben den zuvor beschriebenen kationischen Tensiden sind weiterhin besonders vorteilhaft kationaktive Polymere als haarpflegende Substanzen geeignet. Eine kationaktive Verbindung ist eine Substanz, die auf Grund von kationischen oder kationisierbaren Gruppen, insbesondere primären, sekundären, tertiären oder quaternären Amingruppen eine Substantivität zu menschlichem Haar aufweist. Geeignete kationaktive Stoffe sind ausgewählt aus kationischen Polymeren, Silikonverbindungen mit kationischen oder kationisierbaren Gruppen, kationisch derivatisierten Proteinen oder Proteinhydrolysaten und Betain. Da bis auf die kationischen Proteinderivate alle kationischen Polymere bereits zuvor ausführlich beschrieben wurden, sei auf das zuvor Beschriebene verwiesen.

Kationiserte Proteinhydrolysate zählen zu den kationaktiven Substanzen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann.

Ganz besonders bevorzugt sind die kationischen Proteinhydrolysate und -derivate auf pflanzlicher Basis.

Es ist erfindungsgemäß möglich, dass die Mittel zusätzlich zu dem Polymer mit der/den Struktureinheit(en) der Formel (IV) mindestens ein weiteres filmbildendes und/oder festigendes Polymer (A) enthält.

Das zusätzliche filmbildende und/oder festigende Polymer (A) ist in dem erfindungsgemäßen Mittel vorzugsweise in einer Menge von 3,0 bis 40 Gew.%, besonders bevorzugt von 3,0 bis 30 Gewichtsprozent, ganz besonders bevorzugt in einer Menge von 3,0 bis 20 Gewichtsprozent und am bevorzugtesten in einer Menge von 5,0 bis 10 Gewichtsprozent enthalten. Selbstverständlich können auch mehrere zusätzliche filmbildende und/oder festigende Polymere in dem erfindungsgemäßen Mittel enthalten sein. Dabei können diese filmbildenden und/oder festigenden Polymere sowohl permanent als auch temporär kationisch, anionisch, nichtionisch oder amphoter sein. Weiterhin umfasst

die vorliegende Erfindung auch die Erkenntnis, dass bei der Verwendung von mindestens zwei filmbildenden und/oder festigenden Polymeren diese selbstverständlich unterschiedliche Ladungen aufweisen können. Erfindungsgemäß bevorzugt ein ionisches filmbildendes und/oder festigendes Polymer mit einem amphoteren und/oder nichtionischem filmbildenen und/oder festigenden Polymer gemeinsam verwendet. Auch die Verwendung mindestens zweier gegensätzlich geladener filmbildender und/oder festigender Polymere ist bevorzugt. In letzterem Falle kann eine besondere Ausführungsform wiederum zusätzlich mindestens ein weiteres amphoteres und/oder nichtionisches filmbildendes und/oder festigendes Polymer enthalten.

Geeignete synthetische, filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignet sind z.B. Homopolymere des Vinylcaprolactams, des Vinylpyrrolidons oder des N-Vinylformamids. Weitere geeignete synthetische filmbildende, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine® P 191 von der Firma CHEM-Y, Emmerich, oder Sepigel® 305 von der Firma Seppic vertrieben werden; Polyvinylalkohole, die beispielsweise unter den Handelsbezeichnungen Elvanol® von Du Pont oder Vinol® 523/540 von der Firma Air Products vertrieben werden sowie Polyethylenglykol/Polypropylenglykol-Copolymere, die beispielsweise, unter den Handelsbezeichnungen Ucon® der Union Carbide vertrieben werden. Besonders bevorzugt sind Polyvinylpyrrolidon und Polyvinylpyrrolidon/Vinylacetat Copolymere.

Geeignete natürliche filmbildende Polymere sind z.B. Cellulosederivate, z. B. Hydroxypropylcellulose mit einem Molekulargewicht von 30.000 bis 50.000 g/mol, welche beispielsweise unter der Handelsbezeichnung Nisso SI® von der Firma Lehmann & Voss, Hamburg, vertrieben wird.

Festigende Polymere tragen zum Halt und/oder zum Aufbau des Faservolumens, bzw. bei keratinhaltigen Fasern zur Fülle der Gesamtfrisur bei. Diese sogenannten festigenden Polymere sind gleichzeitig auch filmbildende Polymere und daher generell typische Substanzen für Mittel wie Haarfestiger, Haarschäume, Haarwachse, Haarsprays. Die Filmbildung kann dabei durchaus punktuell sein und nur einige Fasern miteinander verbinden.

Substanzen, welche der Faser, bzw. dem Haar, weiterhin hydrophobe Eigenschaften verleihen, sind hierbei bevorzugt, weil sie die Tendenz der Faser, bzw. des Haares, Feuchtigkeit, also Wasser zu absorbieren, verringern. Dadurch wird das schlaffe Herunterhängen von Fasersträhnen, bzw. Haarsträhnen, vermindert und somit wird ein langanhaltender Formaufbau und -erhalt gewährleistet. Als Testmethode hierfür wird häufig der sogenannte curl-retention - Test angewendet. Diese polymeren Substanzen können weiterhin erfolgreich in leave-on und rinse-off Haarkuren oder Shampoos eingearbeitet werden. Da Polymere häufig multifunktional sind, das heißt mehrere anwendungstechnisch erwünschte Wirkungen zeigen, finden sich zahlreiche Polymere in mehreren auf die Wirkungsweise eingeteilten Gruppen, so auch im CTFA Handbuch. Wegen der Bedeutung gerade der festigenden Polymere sollen diese daher explizit in Form ihrer INCI - Namen aufgelistet werden. In dieser Liste der erfindungsgemäß ganz besonders bevorzugt zu verwendenden Polymere finden sich somit selbstverständlich gerade auch auch die kationischen Polymere wieder.

Ganz besonders bevorzugt sind Acrylates/t-Butylacrylamide Copolymer, Octylacrylamide/Acrylates/Butylaminoethyl Methacrylate Copolymer, Polyurethane-1, Polyvinylcaprolactam und VPNA Copolymer. Insbesondere bevorzugt wird ein den erfindungsgemäßen Mitteln eine Mischung aus Acrylates/t-Butylacrylamide Copolymer und Octylacrylamide/Acrylates/Butylaminoethyl Methacrylate Copolymer eingesetzt.

Die erfindungsgemäßen kationischen Polymere können sowohl festigende und/oder filmbildende und/oder antistatische und/oder avivierende Polymere als auch Polymere mit konditionierenden und/oder verdickenden Eigenschaften sein. Bei den geeigneten kationaktiven Polymeren handelt es sich vorzugsweise um haarfestigende und/oder um faserkonditionierende Polymere. Unter Polymeren sind sowohl natürliche als auch synthetische Polymere, welche kationisch oder amphoter geladen sein können, zu verstehen.

Bevorzugt sind solche Polymere, die eine ausreichende Löslichkeit in Wasser oder Alkohol besitzen, um in dem erfindungsgemäßen Mittel in vollständig gelöster Form vorzuliegen. Die kationische Ladungsdichte beträgt vorzugsweise 1 bis 7 meq/g.

Unter kationischen Polymeren sind Polymere zu verstehen, welche in der Haupt- und/oder Seitenkette eine Gruppe aufweisen, welche "temporär oder "permanent" kationisch sein kann. Als "permanente kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Die kationischen Polymere können Homo- oder Copolymere sein, wobei die quaternären Stickstoffgruppen entweder in der Polymerkette oder vorzugsweise als Substituent an einem oder mehreren der Monomeren enthalten

sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquatemium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium- 11) sowie quaternäre Silikonpolymere bzw. -oligomere wie beispielsweise Silikonpolymere mit quaternären Endgruppen (Quaternium-80).

Von den kationischen Polymeren, die in dem erfindungsgemäßen Mittel enthalten sein können, ist zum Beispiel Vinylpyrrolidon/Dimethylaminoethylmethacrylatmethosulfat Copolymer, das unter den Handelsbezeichnungen Gafquat® 755 N und Gafquat® 734 von der Firma Gaf Co., USA vertrieben wird und von denen das Gafquat® 734 besonders bevorzugt ist, geeignet. Weitere kationische Polymere sind beispielsweise das von der Firma BASF, Deutschland unter dem Handelsnamen luviquat® HM 550 vertriebene Copolymer aus Polyvinylpyrrolidon und Imidazoliminmethochlorid, das von der Firma Calgon/USA unter dem Handelsnamen Merquat® Plus 3300 vertriebene Terpolymer aus Dimethyldiallylammoniumchlorid, Natriumacrylat und Acrylamid und das von der Firma ISP unter dem Handelsnamen Gafquat® HS 100 vertriebene Vinylpyrrolidon/Methacrylamidopropyltrimethylammoniumchlorid Copolymer.

Ein weiteres bevorzugtes kationische Polymer ist ein Homopolymer der allgemeinen Formel (C1-I),

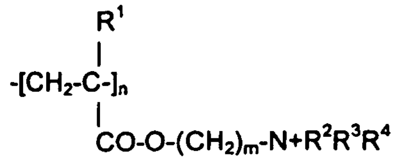 $X^-$ (C1-I)

in der $R^1$= -H oder $-CH_3$ ist, $R^2$, $R^3$ und $R^4$ unabhängig voneinander ausgewählt sind aus $C_{1-4}$-Alkyl-, -Alkenyl- oder -Hydroxyalkylgruppen, m = 1, 2, 3 oder 4, n eine natürliche Zahl und $X^-$ ein physiologisch verträgliches organisches oder anorganisches Anion ist, sowie Copolymere, bestehend im wesentlichen aus den in Formel (C1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt: $R^1$ steht für eine Methylgruppe, $R^2$, $R^3$ und $R^4$ stehen für Methylgruppen, m hat den Wert 2.

Als physiologisch verträgliches Gegenionen $X^-$ kommen beispielsweise Halogenidionen, Sulfationen, Phosphationen, Methösulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (C1-I) enthalten als nichtionogene Monomereinheiten bevorzugt Acrylamid, Methacrylamid, Acrylsäure-C1-4-alkylester und Methacrylsäure-C1-4-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate.

Besonders bevorzugte kationaktive Stoffe sind Chitosan, Chitosansalze und Chitosan-Derivate. Die Chitosanderivate sind ein Beispiel für ein kationisches Polymer, welches ausgeprägte Eigenschaften als Filmbildner hat. Bei den erfindungsgemäß einzusetzenden Chitosanen handelt es sich um vollständig oder partiell deacetylierte Chitine. Zur Herstellung von Chitosan geht man vorzugsweise von dem in den Schalenresten von Krustentieren enthaltenem Chitin aus, welches als billiger und natürlicher Rohstoff in großen Mengen zur Verfügung steht. Das Molekulargewicht des Chitosans kann über ein breites Spektrum verteilt sein, beispielsweise von 20.000 bis ca. 5 Millionen g/mol. Geeignet ist beispielsweise ein niedermolekulares Chitosan mit einem Molekulargewicht von 30.000 bis 70.000 g/mol. Vorzugsweise liegt das Molekulargewicht jedoch über 100.000 g/mol, besonders bevorzugt von 200. 000 bis 700.000 g/mol. Der Deacetylierungsgrad beträgt vorzugsweise 10 bis 99%, besonders bevorzugt 60 bis 99%.

Ein geeignetes Chitosan wird beispielsweise von der Firma Kyowa Oil& Fat, Japan, unter dem Handelsnamen Flonac® vertrieben. Es hat ein Molekulargewicht von 300.000 bis 700.000 g/mol und ist zu 70 bis 80% entacetyliert. Ein bevorzugtes Chitosansalz ist Chitosoniumpyrrolidoncarboxylat, welches beispielsweise unter der Bezeichnung Kytamer® PC von der Firma Amerchol, USA, vertrieben wird. Das enthaltene Chitosan hat ein Molekulargewicht von ca. 200.000 bis 300.000 g/mol und ist zu 70 bis 85% entacetyliert. Als Chitosanderivate kommen quaternierte, alkylierte oder hydroxyalkylierte Derivate, beispielsweise Hydroxyethyl- oder Hydroxybutylchitosan in Betracht. Weitere Chitosanderivate sind unter den Handelsbezeichnungen Hydagen® CMF, Hydagen® HCMF und Chitolam® NB/101 im Handel frei verfügbar.

Die Chitosane oder Chitosanderivate liegen vorzugsweise in neutralisierter oder partiell neutralisierter Form vor. Der Neutralisationsgrad für das Chitosan oder das Chitosanderivat liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie beispielsweise Ameisensäure, Weinsäure, Äpfelsäure, Milchsaure, Zitronensäure, Pyrrolidoncarbonsäure, Salzsäure u.a., von denen die Pyrrolidoncarbonsäure besonders bevorzugt ist.

Weitere geeignete kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Weiterhin können als Polymere amphotere Polymere verwendet werden. Unter dem Begriff amphotere Polymere werden sowohl solche Polymere, die im Molekül sowohl freie Aminogruppen als auch freie -COOH- oder $SO_3H$-Gruppen enthalten und zur Ausbildung innerer Salze befähigt sind, als auch zwitterionische Polymere, die im Molekül quartäre Ammoniumgruppen und $-COO^-$- oder $-SO_3$-Gruppen enthalten, und solche Polymere zusammengefaßt, die -COOH- oder $SO_3H$-Gruppen und quartäre Ammoniumgruppen enthalten.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Bevorzugt eingesetzte amphotere Polymere sind solche Polymerisate, die sich im wesentlichen zusammensetzen aus

(a) Monomeren mit quartären Ammoniumgruppen der allgemeinen Formel (G3-I),

$$R^1\text{-CH=CR}^2\text{-CO-Z-}(C_nH2_n)\text{-N}^{(+)}R^3R^4R^5\ A^{(-)} \qquad \text{(G3-I)}$$

in der $R^1$ und $R^2$ unabhängig voneinander stehen für Wasserstoff oder eine Methylgruppe und $R^3$, $R^4$ und $R^5$ unabhängig voneinander für Alkylgruppen mit 1 bis 4 Kohlenstoffatomen, Z eine NH-Gruppe oder ein Sauerstoffatom, n eine ganze Zahl von 2 bis 5 und $A^{(-)}$ das Anion einer organischen oder anorganischen Säure ist, und

(b) monomeren Carbonsäuren der allgemeinen Formel (G3-II),

$$R^6\text{-CH=CR}^7\text{-COOH} \qquad \text{(G3-II)}$$

in denen $R^6$ und $R^7$ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen $R^3$, $R^4$ und $R^5$ Methylgruppen sind, Z eine NH-Gruppe und $A^{(-)}$ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Schließlich kann die erfindungsgemäße Zusammensetzung insbesondere auf die festigende, avivierende und antistatische Wirkung gezielt beeinflusst werden, wenn anionische Polymere mit formuliert werden. Bei den anionischen Polymeren handelt es sich unter anderem um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomeren sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik®11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythmt und Methylenbisacrylamid zum Einsatz kommen. Ein solches Polymer ist in dem Handelsprodukt Sepigel®305 der Firma SEPPIC enthalten. Die Verwendung dieses Compounds, das neben der Polymerkomponente eine Kohlenwasserstoffmischung (C13-C14-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vemetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze® QM im Handel erhältlich.

Weiterhin erfindungsgemäß geeignete anionische Polymere sind u. a.:

- Vinylacetat/Crotonsäure-Copolymere, wie sie beispielsweise unter den Bezeichnungen Resyn® (NATIONAL STARCH), Luviset® (BASF) und Gafset® (GAF) im Handel sind.
- VinylpyrrolidonNinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymere.
- Acrylsäure/Ethylacrylat/N-tert.Butylacrylamid-Terpolymere, die beispielsweise unter der Bezeichnung Ultrahold® strong (BASF) vertrieben werden.

Eine weitere ganz besonders bevorzugte Gruppe von Polymeren sind Polyurethane. Die Polyurethane bestehen aus mindestens zwei verschiedenen Monomertypen,

- einer Verbindung (V1) mit mindestens 2 aktiven Wasserstoffatomen pro Molekül und
- einem Di- oder Polyisocyanat (V2).

Bei den Verbindungen (V1) kann es sich beispielsweise um Diole, Triole, Diamine, Triamine, Polyetherole und Polyesterole handeln. Dabei werden die Verbindungen mit mehr als 2 aktiven Wasserstoffatomen üblicherweise nur in geringen Mengen in Kombination mit einem großen Überschuß an Verbindungen mit 2 aktiven Wasserstoffatomen eingesetzt.

Beispiele für Verbindungen (V1) sind Ethylenglykol, 1,2- und 1,3-Propylenglykol, Butylenglykole, Di-, Tri-, Tetra- und Poly-Ethylen- und -Propylenglykole, Copolymere von niederen Alkylenoxiden wie Ethylenoxid, Propylenoxid und Butylenoxid, Ethylendiamin, Propylendiamin, 1,4-Diaminobutan, Hexamethylendiamin und $\alpha,\omega$-Diamine auf Basis von langkettigen Alkanen oder Polyalkylenoxiden.

Polyurethane, bei denen die Verbindungen (V1) Diole, Triole und Polyetherole sind, können erfindungsgemäß bevorzugt sein. Insbesondere Polyethylenglykole und Polypropylenglykole mit Molmassen zwischen 200 und 3000, insbesondere zwischen 1600 und 2500, haben sich in einzelnen Fällen als besonders geeignet erwiesen.

Polyesterole werden üblicherweise durch Modifizierung der Verbindung (V1) mit Dicarbonsäuren wie Phthalsäure, Isophthalsäure und Adipinsäure erhalten.

Als Verbindungen (V2) werden überwiegend Hexamethylendiisocyanat, 2,4- und 2,6-Toluoldiisocyanat, 4,4'-Methylendi(phenylisocyanat) und insbesondere Isophorondiisocyanat eingesetzt.

Weiterhin können die erfindungsgemäß verwendeten Polyurethane noch Bausteine wie beispielsweise Diamine als Kettenverlängerer und Hydroxycarbonsäuren enthalten. Dialkylolcarbonsäuren wie beispielsweise Dimethylolpropionsäure sind besonders geeignete Hydroxycarbonsäuren. Hinsichtlich der weiteren Bausteine besteht keine grundsätzliche Beschränkung dahingehend, ob es sich um nichtionische, anionischen oder kationische Bausteine handelt.

Bezüglich weiterer Informationen über den Aufbau und die Herstellung der Polyurethane wird ausdrücklich auf die Artikel in den einschlägigen Übersichtswerken wie Römpps Chemie-Lexikon und Ullmanns Enzyklopädie der technischen Chemie Bezug genommen.

Als in vielen Fallen erfindungsgemäß besonders geeignet haben sich Polyurethane erwiesen, die wie folgt charakterisiert werden können: ausschließlich aliphatische Gruppen im Molekül, keine freien Isocyanatgruppen im Molekül, Polyether- und Polyesterpolyurethane, anionische Gruppen im Molekül.

Es hat sich ebenfalls in einigen Fällen als vorteilhaft erwiesen, wenn das Polyurethan in dem System nicht gelöst, sondern stabil dispergiert ist.

Weiterhin hat es sich als für die Herstellung der erfindungsgemäßen Mittel als vorteilhaft erwiesen, wenn die Polyurethane nicht direkt mit den weiteren Komponenten gemischt, sondern in Form von wäßrigen Dispersionen eingebracht wurden. Solche Dispersionen weisen üblicherweise einen Feststoffgehalt von ca. 20-50 %, insbesondere etwa 35-45% auf und sind auch kommerziell erhältlich.

Ein erfindungsgemäß ganz besonders bevorzugtes Polyurethan ist unter der Handelsbezeichnung Luviset® PUR (BASF) im Handel.

Die erfindungsgemäßen Mittel können in einer weiteren Ausführungsform zusätzlich nichtionogene Polymere enthalten.

Geeignete nichtionogene Polymere sind beispielsweise:

- VinylpyrrolidonNinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel®-(AQUALON) vertrieben werden.
- Schellack
- Polyvinylpyrrolidone, wie sie beispielsweise unter der Bezeichnung Luviskol® (BASF) vertrieben werden.

Es ist erfindungsgemäß auch möglich, daß die verwendeten Zubereitungen mehrere, insbesondere zwei verschiedene Polymere gleicher Ladung und/oder jeweils ein ionisches und ein amphoteres und/oder nicht ionisches Polymer enthalten.

Weitere bevorzugte Polymere sind alle Polymere, welche im "International Cosmetic Ingredient Dictionary and Handbook", (seventh edition 1997, The Cosmetic, Toiletry, and Fragrance Association 1101 17th Street, N.W., Suite 300, Washington, DC 20036-4702) als Polymere in einem der Kapitel über Polymere wie beispielsweise "film formers" oder "hair fixatives" genannt und im Handel erhältlich sind. Auf diese Schrift und die daraus zitierten Abschnitte wird ausdrücklich Bezug genommen.

Das erfindungsgemäße Mittel wird bevorzugt in einem wässrigen oder einem organischen Medium oder in einer Mischung aus einem wässrigen und organischen Medium mit vorzugsweise mindestens 8, besonders bevorzugt mindestens 10 Gewichtsprozent Wasser konfektioniert. Als organisches Medium eignen sich beispielsweise die für kosmetische Zwecke üblicherweise verwendeten niederen Alkohole mit 1 bis 4 Kohlenstoffatomen wie zum Beispiel Ethanol und Isopropanol. Als weitere geeignete organische Lösemittel oder Gemisch aus Lösemitteln eignen sich organische Lösemittel mit einem Siedepunkt unter 400°C, bevorzugt in einer Menge von 0,1 bis 15 Gewichtsprozent, besonders bevorzugt von 1 bis 10 Gewichtsprozent. Besonders geeignet sind hier unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche, organische Lösungsmittel sind Aceton, Ethylacetat, Glycerin, Ethylenglykol und Propylenglykol, die jeweils allein oder im Gemisch in einer Menge bis 30 Gewichtsprozent in dem erfindungsgemäßen Mittel enthalten sein können.

Das erfindungsgemäße Mittel kann in einem pH-Bereich von 2 bis 11 vorliegen. Besonders bevorzugt ist der pH-Bereich zwischen 2 und 8.

Als weitere Komponente enthalten die erfindungsgemäßen Zubereitungen ist vorzugsweise ein Treibmittel.

Zur Anwendung der erfindungsgemäßen Zusammensetzungen als Aerosolsprays müssen Treibmittel verwendet werden. Die erfindungsgemäß bevorzugten Treibmittel sind ausgewählt aus den Kohlenwasserstoffen mit 3 bis 5 Kohlenstoffatomen, wie Propan, n-Butan, iso-Butan, n-Pentan und iso-Pentan, Dimethylether, Kohlendioxid, Distickstoffoxid, Fluorkohlenwasserstoffen und Fluorchlorkohlenwasserstoffen sowie Mischungen dieser Substanzen. Ganz besonders bevorzugte Treibgase sind Propan, Butan, Isobutan, Pentan, Isopentan, Dimethylether und die Gemische dieser zuvor genannten Treibgase jeweils untereinander. Erfindungsgemäß bevorzugteste Treibgase sind Dimethylether, Kohlenwasserstoffe und deren Gemische. Innerhalb der Gruppe der Kohlenwasserstoffe als Treibgasen bevorzugt sind n-Butan und Propan.

Vorteilhafterweise wird das Treibmittel so ausgewählt, daß es gleichzeitig als Lösungsmittel für weitere Inhaltsstoffe wie beispielsweise Öl- und Wachskomponenten, den Fettstoffen (D) dienen kann. Das Treibmittel kann dann als Lösungsmittel für diese letztgenannten Komponenten dienen, wenn diese bei 20 °C zu mindestens 0,5 Gew.%, bezogen auf das Treibmittel, in diesem löslich sind.

Gemäß einer bevorzugten Ausführungsform enthalten die erfindungsgemäßen Zubereitungen die genannten Kohlenwasserstoffe, Dimethylether oder Mischungen der genannten Kohlenwasserstoffe mit Dimethylether als einziges Treibmittel. Die Erfindung umfaßt aber ausdrücklich auch die Mitverwendung von Treibmittel vom Typ der Fluorchlorkohlenwasserstoffe, insbesondere aber der Fluorkohlenwasserstoffe.

Die Treibgase sind vorzugsweise in Mengen von 5 - 98 Gew.%, bevorzugt 10 - 98 Gew.% und besonders bevorzugt 20 - 98 Gew.%, ganz besonders bevorzugt von 40 bis 80 Gew.-%, jeweils bezogen auf die gesamte Aerosolzusammensetzung, enthalten.

Die erfindungsgemäßen Zusammensetzungen können in handelsüblichen Aerosoldosen verpackt sein. Die Dosen können aus Weißblech oder aus Aluminium sein. Weiterhin können die Dosen innen beschichtet sein, um die Gefahr der Korrosion so gering wie möglich zu halten.

Wenn die erfindungsgemäßen Zusammensetzungen als Non-Aerosol Sprühapplikation angewendet werden, ist selbstverständlich kein Treibgas enthalten. Jedoch sind die Sprühköpfe in jedem Falle nach den entsprechenden erforderlichen Sprühraten auszuwählen.

Die Dosen sind mit einem geeigneten Sprühkopf ausgestattet. Je nach Sprühkopf sind Ausstoßraten, bezogen auf voll gefüllte Dosen, von 0,1 g/s bis 5,0 g/s möglich. Die Sprührate wird dabei so bestimmt, dass eine mit Treibgas und der entsprechenden Zusammensetzung gefüllte und mit dem betreffenden Ventil verschlossene Aerosoldose bei Raumtemperatur (etwa 23 °C) zunächst gewogen wird. Die Dose wird samt Inhalt 10 mal kräftig von Hand geschüttelt, damit sich der Inhalt gut vermischt. Dann wird für 10 s das Ventil der senkrecht stehenden Dose betätigt. Danach wird wiederum gewogen. Der Vorgang wird 5 mal hintereinander durchgeführt und das statistische Mittel aus den Ergebnissen gebildet. Die Differenz der beiden Wägungen ist die Sprührate pro 10 s. Daraus lässt sich durch einfaches Dividieren die Sprührate je Sekunde bestimmen. Im Falle von Non-Aerosolen wird der Sprühmechanismus entsprechend 10 mal betätigt. Unter der Sprührate ist in letzterem Falle die durchschnittliche ausgebrachte Menge je Sprühstoß (Pumpstoß) zu verstehen. Sprühraten von 0,1 bis 0,5 g/s sind dabei bevorzugt. Sprühraten von 0,1 bis 0,4 g/s sind besonders bevorzugt.

Ein weiterer charakteristischer Einfluß für die Effizienz und Formulierbarkeit als Kompaktspray ist das Sprühbild. Das Sprühbild wird durch das Ventil und dessen Beschaffenheit entscheidend beeinflusst. Wenn beispielsweise in einer Haarsprayformulierung der Filmbildner bis auf das fünffache gegenüber einer konventionellen Rezeptur erhöht wird, so sind neben den zu beachtenden und zu vermeidenden erhöhten Viskositäten der Rezeptur auch die bereits diskutierte Sprührate wesentliche für die Formulierung zu beachtende Merkmale. Zusätzlich muß jedoch insbesondere auch das Sprühbild, das heißt der Öffnungskegel des Ventiles, und die Tröpfchengröße beachtet werden.

Wenn der Öffnungskegel einen zu großen Öffnungswinkel aufweist, dann wird das Produkt bei einem üblichen Abstand der Sprühdose vom Kopf des Anwenders von etwa 10 bis 40 cm auf eine zu kleine Haaroberfläche aufgebracht. Dies führt zu einer Veränderung der Wirksamkeit der Zusammensetzung. Es treten entweder Verklebungen durch zu hohe Produktmengen oder eine zu geringe Festigungswirkung durch eine zu geringe aufgebrachte Produktmenge auf. Im letzteren Falle ist der Öffnungskegel zu groß, so dass eine zu große Haaroberfläche mit der Zusammensetzung behandelt wird. Es hat sich nun gezeigt, dass der Öffnungskegel idealerweise zwischen 25 ° und 65 ° liegen muß. Ein Winkel von 30 ° bis 60 ° ist dabei bevorzugt. Ganz besonders bevorzugt sind Öffnungskegel zwischen 35 ° und 50 °.

Die mittlere Tröpfchengröße beim Versprühen ist bevorzugt kleiner als 40 µm. Vorzugsweise ist die mittlere Teilchengröße kleiner als 38 µm. Überraschenderweise hat sich gezeigt, dass sich die erfindungsgemäßen Mittel trotz des hohen Anteils an filmbildendem und/oder festigendem Polymer zuverlässig versprühen lassen, wenn eine entsprechend kleine mittlere Tröpfchengröße vorliegt. Bedingt durch die hohe Wirkstoffkonzentration und die kleine Tröpfchengröße beim Versprühen wird gewährleistet, dass einer Aerosoldose, die das Mittel enthält, bei im Vergleich zu üblichen Sprays unveränderter Handhabung durch den Verbraucher eine geringere Menge des Mittels entnommen, jedoch der gleiche festigende Effekt erzielt wird.

Die mittlere Tröpfchengröße wird mit einem Laserbeugungsmessgerät vom Typ Masterizer, Series 2600 Droplet and Particle Size Analyzer der Fa. Malvern bestimmt. Hierzu wird die Probe in einem definierten Abstand durch den Lichtstrahl des Lasers gesprüht und anhand der Laserbeugung die Teilchengrößenverteilung bestimmt.

Die Viskosität der zu versprühenden Rezeptur kann je nach Konzentration der filmbildenden Polymeren ebenfalls einen Einfluß zeigen. Gleichzeitig sind jedoch auch Ventile bekannt, mit deren Hilfe sich selbst Gele versprühen lassen. Es kann jedoch erfindungsgemäß bevorzugt sein, wenn die Viskositäten der Rezepturen kleiner als 5000 mPas gemessen nach Brookfield mit Spindel 3 bei 20 Upm und 25 °C betragen. Die Viskositäten der Rezepturen werden dabei vor der Zugabe von Treibgas gemessen. Besonders bevorzugt kann es sein, wenn die Viskositäten kleiner als 2500 mPas und ganz besonders bevorzugt kleiner als 1000 mPas sind.

Schließlich kann das Problem des Verklebens der Ventile zusätzlich zur gezielten Auswahl der filmbildenden Polymere auch durch eine entsprechende sorgfältige Verarbeitung, Materialauswahl und/oder Vorbehandlung der Ventile positiv beeinflusst werden. Wesentlich ist dabei, dass alle mit der Zusammensetzung in Berührung kommenden Teile des Ventiles eine möglichst glatte Oberfläche aufweisen. Je glatter die Oberfläche ist, desto weniger kann die Zusammensetzung an ihr durch Adhäsion anhaften. Somit wird einer Verklebung der Düse entgegengewirkt. Die Erzielung besonders glatter Oberflächen sind dem Fachmann bekannt, beispielsweise durch eine Ausführung der Oberfläche in Form von Nanopartikeln zur Erzielung eines Lotosblüteneffektes oder einer Polierung der Oberflächen wie beispielsweise einer Elektropolierung.

Eine Ausführungsform der vorliegenden Erfindung kann mit dem Auge klar erkennbare optisch sichtbare Partikel mit einer Teilchengröße von 0,1 bis 3 mm in der Zusammensetzung enthalten. Diese Partikel werden während der Anwendung beim Ausbringen aus der Verpackung mechanisch zerstört.

Weiterhin können in den erfindungsgemäß verwendeten Zubereitungen zusätzlich Proteinhydrolysate und/oder Aminosäuren und deren Derivate enthalten sein. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgeweicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Die Proteinhydrolysate oder deren Derivate sind in den erfindungsgemäß verwendeten Mitteln bevorzugt in Mengen von 0,1 bis 10 Gew.-%, bezogen auf das gesamte Mittel, enthalten. Mengen von 0,1 bis 5 Gew.-% sind besonders bevorzugt.

Ebenfalls als vorteilhaft hat es erwiesen Vitamine, Provitamine und Vitaminvorstufen sowie deren Derivate den erfindungsgemäßen Zusammensetzungen zuzusetzen. Dabei kann es bevorzugt sein nur solche Vitamine, Provitamine und Vitaminvorstufen und deren Derivate auszuwählen, die nur in Alkohol und/oder Alkohol - Wassergemischen löslich sind.

Dabei sind erfindungsgemäß solche Vitamine, Pro-Vitamine und Vitaminvorstufen bevorzugt, die üblicherweise den Gruppen A, B, C, E, F und H zugeordnet werden.

Schließlich läßt sich die Wirkung auch durch den kombinierten Einsatz mit Pflanzenextrakten steigern.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Baldrian, Hopfen, Henna, Kamille, Klettenwurzel, Gallapfel, Chardonnay, Grape Seed, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian,

Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Baldrian, Kamille, Klettenwurzel, Gallapfel, Chardonnay, Grape Seed, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Grünem Tee, Gallapfel, Chardonnay, Grape Seed, Baldrian, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Zusätzlich kann es sich als vorteilhaft erweisen, wenn neben der erfindungsgemäßen Kombination Penetrationshilfsstoffe und/ oder Quellmittel enthalten sind. Diese Hilfsstoffe sorgen für eine bessere Penetration von Wirkstoffen in die keratinhaltige Faser oder helfen die keratinhaltige Faser aufzuquellen. Hierzu sind beispielsweise zu zählen Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Bezüglich weiterer fakultativer Komponenten sowie die eingesetzten Mengen dieser Komponenten wird ausdrücklich auf die dem Fachmann bekannten einschlägigen Handtücher, z. B. die oben genannte Monographie von K. H. Schrader verwiesen.

Die Konfektionierung der erfindungsgemäßen Zusammensetzung erfolgt in einer bevorzugten Ausführungsform in einer Form, die das Versprühen der Zusammensetzung erlaubt. Beispielsweise kann die erfindungsgemäße Zusammensetzung als Aerosol, als Non-Aerosol Sprühlotion, welche mittels einer mechanischen Vorrichtung zum Versprühen zum Einsatz kommt, als Aerosol-Schaum oder als Non-Aerosol Schaum, welcher in Kombination mit einer geeigneten mechanischen Vorrichtung zum Verschäumen der Zusammensetzung vorliegt konfektioniert werden.

Eine geeignete Applikationsform ist eine Aerosol- und/oder Non-Aerosol Sprühapplikation. Hierbei wird das erfindungsgemäße Mittel mit Hilfe einer geeigneten mechanisch betriebenen Sprühvorrichtung versprüht. Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Wenn das erfindungsgemäße Mittel in Form eines festigenden Haarschaumes (Mousse) vorliegt, so enthält es mindestens eine für diesen Zweck bekannte, übliche schaumgebende Substanz. Das Mittel wird mit oder ohne Hilfe von Treibgasen oder chemischen Treibmitteln verschäumt und als Schaum in das Haar eingearbeitet und ohne Ausspülen im Haar belassen. Ein erfindungsgemäßer Haarschaum weist als zusätzliche Komponente ein chemisches Treibmittel und/oder eine mechanische Vorrichtung zum Verschäumen der Zusammensetzung auf. Unter mechanischen Schäumvorrichtungen sind solche Vorrichtungen zu verstehen, welche das Verschäumen einer Flüssigkeit mit oder ohne Verwendung eines Treibmittels ermöglichen. Als geeignete mechanische Schäumvorrichtung kann beispielsweise ein handelsüblicher Pumpschäumer oder ein Aerosolschaumkopf verwendet werden.

Ein zweiter Gegenstand der Erfindung ist ein Verfahren zur Formfixierung faserhaltiger Materialien, in dem auf die gegebenenfalls vorgefeuchteten Fasern ein Mittel des ersten Erfindungsgegenstandes aufgetragen wird.

Bei den Fasern handelt es sich bevorzugt um Baumwolle, Seide, Viskose, Schurwolle oder keratinhaltige Fasern, insbesondere um menschliches Haar. Als faserhaltiges Material dienen ebenfalls bevorzugt aus oben genannten Fasern gewobenes Material, wie z.B. Stoffe oder Textilien, oder Faserbündel, wie z.B. menschliches Haar.

Das faserhaltige Material kann vor, während oder direkt nach der Aufbringung des erfindungsgemäßen Mittels in Form gebracht werden. Dabei wird dem faserhaltigen Material gegebenenfalls mit Umformungshilfsmitteln, wie Schablonen oder Wickelhilfen (wie beispielsweise Dauerwickler) versehen, die dem Material eine neue Form aufgeprägt.

Das erfindungsgemäße Mittel wird bevorzugt nach der Einwirkungszeit von dem faserhaltigen Material abgespült. Die Einwirkungszeit beträgt bevorzugt 5 bis 60 Minuten. Die Umformungshilfsmittel werden bevorzugt nach Ablauf

der Einwirkungszeit entfernt.

Das faserhaltige Material, insbesondere menschliches Haar, wird bevorzugt während der Einwirkungszeit permanent oder zeitweise auf eine Temperatur von 60°C bis 200°C, insbesondere von 80°C bis 180°C temperiert. Die Fasern können mit heißer Luft, beispielsweise mit einem Fön, oder mit entsprechend aufgeheizten Materialien, wie z.B. Bügel- bzw. Glätteisen, temperiert werden.

Insbesondere bei der Verformung menschlicher Haare ist es ebenso denkbar, lockiges Haar im Rahmen einer Haarglättung umzuformen, d.h. zu glätten. Zu diesem Zweck werden Verfahren bevorzugt in denen nach dem Auftragen des erfindungsgemäßen Mittels während der Einwirkungszeit das Haar zur Haarglättung mit erhitzten Glättplatten, insbesondere Metall- oder Keramikplatten, behandelt wird. Die Platten können gegebenenfalls mit hitzebeständigen Werkstoffen beschichtet sein. Die Temperaturen der Glättplatten liegen dabei bevorzugt in dem oben genannten Intervall. Die Glättplatte wird auf die zu glättende Faser gedrückt und die an die Faser gedrückte Platte entlang der Faser bewegt. Besonders bevorzugt wird die zu glättende keratinhaltige Faser zwischen zwei entsprechend temperierte Platten gepreßt und beide Platten zugleich entlang der längsten räumlichen Ausdehnung der Faser bewegt. Dabei ist es wiederum bevorzugt, daß beide Platten miteinander verbunden sind, so daß beide Platten gleichmäßig entlang der Faser bewegt werden können. Die Bewegung entlang der Faser findet entlang der längsten räumlichen Ausdehnung der Faser statt. Wird die Wärmebehandlung am lebenden Haar durchgeführt, so ist die Faser an einem Ende (Haarwurzel) befestigt. Die Platten werden in diesem Fall bevorzugt gleichmäßig von der Haarwurzel weg entlang der gesamten Faser bewegt. Durch diese Bewegung erfolgt eine mechanische Gattung der Faser. Ein entsprechendes Gerät zur Wärmebehandlung ist beispielsweise das Gerät "Ceramic Flat-Master" (Vertrieben durch: Efalock, Deutschland).

Das erfindungsgemäße Verfahren eignet sich bevorzugt zur Anwendung an keratinhaltigen Fasern, insbesondere menschlichen Haaren.

Bei Anwendung der erfindungsgemäßen Mittel, insbesondere im Rahmen des erfindungsgemäßen Verfahrens wurde beobachtet, dass

- sich faserhaltiges Material, insbesondere keratinhaltige Fasern (bevorzugt menschliches Haar) mit dem erfindungsgemäßen Mittel beschichten lassen,
- die erzielte Beschichtung eine erhöhte Waschresitzenz besitzt,
- die Form des faserhaltigen Materials, insbesondere keratinhaltiger Fasern (bevorzugt menschliches Haar) mit dem erfindungsgemäßen Mittel fixiert wird,
- die Farbe des faserhaltigen Materials, insbesondere keratinhaltiger Fasern (bevorzugt menschliches Haar) fixiert wird,
- die resultierende Beschichtung des faserhaltigen Materials, insbesondere keratinhaltige Fasern (bevorzugt menschliches Haar) in der Resistenz gegenüber mechanischer Beanspruchung (wie biegen, reiben, kämmen) verbessert wird,
- die faserhaltigen Materialien einen Schutz vor ultravioletter Strahlung erhalten.

Alls diese Verwendungsformen des erfindungsgemäßen Mittels sind Gegenstand der vorligenenden Erfindung.

Im Rahmen der verbesserten Farbfixierung wurde beobachtet, dass durch die Behandlung des faserhaltigen Materials mit dem erfindungsgemäßen Mittel die natürliche Farbe, aber auch eine mit Hilfe von Direktfarbstoffen oder Oxidationsfarbstoffen erzielte Färbung haltbarer, insbesondere gegenüber Licht und Waschungen, wird. Selbstverständlich treten im Rahmen der erfindungsgemäßen Formfixierung keine Farbänderungen der Fasern auf, wie dies beispielsweise bei der Kaltwelle festgestellt werden kann.

Ein weiterer Gegenstand der Erfindung sind folgende Polymere:

Die Polymere (P122), (P122a), (P295) und (P296) des ersten Erfindungsgegstandes sind ebenfalls per se ein Erfindungsgegenstand.

## Beispiele

1.0 Monomersynthese

1.1 Synthese von 2-[(4,6-Dichlor-1,3,5-triazin-2-yl)amino]ethyl-2-methylacrylat (Monomer 1)

In einem 500 mL Dreihalsdoppelmantelkolben mit Tropftrichter wurden 9,20 g 2-Aminoethylmethacrylat hydrochlorid in 150 mL Wasser gelöst und die Lösung unter Rühren auf ca. 0°C abgekühlt. Eine Lösung von 9,31 g Cyanurchlorid in 50 mL Aceton wurde über einen Zeitraum von 15 Minuten zugetropft. Anschließend wurde innerhalb von 1 Stunde portionsweise 8,40 g Natriumhydrogencarbonat zugegeben. Die Kühlung wurde entfernt und die Reaktionsmischung

unter Rühren auf Raumtemperatur aufgewärmt und bei Raumtemperatur 1 Stunde rühren gelassen. Danach wurde die Reaktionslösung ernaut auf 0°C gekühlt und kalt filtriert. Der Filterkuchen wurde mit wenigen Millilitern Aceton gewaschen und anschließend getrocknet. Das Rohprodukt wurde zweimal aus 30 mL Toluol umkristallisiert. Es wurden 5,32 g eines Feststoffs erhalten.

2.0 Polymersynthese

2.1 Synthese eines Copolymers aus Monomer 1 / Methylmethacrylat / 2-Ethylhexyl-acrylat (Polymer 1)

In einem 250ml-Dreihalsrundkolben wurden 1,00 g Monomer 1 in 20,0 g wasserfreiem Dioxan gelöst. 5,00 g Methylmethacrylat, 5,00 g 2-Ethylhexylacrylat und 0,22 g lucidol® CH-50 X (50 Gew.-% Dibenzoylperoxid in Dicyclohexylphthalat) wurden zugewogen. Unter Stickstoff wurde 7 Stunden bei 80°C gerührt. Die viskose Reaktionslösung wurde in Methanol zur Fällung gebracht, nachgewaschen und im Vakuumtrockenschrank bei 50°C getrocknet. Es wurde 9,86 g eines Feststoffs erhalten.

2.2 Synthese eines Copolymers aus Monomer 1 / Methylmethacrylat / 2-Ethylhexyl-acrylat (Polymer 2)

In einem 250ml-Dreihalsrundkolben wurden 1,00 g Monomer 1 in 20,0 g wasserfreiem Dioxan gelöst. 7,50 g Methylmethacrylat, 2,50 g 2-Ethylhexylacrylat und 0,22 g Lucidol® CH-50 X (50 Gew.-% Dibenzoylperoxid in Dicyclohexylphthalat) wurden zugewogen. Unter Stickstoff wurde 7 Stunden bei 80°C gerührt. Die viskose Reaktionslösung wurde in Methanol zur Fällung gebracht, nachgewaschen und im Vakuumtrockenschrank bei 50°C getrocknet. Es wurde 9,45 g eines Feststoffs mit einer mittleren Molmasse von ca. 77000 Dalton erhalten (ermittelt über GPC).

2.3 Synthese eines Copolymers aus Monomer 1 / Methylmethacrylat / 2-Ethylhexyl-acrylat (Polymer 3)

In einem 250ml-Dreihalsrundkolben wurden 1,00 g Monomer 1 in 20,0 g wasserfreiem Dioxan gelöst. 1,00 g Methylmethacrylat, 10,00 g 2-Ethylhexylacrylat und 1,20 g Lucidol® CH-50 X (50 Gew.-% Dibenzoylperoxid in Dicyclohexylphthalat) wurden zugewogen. Unter Stickstoff wurde 7 Stunden bei 80°C gerührt. Die viskose Reaktionslösung wurde in Methanol zur Fällung gebracht, nachgewaschen und im Vakuumtrockenschrank bei Raumtemperatur über konz. Schwefelsäure getrocknet. Es wurde 11,17 g eines Feststoffs erhalten.

2.4 Synthese eines Copolymers aus Monomer 1 / 2-Ethylhexyl-acrylat (Polymer 4)

In einem 250ml-Dreihalsrundkolben wurden 1,00 g Monomer 1 in 20,0 g wasserfreiem Dioxan gelöst. 10,00 g 2-Ethylhexylacrylat und 1,20 g Lucidol® CH-50 X (50 Gew.-% Dibenzoylperoxid in Dicyclohexylphthalat) wurden zugewogen. Unter Stickstoff wurde 7 Stunden bei 80°C gerührt. Die viskose Reaktionslösung wurde in Methanol zur Fällung gebracht, nachgewaschen und im Vakuumtrockenschrank bei Raumtemperatur über konz. Schwefelsäure getrocknet. Es wurde 10,43 g eines Feststoffs erhalten.

2.5 Synthese eines Copolymers aus Monomer 1 / Methylmethacrylat (Polymer 5)

In einem 250ml-Dreihalsrundkolben wurden unter Stickstoff 4,93 g Monomer 1 in 80 mL wasserfreiem Dioxan gelöst. 6,47 g Methylmethacrylat und 0,03 g Azoisobutyronitril (AIBN) wurden zugewogen. Unter Stickstoff wurde 1 Stunde bei Raumtemperatur, 2 Stunden bei 50°C, und 15 Stunden unter Rückfluss gerührt, wobei zwischenzeitlich zukzessive 0,1 g AIBN zugeführt wurden. Die Reaktionslösung wurde auf ¼ ihres Volumens eingeengt und in einen Dialyseschlauch (Spectra Por MWCO 500) 24 Stunden dialysiert. Der entstandene Feststoff wurde isoliert und im Vakuum über $P_2O_5$ getrocknet. Es wurde 9,47 g eines Feststoffs erhalten. Das Polymer besaß eine mittlere Molmasse von ca. 49000 Dalton (ermittelt über GPC).

2.6 Synthese eines Copolymers aus Monomer 1 / Methylmethacrylat / 2-Ethylhexylacrylat / PEG-Monomethylethermethacrylat (Polymer 6)

In einem 250ml-Dreihalsrundkolben wurden unter Stickstoff 1,00 g Monomer 1 in 20 mL wasserfreiem Dioxan gelöst. 2,50 g Methylmethacrylat, 2,50 g 2-Ethylhexylacrylat und 5,00 g PEG-Monomethylethermethacrylat (Fa. Aldrich, # 44,7951; Batch 17711PC; Molekulargewicht 1100 g/mol) und 0,22 g Lucidol® CH-50 X (50 Gew.-% Dibenzoylperoxid in Dicyclohexylphthalat) wurden zugewogen. Unter Stickstoff wurde 7 Stunden bei 80°C gerührt. Die Reaktionslösung wurde in 700 mL Ethanol eingerührt und diese Lösung bei Raumtemperatur im Membranpumpenvakuum am Rotationsverdampfer eingeengt und anschließend im Vakuumtrockenschrank bei 25°C getrocknet. Es wurden 10,98 g eines

Feststoffs erhalten. Das Polymer löste sich in Ethanol und war in Isopropanol/WasserGemischen dispergierbar. Die Löslichkeitseigenschaften wurden verbessert.

3.0 Ermittlung des Formerhalts umgeformter keratinhaltiger Fasern (Haare) nach einmaligem Waschen und nach einmaligem Waschen & Kämmen

25 cm lange und 0,5 g schwere Haarsträhnen (Typ 6634, Fa. Alkinco) wurden an einem Ende abgebunden und verklebt. Jede Haarstähne wurde in einer 12%-igen Texapon NSO-Lösung bei pH-6,5 für 30 Minuten gereinigt und für 5 Minuten mit 38°C warmem Wasser ausgespült. Die gewaschenen Strähnen wurden im Trockenschank bei 80°C getrocknet.
Jede Strähne wurde in der Länge L vermessen.
Die Strähnen wurden mit 2 Sprühstössen Leitungswasser (0,5 g) befeuchtet. Sodann wurde 1 mL eines Umformmittels gemäß Tabelle 1 mittels Pipette appliziert und die Strähne auf einen Spiralwickler der Firma Firpac-Medis (Durchmesser 7 mm) mit Hilfe eines Spanngewichts von 140 g gewickelt.

Tabelle 1: Umformmittel

| Inhalt | Umformittel-Nummer |
|---|---|
| 0,5 g Polymer 1 in 9,5 g 1,4-Dioxan | U-1 |
| 0,5 g Polymer 2 in 9,5 g 1,4-Dioxan | U-2 |
| 0,5 g Polymer 3 in 9,5 g 1,4-Dioxan | U-3 |
| 0,5 g Polymer 4 in 9,5 g 1,4-Dioxan | U-4 |
| 0,2 g Polymer 4 in 9,8 g 1,4-Dioxan | U-5 |
| 1 g Polymer 5 in 9 g Aceton | U-6 |
| 0,5 g Polymer 6 in 9,5 g Wasser | U-7 |

3.1 Durchführung der Umformung und Bestimmung der Curlretention

Je Umformmittel der Tabelle 1 und der zu bestimmenden Waschkategorie (*vide infra*) wurden 3 Strähnen wie zuvor beschrieben präpariert. Aus den jeweiligen Messgrössen wurde das arithmetische Mittel gebildet.
Die Strähnen wurden danach auf dem Wickler über einen Zeitraum von 2 Stunden im Trockenschrank bei 150°C getempert und nach dem Abkühlen vorsichtig abgewickelt. Anschließend wurden die Strähnen für 90 Minuten in einen Klimaraum (22°C, 40 % Luftfeuchtigkeit) an Klammern aushängen gelassen. An der gelockten Strähne wurde die Länge der Locke ($L_0$) gemessen.

Die Strähnen wurden sodann wie folgt in einem 250 mL Standzylinder gewaschen:

Waschkategorie A betrifft die Bestimmung der Curlretention bei Waschen in dem Standzylinder über einen Zeitraum von 1 Minute mit 38°C warmem Wasser.
Waschkategorie B betrifft die Bestimmung der Curlretention bei Waschen in dem Standzylinder über einen Zeitraum von 1 Minute in 12 Gew.%iger Texapon® NSO Lösung (Texapon® NSO: wässrige Lösung von 28 Gew.-% mit 2 Molekülen ethoxiliertem Laurylsulfat; INCI-Bezeichnung: Sodium Laureth Sulfate) bei pH 6,5 und anschließendem zweimaligem Spülen Ober 30 Sekunden in dem Standzylinder mit 38°C warmem Wasser.
Die Strähnen tauchten jeweils in die befüllten Standzylinder komplett ein. Während des Waschvorgangs erzeugte ein Magnetrührer eine Strömung durch starkes Rühren.
Die tropfnassen Strähnen wurden nach dem Waschvorgang in den Klimaraum gehängt und dort über einen Zeitraum von 15 Stunden getrocknet. Danach wird die Länge der gewaschenen Locke ($L_{t1}$) bestimmt.
Danach werden die Haare mit einem feinen Stielkamm 10 Mal durchgekämmt und die Länge der Locke ($L_{t2}$) erneut bestimmt.
Die Curlretention (CR) wird wiefolgt berechnet:

$$CR_{waschen} = 100 \cdot (L - L_{t1}) / (L - L_0)$$

$$CR_{waschen\ \&\ kämmen} = 100 \cdot (L - L_{t2}) / (L - L_0)$$

Tabelle 2: Ergebnisse der Curlretention-Bestimmung (Waschkategorie A)

| Umformmittel | $CR_{waschen}$ | $CR_{waschen\&kämmen}$ |
|---|---|---|
| U-1 | 76,3 | 57,7 |
| U-2 | 55,8 | 47,0 |
| U-3 | 91,0 | 83,6 |
| U-4 | 86,6 | 65,3 |
| U-5 | 46,9 | 36,9 |
| U-6 | 61,5 | 42,6 |

Tabelle 3: Ergebnisse der Curlretention-Bestimmung (Waschkategorie B)

| Umformmittel | $CR_{waschen}$ | $CR_{waschen\&kämmen}$ |
|---|---|---|
| U-1 | 79,9 | 66,5 |
| U-2 | 62,9 | 48,9 |
| U-3 | 93,5 | 81,5 |
| U-4 | 85,3 | 62,9 |
| U-5 | 69, 9 | 58,4 |
| U-6 | 57,0 | 43,8 |
| U-7 | 81, 8 | 79,7 |

Alle erfindungsgemäßen Umformmittel lieferten eine gegenüber waschen bzw. waschen und kämmen in ihrer Resistenz verbesserte Formgebung.

**Patentansprüche**

1. Mittel, welches in einem kosmetischen Träger mindestens ein Polymer mit mindestens einer Struktureinheit der Formel (IV) enthält

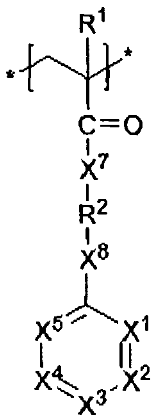

(IV)

wobei

- $X^1$, $X^2$, $X^3$, $X^4$ und $X^5$ unabhängig voneinander für eine Methanylylidengruppe, ein Stickstoffatom oder eine Halogenmethanylylidengruppe stehen, mit der Maßgabe, dass mindestens zwei der Reste $X^1$ bis $X^5$ ein Stick-

stoffatom und mindestens zwei der Reste $X^1$ bis $X^5$ eine Halagenmethanylylidengruppe und höchstens ein Rest aus $X^1$ bis $X^5$ eine Methanylylidengruppe bedeutet,
- $X^7$ für ein Sauerstoffatom oder eine Gruppe NH steht,
- $X^8$ für eine direkte Bindung, ein Sauerstoffatom oder eine Gruppe IVH steht,
- $R^1$ ein Wasserstoffatom oder eine ($C_1$-bis $C_4$)-Alkylgruppe bedeutet,
- $R^2$ für eine direkte Bindung, ($C_2$- bis $C_6$-)Alkylengruppe, eine oligoalkylenoxidgruppe mit 1 bis 20 Einheiten ($C_2$- bis $C_4$)-Alkylenoxid oder eine Phenylengruppe steht.

2. Mittel nach Anspruch 1, **dadurch gekennzeichnet, daß** $X^1$, $X^3$, und $X^5$ für ein Stickstoffatom, $X^2$ und $X^4$ für eine Halogenmethanylylidengruppe und $X^8$ für eine Methanylylidengruppe stehen.

3. Mittel nach Anspruch 1, **dadurch gekennzeichnet, daß** $X^1$, $X^2$ und $X^4$ für eine Halogenmelhanylylidengruppe, $X^6$ für eine Methanylylidengruppe und $X^3$ und $X^5$ für ein Stickstoffatom stehen.

4. Mittel nach einem der Ansprüche 1 bis 3, **dadurch gekennzeichnet, daß** das besagte Polymer ein mittlere Molmasse von 1500 bis 1250000 besitzt.

5. Mittel nach einem der Ansprüche 1 bis 4, **dadurch gekennzeichnet, daß** es sich um ein Copolymer handelt.

6. Mittel nach Anspruch 5, **dadurch gekennzeichnet, daß** das Polymer neben besagter Struktureinheit zusätzlich mindestens eine der nachfolgenden Struktureinheiten enthält

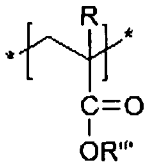 (M1) 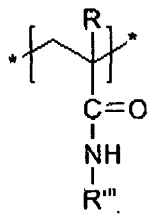 (M2) 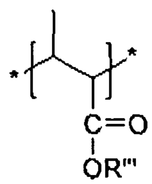 (M3)

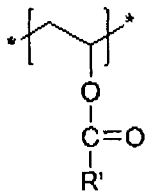 (M4) 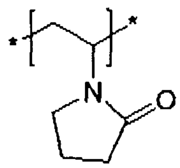 (M5) 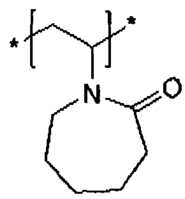 (M6)

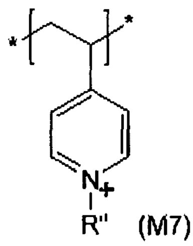 (M7) 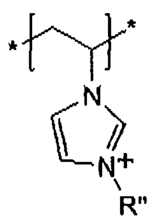 (M8) 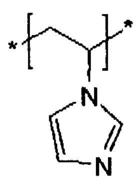 (M9)

worin
R steht für ein Wasserstoffatom oder eine ($C_1$- bis $C_4$)-Alkylgruppe,
R' steht für eine ($C_1$- bis $C_{30}$)-Alkylgruppe oder eine Arylgruppe,
R" steht für eine ($C_1$- bis $C_4$)-Alkylgruppe, eine ($C_1$- bis $C_4$)-Aralkylgruppe oder eine ($C_2$- bis $C_4$)-Alkenylgruppe,
R'" steht für ein Wasserstoffatom, eine eine lineare oder verzweigte ($C_1$- bis $C_{30}$)-Alkylgruppe, eine ($C_2$- bis $C_4$)-Alkenylgruppe, eine ω-Amino($C_2$-$C_6$)alkylgruppe, eine ω-Di($C_1$- bis $C_4$)alkylamino($C_2$- bis $C_6$)alkylgruppe, ω-Tri($C_1$-

bis $C_4$)alkylammonio($C_2$- bis $C_6$)alkylgruppe, eine Gruppe -$(CH_2CH_2O)_n$-$R^{IV}$ mit n gleich einer ganzen Zahl von 1 bis 60 und $R^{IV}$ steht für ein Wasserstoffatom oder eine ($C_1$- bis $C_{30}$)-Alkylgruppe.

7. Mittel nach einem der Ansprüche 1 bis 6, **dadurch gekennzeichnet, daß** die Struktureinheit der Formel (IV) zu mindestens 5 Mol%, insbesondere mindestens 20 Mol%, in dem Polymer enthalten ist.

8. Verfahren zur Formfixierung von faserhaltigem Material, **dadurch gekennzeichnet, daß** auf das gegebenenfalls vorgefeuchtete faserhaltige Material ein Mittel gemäß einem der Ansprüche 1 bis 7 aufgetragen wird.

9. Verfahren nach Anspruch 8, **dadurch gekennzeichnet, daß** das Mittel nach einer Einwirkungszeit abgespült wird.

10. Verfahren nach einem der Ansprüche 8 oder 9, **dadurch gekennzeichnet, daß** die Einwirkungszeit 15 bis 60 Minuten beträgt.

11. Verfahren nach einem der Ansprüche 6 oder 9, **dadurch gekennzeichnet, daß** das Haar vor dem Auftragen des Mittels auf Umformungshilfen gewickelt wird.

12. Verfahren nach einem der Ansprüche 8 bis 10, **dadurch gekennzeichnet, daß** das faserhaltige Material während einer Einwirkungszeit permanent oder zeitweise auf eine Temperatur von 60°C bis 200°C, insbesondere von 80°C bis 180°C temperiert wird.

13. Verfahren nach einem der Ansprüche 8 oder 9, **dadurch gekennzeichnet, daß** nach dem Auftragen des Mittels während der Einwirkungszeit das faserhaltige Material zur Glättung mit erhitzten Glättplatten behandelt wird.

14. Verwendung eines Mittels gemäß einem der Ansprüche 1 bis 7 zur Beschichtung faserhaltigen Materials, insbesondere menschlicher Haare.

15. Verwendung eines Mittels gemäß einem der Ansprüche 1 bis 7 zur Formfixierung faserhaltigen Materials, insbesondere menschlicher Haare.

16. Verwendung eines Mittels gemäß einem der Ansprüche 1 bis 7 zur Erhöhung der Waschresistenz von Beschichtungen auf faserhaltigem Material.

17. Verwendung eines Mittels gemäß einem der Ansprüche 1 bis 7 zur Verbesserung der Resistenz von Beschichtungen faserhaltigen Materials gegen mechanische Beanspruchung.

18. Polymer, enthaltend nachstehende Kombination aus jeweils mindestens einer der angeführten Struktureinheiten

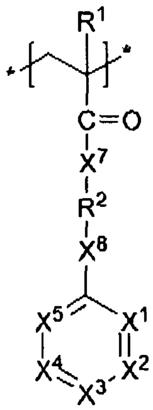 und 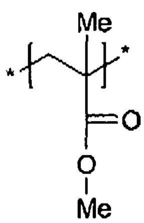 und 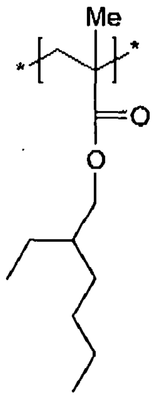 (P122)

(IV) (M1-15) (M1-22)

wobei die Reste $R^1$, $R^2$, $X^1$ bis $X^8$ wie in Anspruch 1 definiert sind.

19. Polymer, enthaltend nachstehende Kombination aus jeweils mindestens einer der angeführten Struktureinheiten

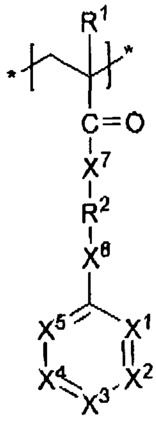 und 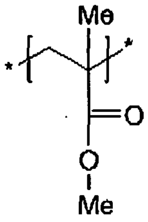 und 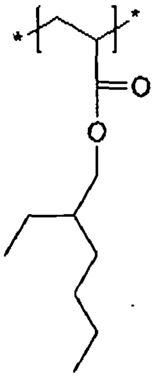 (P122a)

(IV) (M1-15) (M1-8)

wobei die Reste $R^1$, $R^2$, $X^1$ bis $X^8$ wie in Anspruch 1 definiert sind.

**20.** Polymer, enthaltend nachstehende Kombination aus jeweils mindestens einer der angeführten Struktureinheiten

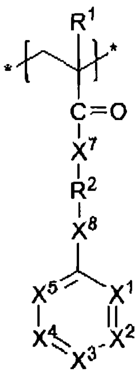 und 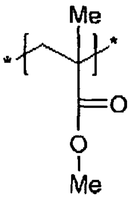 und 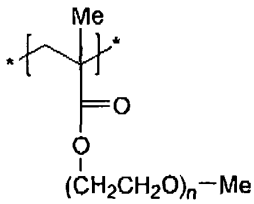

(IV) (M1-15) (P295)

wobei die Reste $R^1$, $R^2$, $X^1$ bis $X^8$ wie in Anspruch 1 definiert sind und n für eine ganze Zahl von 1 bis 60 steht.

**21.** Polymer, enthaltend nachstehende Kombination aus jeweils mindestens einer der angeführten Struktureinheiten

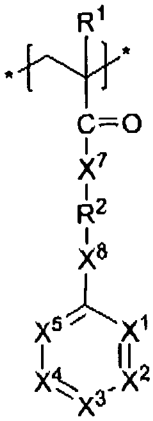 und 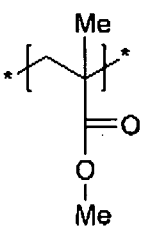 und 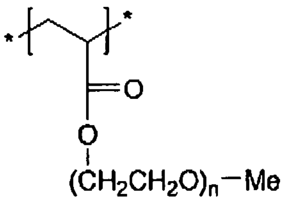

(IV) (M1-15) (P296)

wobei die Reste $R^1$, $R^2$, $X^1$ bis $X^8$ wie in Anspruch 1 definiert sind und n für eine ganze Zahl von 1 bis 60 steht.

**Claims**

1. An agent, which contains in a cosmetic carrier, at least one polymer with a least one structural unit of formula (IV)

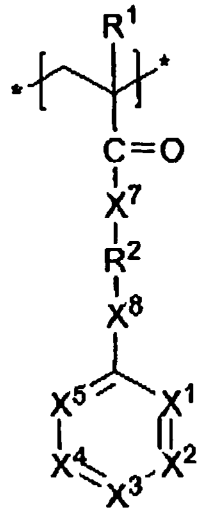

(IV)

wherein

- $X^1$, $X^2$, $X^3$, $X^4$ and $X^6$ independently of each other represent a methanylylidene group, a nitrogen atom, or a halomethanylylidene group, with the proviso that at least two of the radicals $X^2$ to $X^5$ represent a nitrogen atom and at least two of the radicals $X^1$ to $X^5$ represent a halomethanylylidene group and at most one radical from $X^1$ to $X^5$ represents a methanylylidene group,
- $X^7$ represents a oxygen atom or an NH group,
- $X^8$ represents a direct bond, an oxygen atom or an NH group,
- $R^1$ represents a hydrogen atom or a $C_1$-$C_4$ alkyl group,
- $R^2$ represents a direct bond, a $C_2$-$C_6$ akylene group, an oligoalkylene oxide group with 1 to 20 units of $C_2$-$C_4$ alkylene oxide or a phenylene group.

2. The agent according to claim 1, **characterized in that** $X^1$, $X^3$ and $X^5$ represent a nitrogen atom, $X^2$ and $X^4$ represent a halomethanylylidene group and $X^6$ a methanylylidene group.

3. The agent according to claim 1, **characterized in that** $X^1$, $X^2$ and $X^4$ represent a halomethanylylidene group, $X^6$ a methanylylidene group and $X^3$ and $X^5$ a nitrogen atom.

4. The agent according to any of claims 1 to 3, **characterized in that** said polymer has an average molar mass from 1,500 to 1,250,000.

5. The agent according to any of claims 1 to 4, **characterized in that** it is a copolymer.

6. The agent according to claim 5, **characterized in that** the polymer in addition to said structural unit, further contains at least one of the following structural units

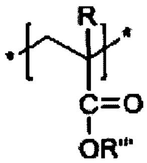 (M1) 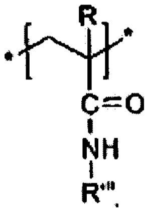 (M2) 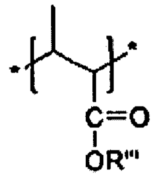 (M3)

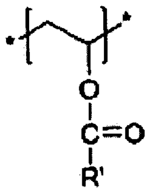 (M4) 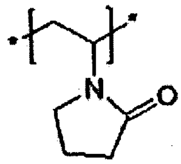 (M5) 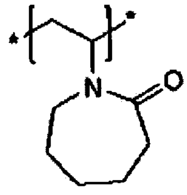 (M6)

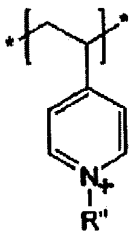 (M7) 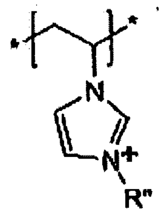 (M8) 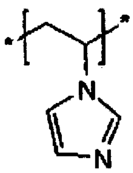 (M9)

wherein
R represents a hydrogen atom or a $C_1$-$C_4$ alkyl group,
R represents a $C_1$-$C_{30}$ alkyl group or an aryl group,
R'' represents a $C_1$-$C_4$ alkyl group, a $C_1$-$C_4$ aralkyl group or a $C_2$-$C_4$ alkenyl group,
R''' represents a hydrogen atom, a linear or branched $C_1$-$C_{30}$ alkyl group, a $C_2$-$C_4$ alkenyl group, an ω-amino-($C_2$-$C_6$) alkyl group, an ω-di($C_1$-$C_4$)alkylamino ($C_2$-$C_6$)alkyl group, ω-tri($C_1$-$C_4$)alkylammonio($C_2$-$C_6$)alkyl group, a -$(CH_2CH_2O)_n$-$R^{IV}$ group, with n equal to an integer from 1 to 60 and $R^{IV}$ represents a hydrogen atom or a $C_1$-$C_{30}$ alkyl group.

**7.** The agent according to any of claims 1 to 6, **characterized in that** the structural unit of formula (IV) is at least contained in an amount of 5 mol%, in particular at least 20mol% in the polymer.

**8.** A method for fixing the shape of fiber-containing material, **characterized in that** an agent according to any of claims 1 to 7 is applied on fiber-containing material optionally moistened beforehand.

**9.** The method according to claim 8, **characterized in that** the agent is rinsed after an exposure time.

**10.** The method according to any of claims 8 or 9, **characterized in that** the exposure time is from 15 to 60 minutes.

**11.** The method according to any of claims 8 or 9, **characterized in that** the hair is wound on reshaping aids before applying the agent.

**12.** The method according to any of claims 8 to 10, **characterized in that** the fiber-containing material is tempered during an exposure time permanently or temporarily at a temperature of 60°C to 200°C, in particular of 80°C to 180°C.

**13.** The method according to any of claims 8 or 9, **characterized in that** after applying the agent during the exposure time, the fiber-containing material is treated for smoothing with heated smoothing plates.

**14.** The use of an agent according to any of claims 1 to 7 for coating fiber-containing material, in particular human hair.

**15.** The use of an agent according to any of claims 1 to 7 for fixing the shape of fiber-containing material, in particular human hair.

**16.** The use of an agent according to any of claims 1 to 7 for increasing the washing resistance of coatings on fiber-containing material.

**17.** The use of an agent according to any of claims 1 to 7 for improving the resistance of coatings of fiber-containing material against mechanical stress.

**18.** A polymer, containing the following combination of respectively at least one of the listed structural units

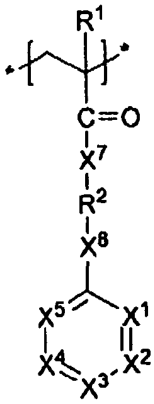 and 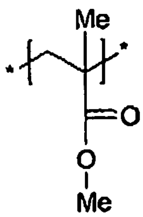 and 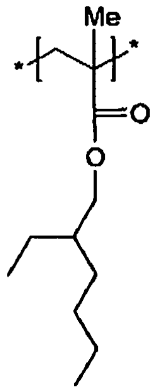 (P122)

(IV) (M1-15) (M1-22)

wherein the radicals $R^1$, $R^2$, $X^1$ to $X^8$ are defined as in claim 1.

**19.** A polymer, containing the following combination of respectively at least one of the listed structural units

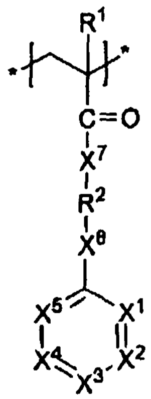 and 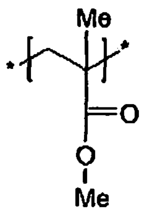 and 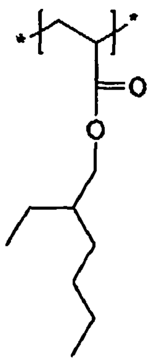 (P122a)

(IV) (M1-15) (M1-8)

wherein the radicals $R^1$, $R^2$, $X^1$ to $X^8$ are as defined in claim 1.

**20.** A polymer, containing the following combination of respectively at least one of the listed structural units

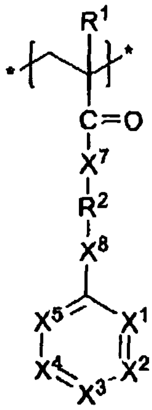 and 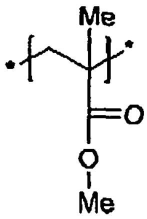 and 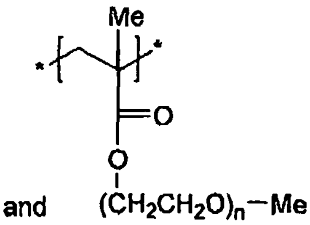

(IV) (M1-15) (P295)

wherein the radicals $R^1$, $R^2$, $X^1$ to $X^8$ are as defined in claim 1 and n represents an integer from 1 to 60.

**21.** A polymer, containing the following combination of respectively at least one of the listed structural units

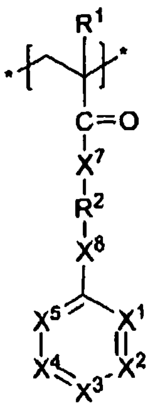 and 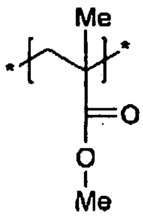 and 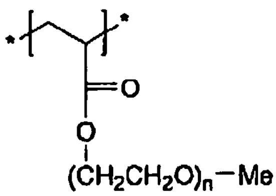

(IV) (M1-15) (P296)

wherein the radicals $R^1$, $R^2$, $X^1$ to $X^8$ are as defined in claim 1 and n represents an integer from 1 to 60.

**Revendications**

**1.** Agent qui contient, dans un support cosmétique, au moins un polymère comprenant au moins une unité de structure répondant à la formule (IV)

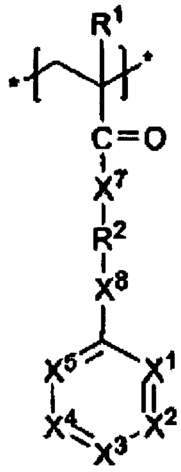

(IV)

dans laquelle

- $X^1$, $X^2$, $X^3$, $X^4$ et $X^5$ représentent, indépendamment l'un de l'autre, un groupe méthanylylidène, un atome d'azote ou un groupe halogénométhanylylidène, avec cette mesure qu'au moins deux des radicaux $X^1$ à $X^5$ représentent un atome d'azote et au moins deux des radicaux $X^1$ à $X^5$ représentent un groupe halogénométhanylylidène et au maximum un radical parmi les radicaux $X^1$ à $X^5$ représente un groupe méthanylylidène ;
- $X^7$ représente un atome d'oxygène ou un groupe NH ;
- $X^8$ représente une liaison directe, un atome d'oxygène ou un groupe NH ;
- $R^1$ représente un atome d'hydrogène ou un groupe alkyle en $C_1$-$C_4$;
- $R^2$ représente une liaison directe, un groupe alkylène en $C_2$-$C_6$, un groupe oxyde d'oligoalkylène contenant de 1 à 20 unités d'oxyde d'alkylène en $C_2$-$C_4$ ou un groupe phénylène.

2. Agent selon la revendication 1, **caractérisé en ce que** $X^1$, $X^3$ et $X^5$ représentent un atome d'azote, $X^2$ et $X^4$ représentent un groupe halogénométhanylylidène et $X^6$ représente un groupe méthanylylidène.

3. Agent selon la revendication 1, **caractérisé en ce que** $X^1$, $X^2$ et $X^4$ représentent un groupe halogénométhanylylidène, $X^6$ représente un groupe méthanylylidène et $X^3$ et $X^5$ représentent un atome d'azote.

4. Agent selon l'une quelconque des revendications 1 à 3, **caractérisé en ce que** le polymère en question possède une masse molaire moyenne de 1500 à 1.250.000.

5. Agent selon l'une quelconque des revendications 1 à 4, **caractérisé en ce qu'**il s'agit d'un copolymère.

6. Agent selon la revendication 5, **caractérisé en ce que** le polymère contient, à côté de l'unité de structure en question, en outre au moins une des unités de structure suivantes :

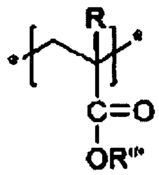

(M1)

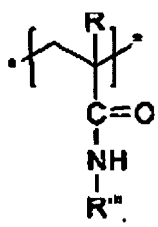

(M2)

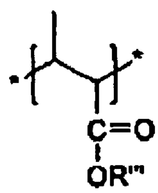

(M3)

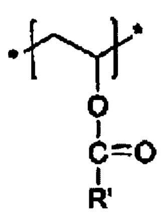 (M4) 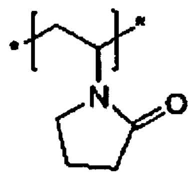 (M5) 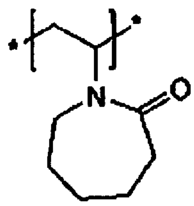 (M6)

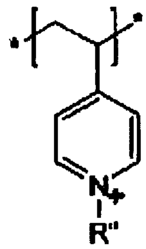 (M7) 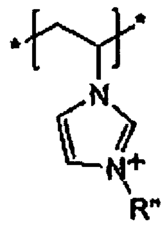 (M8) 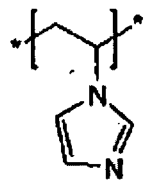 (M9)

dans lesquelles
R représente un atome d'hydrogène ou un groupe alkyle en $C_1$-$C_4$ ;
R' représente un groupe alkyle en $C_1$-$C_{30}$ ou un groupe aryle ;
R" représente un groupe alkyle en $C_1$-$C_4$, un groupe aralkyle en $C_1$-$C_4$ ou un groupe alcényle en $C_2$-$C_4$,
R"' représente un atome d'hydrogène, un groupe alkyle en $C_1$-$C_{30}$ linéaire ou ramifié, un groupe alcényle en $C_2$-$C_4$, un groupe ω-aminoalkyle en $C_2$-$C_6$, un groupe ω-dialkyl(en $C_1$-$C_4$)aminoalkyle en $C_2$-$C_6$, un groupe ω-trialkyl(en $C_1$-$C_4$)ammonioalkyle en $C_2$-$C_6$, un groupe $-(CH_2CH_2O)_n-R^{IV}$ où n représente un nombre entier de 1 à 60 et $R^{IV}$ représente un atome d'hydrogène ou un groupe alkyle en $C_1$-$C_{30}$.

7. Agent selon l'une quelconque des revendications 1 à 6, **caractérisé en ce que** l'unité de structure répondant à la formule (IV) est contenue dans le polymère à concurrence d'au moins 5 moles %, en particulier à concurrence d'au moins 20 moles %.

8. Procédé pour la fixation de forme de matériaux contenant des fibres, **caractérisé en ce qu'**on applique sur le matériau contenant des fibres éventuellement préhumidifié un agent selon l'une quelconque des revendications 1 à 7.

9. Procédé selon la revendication 8, **caractérisé en ce qu'**on élimine l'agent par rinçage après l'avoir laissé agir pendant un laps de temps.

10. Procédé selon l'une quelconque des revendications 8 ou 9, **caractérisé en ce que** le laps de temps d'action s'élève de 15 à 60 minutes.

11. Procédé selon l'une quelconque des revendications 8 ou 9, **caractérisé en ce que** les cheveux, avant l'application de l'agent, sont enroulés sur des auxiliaires de façonnement.

12. Procédé selon l'une quelconque des revendications 8 à 10, **caractérisé en ce que** le matériau contenant des fibres est maintenu, pendant un laps de temps d'action, de manière permanente ou de temps en temps à une température de 60 °C à 200 °C, en particulier de 80 °C à 180 °C.

13. Procédé selon l'une quelconque des revendications 8 ou 9, **caractérisé en ce que**, après l'application de l'agent, pendant le laps de temps d'action, le matériau contenant des fibres est traité à des fins de lissage avec des plaques de lissage chauffées.

14. Utilisation d'un agent selon l'une quelconque des revendications 1 à 7 pour l'enduction de matériaux contenant des fibres, en particulier de cheveux humains.

15. Utilisation d'un agent selon l'une quelconque des revendications 1 à 7 pour la fixation de forme de matériaux

contenant des fibres, en particulier de cheveux humains.

**16.** Utilisation d'un agent selon l'une quelconque des revendications 1 à 7 pour augmenter la résistance au lavage d'enductions sur un matériau contenant des fibres.

**17.** Utilisation d'un agent selon l'une quelconque des revendications 1 à 7 pour améliorer la résistance d'enductions de matériaux contenant des fibres contre la sollicitation mécanique.

**18.** Polymère, contenant la combinaison suivante de respectivement au moins une des unités de structure indiquées :

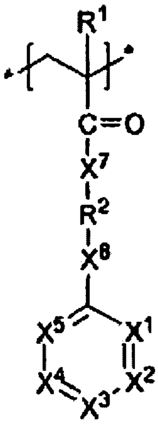

**(IV)**

**et**

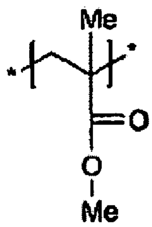

**(M1-15)**

**et**

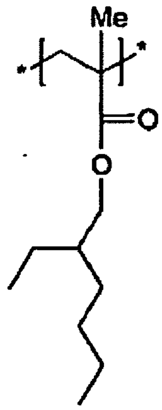

**(M1-22)**

**(P122)**

dans lesquelles les radicaux $R^1$, $R^2$, $X^1$ à $X^8$ sont tels que définis à la revendication 1.

**19.** Polymère, contenant la combinaison suivante de respectivement au moins une des unités de structure indiquées :

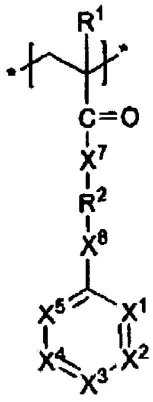

**(IV)**

**et**

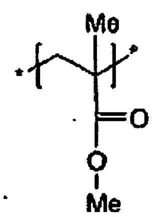

**(M1-15)**

**et**

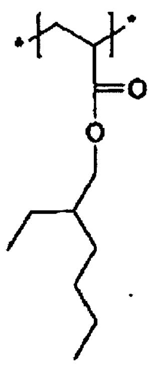

**(M1-8)**

**(P122a)**

dans lesquelles les radicaux $R^1$, $R^2$, $X^1$ à $X^8$ sont tels que définis à la revendication 1.

**20.** Polymère, contenant la combinaison suivante de respectivement au moins une des unités de structure indiquées :

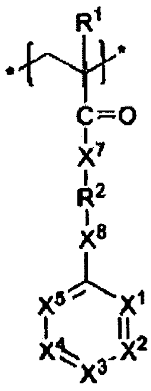 et 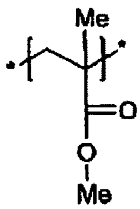 et 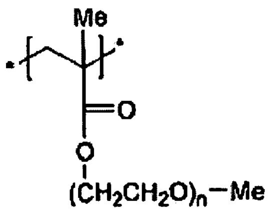

(IV) (M1-15) (P295)

dans lesquelles les radicaux $R^1$, $R^2$, $X^1$ à $X^8$ sont tels que définis à la revendication 1 et n représente un nombre entier de 1 à 60.

**21.** Polymère, contenant la combinaison suivante de respectivement au moins une des unités de structure indiquées :

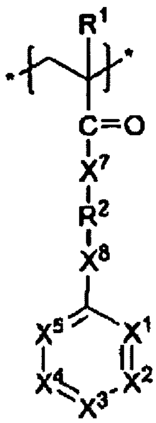 et 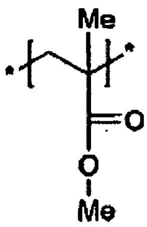 et 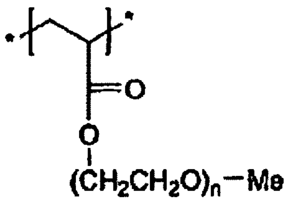

(IV) (M1-15) (P296)

dans lesquelles les radicaux $R^1$, $R^2$, $X^1$ à $X^8$ sont tels que définis à la revendication 1 et n représente un nombre entier de 1 à 60.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 1224320
- US 4168976 A
- DE OS1224320 A
- DE OS19756454 A
- DE 3725030 A
- DE 3723354 A
- DE 3926344 A
- DE OS19736906 A
- EP 0690044 A
- DE OS19738866 A
- DE 3929973


### In der Beschreibung aufgeführte Nicht-Patentliteratur


- **H.-D. Dörfler.** Grenzflächen- und Kolloidchemie. VCH Verlagsgesellschaft mbH, 1994
- Römpp-Lexikon Chemie. Georg Thieme Verlag, 1997, 1764
- **H.-D.Dörfler.** Grenzflächen- und Kolloidchemie. VCH Verlagsgesellschaft mbH, 1994
- International Cosmetic Ingredient Dictionary and Handbook. The Cosmetic, Toiletry, and Fragrance Association, 1997, 20036-4702